# EUROPEAN PATENT APPLICATION

(11) **EP 1 298 143 A1**
(43) Date of publication of application: **02.04.2003**
(21) Application number: 02256757.2
(22) Date of filing: 27.09.2002
(51) Int. Cl.: C07K 14/705, C12N 15/12, C07K 16/18, C12Q 1/68, G01N 33/68, G01N 33/50, C12N 5/10

(54) **44590, A human ABC transporter family member and uses therefor**

(30) Priority: 27.09.2001 US 325427 P
(71) Applicant: Millennium Pharmaceuticals, Inc., Cambridge, MA 02139 (US)
(72) Inventor: Rory A. J. Curtis, Massachusettes 01702 (DE)
(74) Representative: Lunt, Mark George Francis

(57) **Abstract**

The invention provides isolated nucleic acids molecules, designated 44590 nucleic acid molecules, which encode novel ABC transporter family members. The invention also provides antisense nucleic acid molecules, recombinant expression vectors containing 44590 nucleic acid molecules, host cells into which the expression vectors have been introduced, and nonhuman transgenic animals in which a 44590 gene has been introduced or disrupted. The invention still further provides isolated 44590 proteins, fusion proteins, antigenic peptides and anti-44590 antibodies. Diagnostic and therapeutic methods utilizing compositions of the invention are also provided.

## Description

Cellular membranes differentiate the contents of a cell from the surrounding environment. Membranes also may serve as effective barriers against the unregulated influx of hazardous or unwanted compounds, and the unregulated efflux of desirable compounds. However, the cell does need a supply of desired compounds and the removal of waste products. Transport proteins which are embedded (singly or in complexes) in the cellular membrane (reviewed by Oh and Amidon (1999) in *Membrane Transporters as Drug Targets*, ed. Amidon and Sadee, Kluwer Academic/Plenum Publishers, New York, Chapter 1) are major providers of these functions. There are two general classes of membrane transport proteins: channels or pores, and transporters (also known as carriers or permeases). Channels and transporters differ in their translocation mechanisms. Channels are hydrophilic group-lined protein tunnels whose opening by a regulatory event allow free, rapid passage of their charge-, size-, and geometry-selected small ions down their concentration gradients. Transporters specifically and selectively bind the molecules they move, some with and some against their concentration gradients, across membranes. The binding mechanism causes the action of transporters to be slow and saturable.

Transport molecules are specific for a particular target solute or class of solutes, and also are present in one or more specific membranes. Transport molecules localized to the plasma membrane permit an exchange of solutes with the surrounding environment, while transport molecules localized to intracellular membranes *(e.g.,* membranes of the mitochondrion, peroxisome, lysosome, endoplasmic reticulum, nucleus, or vacuole) permit import and export of molecules from organelle to organelle or to the cytoplasm. For example, in the case of the mitochondrion, transporters in the inner and outer mitochondrial membranes permit the import of sugar molecules, calcium ions, and water (among other molecules) into the organelle and the export of newly synthesized ATP to the cytosol.

Transporters can move molecules by two types of processes. In one process, "facilitated diffusion," transporters move molecules with their concentration gradients. In the other process, "active transport," transporters move molecules against their concentration gradients. Active transport to move a molecule against its gradient requires energy. Primary active transporters, such as Na⁺/K⁺ ATPases or ABC transporters use energy from ATP hydrolysis or light, to transport small molecules against the concentration gradient, establish ion gradients or establish membrane potential energy. Secondary active transporters, such as the H⁺/peptide transporter, use the pH or ion gradients established by primary active transporters to transport other molecules.

ATP-binding cassette (ABC) transporters are a superfamily of primary active transporters which convert a bound ATP molecule to ADP and inorganic phosphate (P;) to gain energy to shift its conformation and transport a bound target molecule. ABC transporters are ubiquitous, and have been identified in a wide variety of organisms. Individual ABC transporters are specific for a particular target solute or class of solutes. There are eight subfamilies of ABC transporter molecules, including the MDR/TAP, ALD, MRP/CFTR, ABC1, white, RNAse L inhibitor, ANSA, and GCN20 subfamilies (Klein *et al.* (1999) *Biochim. Biophys. Acta* 1461:237-262).

ABC transporters play important roles in the ability of the cell to regulate homeostasis, to grow and divide, and to communicate with other cells, e.g., to secrete and receive signaling molecules, such as hormones, reactive oxygen species, ions, neurotransmitters, and cytokines. A wide variety of human diseases and disorders are associated with defects in ABC transporter molecules, including cystic fibrosis (Zielenski *et al*. (1995) *Annu. Rev. Genet.* 29: 777-807)), persistent hyperinsulinemic hypoglycemia of infancy (Thomas *et al.* (1995) *Science* 268: 426-429), atherosclerosis (Brooks-Wilson, A. *et al*. (1999) *Nature Genet.* 22:336-345) and multidrug resistance of cancer cells (Chen *et al*. (1986) *Cell* 47:381-389; and A. van Helvoort *et al*. (1996) *Cell* 87: 507-517).

The present invention is based, in part, on the discovery of a novel ABC transporter family member, referred to herein as "44590". The nucleotide sequence of a cDNA encoding 44590 is shown in SEQ ID NO:1, and the amino acid sequence of a 44590 polypeptide is shown in SEQ ID NO:2. In addition, the nucleotide sequence of the coding region is depicted in SEQ ID NO:3.

Accordingly, in one aspect, the invention features a nucleic acid molecule which encodes a 44590 protein or polypeptide, *e.g.,* a biologically active portion of the 44590 protein. In a preferred embodiment, the isolated nucleic acid molecule encodes a polypeptide having the amino acid sequence of SEQ ID NO:2. In other embodiments, the invention provides isolated 44590 nucleic acid molecules having the nucleotide sequence shown in SEQ ID NO: 1 or SEQ ID NO:3. In still other embodiments, the invention provides nucleic acid molecules that are substantially identical (*e.g*., naturally occurring allelic variants) to the nucleotide sequence shown in SEQ ID NO:1 or SEQ ID NO:3. In other embodiments, the invention provides a nucleic acid molecule which hybridizes under a stringent hybridization condition as described herein to a nucleic acid molecule comprising the nucleotide sequence of SEQ ID NO:1 or SEQ ID NO:3, wherein the nucleic acid encodes a full length 44590 protein or an active fragment thereof.

In a related aspect, the invention further provides nucleic acid constructs which include a 44590 nucleic acid molecule described herein. In certain embodiments, the nucleic acid molecules of the invention are operatively linked to native or heterologous regulatory sequences. Also included are vectors and host cells containing the 44590 nucleic acid molecules of the invention *e.g.,* vectors and host cells suitable for producing polypeptides.

In another related aspect, the invention provides nucleic acid fragments suitable as primers or hybridization probes for the detection of 44590-encoding nucleic acids.

In still another related aspect, isolated nucleic acid molecules that are antisense to a 44590 encoding nucleic acid molecule are provided.

In another aspect, the invention features 44590 polypeptides, and biologically active or antigenic fragments thereof that are useful, *e.g*., as reagents or targets in assays applicable to treatment and diagnosis of ABC transporter-associated or other 44590-associated disorders. In another embodiment, the invention provides 44590 polypeptides having a 44590 activity. Preferred polypeptides are 44590 proteins including at least one ABC transporter domain and, preferably, having a 44590 activity, *e*.*g*., a 44590 activity as described herein.

In other embodiments, the invention provides 44590 polypeptides, *e.g.,* a 44590 polypeptide having the amino acid sequence shown in SEQ ID NO:2; an amino acid sequence that is substantially identical to the amino acid sequence shown in SEQ ID NO:2; or an amino acid sequence encoded by a nucleic acid molecule having a nucleotide sequence which hybridizes under a stringent hybridization condition as described herein to a nucleic acid molecule comprising the nucleotide sequence of SEQ ID NO:1 or SEQ ID NO:3, wherein the nucleic acid encodes a full length 44590 protein or an active fragment thereof.

In a related aspect, the invention further provides nucleic acid constructs which include a 44590 nucleic acid molecule described herein.

In a related aspect, the invention provides 44590 polypeptides or fragments operatively linked to non-44590 polypeptides to form fusion proteins.

In another aspect, the invention features antibodies and antigen-binding fragments thereof, that react with, or more preferably specifically or selectively bind 44590 polypeptides.

In another aspect, the invention provides methods of screening for compounds that modulate the expression or activity of the 44590 polypeptides or nucleic acids.

In still another aspect, the invention provides a process for modulating 44590 polypeptide or nucleic acid expression or activity, *e.g.,* using the compounds identified in the screens described herein. In certain embodiments, the methods involve treatment of conditions related to aberrant activity or expression of the 44590 polypeptides or nucleic acids, such as conditions or disorders involving aberrant or deficient ABC transporter function or expression. Examples of such disorders *e.g.,* ABC transporter-associated or other 44590-associated disorders include, but are not limited to, cellular proliferative and/or differentiative disorders, neurological disorders, disorders associated with bone metabolism, immune *e.g*., inflammatory disorders, cardiovascular disorders, endothelial cell disorders, liver disorders, pain or metabolic disorders, and hormonal.

The invention also provides assays for determining the activity of or the presence or absence of 44590 polypeptides or nucleic acid molecules in a biological sample, including for disease diagnosis.

In a further aspect, the invention provides assays for determining the presence or absence of a genetic alteration in a 44590 polypeptide or nucleic acid molecule, including for disease diagnosis.

In another aspect, the invention features a two dimensional array having a plurality of addresses, each address of the plurality being positionally distinguishable from each other address of the plurality, and each address of the plurality having a unique capture probe, *e*.*g*., a nucleic acid or peptide sequence. At least one address of the plurality has a capture probe that recognizes a 44590 molecule. In one embodiment, the capture probe is a nucleic acid, *e.g.,* a probe complementary to a 44590 nucleic acid sequence. In another embodiment, the capture probe is a polypeptide, *e.g.,* an antibody specific for 44590 polypeptides. Also featured is a method of analyzing a sample by contacting the sample to the aforementioned array and detecting binding of the sample to the array.

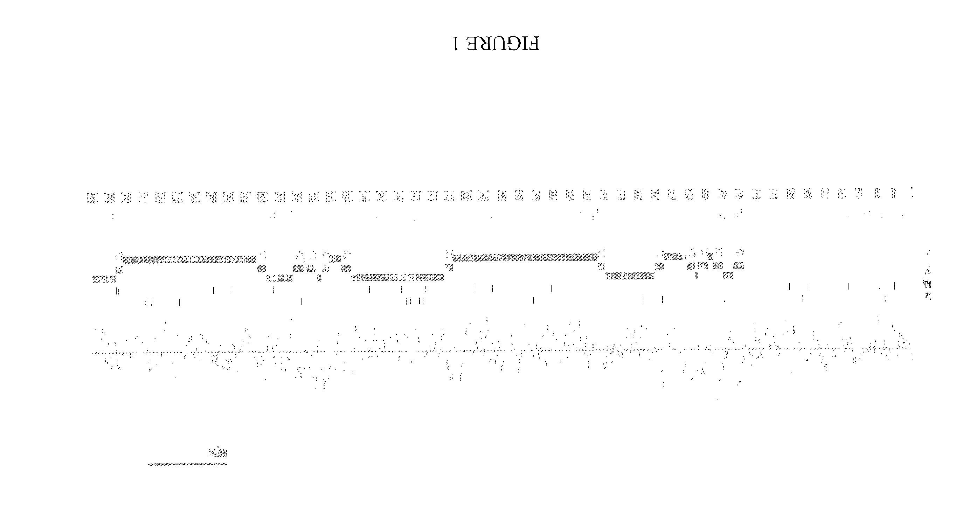

Other features and advantages of the invention will be apparent from the following detailed description, and from the claims. *Figure 1* depicts a hydropathy plot of human 44590. Relatively hydrophobic residues are shown above the dashed horizontal line, and relatively hydrophilic residues are below the dashed horizontal line. The cysteine residues (cys) are indicated by short vertical lines just below the hydropathy trace. The numbers corresponding to the amino acid sequence of human 44590 are indicated. Polypeptides of the invention include fragments which include: all or part of a hydrophobic sequence, *e.g.,* a sequence above the dashed line, *e.g.,* the sequence from about amino acid 440 to 460, from about 1080 to 1090, and from about 1370 to 1385 of SEQ ID NO:2; all or part of a hydrophilic sequence, e.g., a sequence below the dashed line, e.g., the sequence from about amino acid 270 to 280, from about 780 to 795, and from about 1190 to 1200 of SEQ ID NO:2; a sequence which includes a Cys, or a glycosylation site.

The human 44590 sequence ( SEQ ID NO:1), which is approximately 6747 nucleotides long including untranslated regions, contains a predicted methionine-initiated coding sequence of about 5793 nucleotides, including the termination codon (nucleotides indicated as coding of SEQ ID NO:1; SEQ ID NO:3). The coding sequence encodes a 1930 amino acid protein (SEQ ID NO:2).

Human 44590 contains the following regions or other structural features (for general information regarding PFAM identifiers, PS prefix and PF prefix domain identification numbers, refer to Sonnhammer *et al*. (1997) *Protein* 28:405-420);

two ABC transporter domains (PFAM Accession Number PF00005) located at about amino acid residues 706 to 888, and amino acids 1618 to 1800, of SEQ ID NO:2; twelve transmembrane domains (predicted by MEMSAT, Jones *et al*. (1994) *Biochemistry* 33:3038-3049)located at about amino acids 396 to 420, 446 to 468, 480 to 504, 511 to 531, 585 to 606, 723 to 740, 1081 to 1102, 1322 to 1344, 1374 to 1391, 1402 to 1426, 1434 to 1458, and 1521 to 1542 of SEQ ID NO:2; six extracellular loops located at about amino acid residues 421 to 445, 505 to 510, 607 to 722, 1103 to 1321, 1392 to 1401, and 1459 to 1520 of SEQ ID NO:2; five cytoplasmic loops located at about amino acid residues 469 to 479, 532 to 584, 741 to 1080, 1345 to 1373, and 1472 to 1435 of SEQ ID NO:2; one ABC transporters family signature located at about amino acids 814 to 828 of SEQ ID NO:2; two ATP/GTP binding site motif's A (P-loop) located at about amino acid residues 713 to 720, and 1625 to 1632, of SEQ ID NO:2; twenty two N-glycosylation sites (Prosite PS00001) located at about amino acids 51 to 54, 87 to 90, 161 to 164, 255 to 258, 298 to 301, 505 to 508, 859 to 862, 998 to 1001, 1039 to 1042, 1104 to 1107, 1154 to 1157, 1170 to 1173, 1211 to 1214, 1256 to 1259, 1287 to 1290, 1513 to 1516, 1543 to 1546, 1558 to 1561, 1610 to 1613, 1653 to 1656, 1877 to 1880, and 1882 to 1885 of SEQ ID NO:2; one glycosaminoglycan attachment site (Prosite PS00002) located at about amino acid residues 1358 to 1361 of SEQ ID NO:2; three cAMP/cGMP-dependent protein kinase phosphorylation sites (Prosite PS00004) located at about amino acids 820 to 823, 913 to 916, and 1731 to 1734 of SEQ ID NO:2; thirtyfour protein kinase C phosphorylation sites (Prosite PS00005) located at about amino acids 28 to 30, 47 to 49, 81 to 83, 94 to 96, 163 to 165, 201 to 203, 297 to 299, 319 to 321, 323 to 325, 329 to 331, 332 to 334, 372 to 374, 750 to 752, 769 to 771, 779 to 781, 786 to 788, 797 to 799, 805 to 807, 848 to 850, 861 to 863, 879 to 881, 912 to 914, 959 to 961, 1003 to 1005, 1015 to 1017, 1041 to 1043, 1075 to 1077, 1101 to 1103, 1474 to 1476, 1537 to 1539, 1729 to 1731, 1759 to 1761, 1820 to 1822, and 1876 to 1878 of SEQ ID NO:2; twenty four casein kinase II phosphorylation sites (Prosite PS00006) located at about amino acids 270 to 273, 668 to 671, 769 to 772, 797 to 800, 842 to 845, 851 to 854, 990 to 993, 1028 to 1031, 1041 to 1044, 1049, 1052, 1108 to 1111, 1171 to 1174, 1183 to 1186, 1224 to 1227, 1289 to 1292, 1318 to 1321, 1499 to 1502, 1565 to 1568, 1682 to 1685, 1753 to 1756, 1783 to 1786, 1854 to 1857, 1891 to 1894 to 1923 to 1926 of SEQ ID NO:2; two tyrosine kinase phosphorylation sites (Prosite PS00007) located at about amino acid residues 796 to 804, and 1261 to 1269 of SEQ ID NO:2; and twenty two N-myristoylation sites (Prosite PS00008) located at about amino acids 55 to 60, to 107 to 112, 439 to 444, 645 to 650, 691 to 696, 716 to 721, 728 to 733, 812 to 817, 843 to 848, 888 to 893, 964 to 969, 986 to 991, 1020 to 1025, 1100 to 1105, 1180 to 1185, 1239 to 1244, 1252 to 1257, 1359 to 1364, 1614 to 1619, 1625 to 1630, 1865 to 1870, and 1916 to 1921 of SEQ ID NO:2.

The 44590 protein contains a significant number of structural characteristics in common with members of the ABC transporter family. For example, the 44590 protein contains two ABC transporter domains located at about amino acid residues 706 to 888, and 1680 to 1800 of SEQ ID NO:2, and two SR54-type protein, GTPase domains located at about amino acid residues 711 to 723, and 1623 to 1633 of SEQ ID NO:2. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologs of non-human origin, *e.g.*, rat or mouse proteins. Members of a family also can have common functional characteristics.

As used herein, the term "ABC transporter" includes a protein or polypeptide which is capable of converting a bound ATP molecule to ADP and inorganic phosphate (Pᵢ) to gain energy to shift its conformation and transport a bound target molecule across a lipid membrane. Transporters usually are involved in the movement of a biochemical compound (*e.g*., a peptide, an ion, or other small molecule, such as a nucleotide, signaling molecule, vitamin, or cofactor), which normally would not be able to cross a membrane. ABC transporters are ubiquitous, and have been identified in a wide variety of organisms. Individual ABC transporters are specific for a particular target solute or class of solutes. There are eight subfamilies of ABC transporter molecules, including the MDR/TAP, ALD, MRP/CFTR, ABC1, white, RNAse L inhibitor, ANSA, and GCN20 subfamilies (Klein *et al.* (1999) *Biochim. Biophys. Acta* 1461: 237-262). Major differences between the subfamilies include the number of transmembrane domain-nucleotide binding cassette repeats found in the proteins. So-called 'full-size' ABC family transporters have two transmembrane domain-ATP binding cassette domains (*e.g.,* the multidrug transporters (Mdr), and cystic fibrosis transmembrane conductance regulator (CFTR)), while 'half-transporters,' have only one of each domain. Full-size transporters may be found in the plasma membrane, or in the membranes of organelles in homo- or hetero-dimeric form. The 44590 polypeptide may be classified as a full size transporter by identification of twelve transmembrane domains, and two ABC transporter domain repeats

An alignment of the 44590 protein with an ATP-binding cassette transporter; corresponding to Genbank accession number AJ012376 demonstrates about 37.2% sequence identity between the two sequences (as calculated in matblas from the blosum62.iij matrix).

A 44590 polypeptide can include at least two "ABC transporter domains" or regions homologous with "ABC transporter domains" and at least one ABC transporters family signature.

As used herein, the term "ABC transporter domain" includes an amino acid sequence of about 100 to 300 amino acid residues in length and having a bit score for the alignment of the sequence to the ABC transporter domains (HMM) of at least 100. Preferably ABC transporter domains mediate active transport of small hydrophilic molecules across cell membranes. Preferably, ABC transporter domains include at least about 100 to 300 amino acids, more preferably about 150 to 250 amino acid residues, or about 175 to 200 amino acids and have bit scores for the alignment of the sequence to the ABC transporter domain (HMM) of at least 100, more preferably 125, and most preferably 150 or greater.

The ABC transporter domain can include an ABC transporters family signature, Prosite signature sequence PS00211, or sequences homologous thereto. In the 44590 polypetide the molecule contains the following signature sequence: [LIVMFYC]-[SA]-SAPGLVFYKQH]-G-[DENQMW]-DENQMW]-[KRQASPCLIMFW]-[KRNQSTAVM]-[KRACLVM]-LIVMFYPAN]-{PHY}-[LIVMFW]-[SAGCLIVP]-{FYWHP}-{KRHP}-[LIVMFYWSTA] (SEQ ID NO:5). In the above conserved signature sequence, and other motifs or signature sequences described herein, the standard IUPAC one-letter code for the amino acids is used. Each element in the pattern is separated by a dash (-); square brackets ([ ]) indicate the particular residues that are accepted at that position; x indicates that any residue is accepted at that position; curled brackets ({}) indicate particular residues that can NOT be accepted in that position; and numbers in parentheses (()) indicate the number of residues represented by the accompanying amino acid. The ABC transporters family signature is located about the third cytoplasmic loop (between the sixth and seventh transmembrane domains )of human 44590 polypeptide and which corresponds to about amino acids 814 to 828 of SEQ ID NO:2. The ABC transporter domain (HMM) has been assigned the PFAM Accession Number PF00005.

An alignment of the ABC transporter domains (amino acids 706 to 888, and amino acids 1618 to 1800, of SEQ ID NO:2) of human 44590 with the Pfam ABC transporter domain consensus amino acid sequence (SEQ ID NO:4) derived from a hidden Markov model and is described herein.

In a preferred embodiment, a 44590 polypeptide or protein has at least two "ABC transporter domains" or a regions which include at least about 100 to 300, more preferably about 150 to 250 or most preferably 175 to 200 amino acid residues and has at least about 60%, 70% 80% 90% 95%, 99%, or 100% homology with an "ABC transporter domain," *e.g.,* the ABC transporter domains of human 44590 *(e.g.,* residues 706 to 888, and amino acids 1618 to 1800, of SEQ ID NO:2).

To identify the presence of a "ABC transporter" domain in a 44590 protein sequence, and make the determination that a polypeptide or protein of interest has a particular profile, the amino acid sequence of the protein can be searched against the Pfam database of HMMs *(e.g.,* the Pfam database, release 2.1) using the default parameters. For example, the hmmsf program, which is available as part of the HMMER package of search programs, is a family specific default program for MILPAT0063 and a score of 15 is the default threshold score for determining a hit. Alternatively, the threshold score for determining a hit can be lowered (*e.g.,* to 8 bits). A description of the Pfam database can be found in Sonhammer *et al.* (1997) *Proteins* 28:405-420 and a detailed description of HMMs can be found, for example, in Gribskov *et al.* (1990) *Meth. Enzymol*. 183:146-159; Gribskov *et al*. (1987) *Proc. Natl*. *Acad*. *Sci*. *USA* 84:4355-4358; Krogh *et al.* (1994) *J*. *Mol. Biol.* 235:1501-1531; and Stultz *et al.* (1993) *Protein Sci.* 2:305-314, the contents of which are incorporated herein by reference. A search was performed against the HMM database resulting in the identification of an "ABC transporter domain" in the amino acid sequence of human 44590 at about residues 706 to 888, and amino acids 1618 to 1800, of SEQ ID NO:2.

A 44590 polypeptide can include at least one, two, or three, preferably four, five, or six, more preferably seven, eight, or nine, even more preferably ten or eleven, and most preferably, twelve "transmembrane domains" or regions homologous with a "transmembrane domain". As used herein, the term "transmembrane domain" includes an amino acid sequence of about 10 to 40 amino acid residues in length and spans the plasma membrane. Transmembrane domains are rich in hydrophobic residues, *e.g*., at least 50%, 60%, 70%, 80%, 90%, 95% or more of the amino acids of a transmembrane domain are hydrophobic, *e.g.*, leucines, isoleucines, tyrosines, or tryptophans. Transmembrane domains typically have alpha-helical structures and are described in, for example, Zagotta *et al*., (1996) *Annual Rev. Neurosci*. 19:235-263, the contents of which are incorporated herein by reference. The transmembrane domains of human 44590 is located at about residues 396 to 420, about 446 to 468, about 480 to 504, about 511 to 531, about 585 to 606, about 723 to 740, about 1081 to 1102, about 1322 to 1344, about 1374 to 1391, about 1402 to 1426, about 1434 to 1458, and about 1521 to 1542 of SEQ ID NO:2.

In a preferred embodiment, a 44590 polypeptide or protein has at least one, two, or three, preferably four, five, or six, more preferably seven, eight, or nine, even more preferably ten or eleven, and most preferably, twelve "transmembrane domains" or regions which includes at least about 12 to 35 more preferably about 14 to 30 or 15 to 25 amino acid residues and has at least about 60%, 70% 80% 90% 95%, 99%, or 100% homology with a "transmembrane domain," *e.g.*, the transmembrane domains of human 44590 *(e.g.,* about amino acid residues 396 to 420, about 446 to 468, about 480 to 504, about 511 to 531, about 585 to 606, about 723 to 740, about 1081 to 1102, about 1322 to 1344, about 1374 to 1391, about 1402 to 1426, about 1434 to 1458, and about 1521 to 1542 of SEQ ID NO:2).

The transmembrane domain of human 44590 is visualized in the hydropathy plot (Figure 1) as regions of about 15 to 25 amino acids where the hydropathy trace is mostly above the horizontal line.

To identify the presence of a "transmembrane" domain in a 44590 protein sequence, and make the determination that a polypeptide or protein of interest has a particular profile, the amino acid sequence of the protein can be analyzed by a transmembrane prediction method that predicts the secondary structure and topology of integral membrane proteins based on the recognition of topological models (MEMSAT, Jones *et al*., (1994) *Biochemistry* 33:3038-3049).

A 44590 polypeptide can include at least one, two, or three, preferably four, five, or six, more preferably seven, eight, or nine, even more preferably ten or eleven, or twelve and most preferably, thirteen "non-transmembrane regions." As used herein, the term "non-transmembrane region" includes an amino acid sequence not identified as a transmembrane domain. The non-transmembrane regions in 44590 are located at about amino acids 1 to 395, about 421 to 445, about 469 to 479, about 505 to 510, about 532 to 584, about 607 to 722, about 741 to 1080, about 1103 to 1321, about 1345 to 1373, about 1392 to 1401, about 1427 to 1435, about 1459 to 1520, and about 1543 to 1930 of SEQ ID NO:2.

The 44590 polypeptide has thirteen "non-transmembrane regions". ABC transporters of the subclass of ATP binding cassette transporters (e.g., the ABCR transporters) consist of a single polypeptide chain arranged in two tandem halves. Each having a multi-spanning membrane domain, followed by a non-transmembrane nucleotide binding domain. The toplogy of the ABC transporter is exemplified, though not restricted to, a model in which each half of polypeptide has a transmembrane segment, followed by an exocytoplasmic domain, a multi spanning membrane domain, and a nucleotide binding domain.

The non-transmembrane regions of 44590 include at least one, two or, three, preferably four, five or six, and most preferably, seven cytoplasmic regions. When located at the N-terminus, the cytoplasmic region is referred to herein as the "N-terminal cytoplasmic domain." As used herein, an "N-terminal cytoplasmic domain" includes an amino acid sequence having about 1 to 500, preferably about 1 to 450, more preferably about 1 to 400, or even more preferably about 1 to 395 amino acid residues in length, and is located inside of a cell or within the cytoplasm of a cell. The C-terminal amino acid residue of an "N-terminal cytoplasmic domain" is adjacent to an N-terminal amino acid residue of a transmembrane domain in a 44590 protein. For example, an N-terminal cytoplasmic domain is located at about amino acid residues 1 to 395 of SEQ ID NO:2.

In a preferred embodiment, a 44590 polypeptide or protein has an N-terminal cytoplasmic domain or a region which includes about 1 to 500, preferably about 1 to 450, and more preferably about 1 to 395 amino acid residues and has at least about 60%, 70% 80% 90% 95%, 99%, or 100% homology with an "N-terminal cytoplasmic domain," *e.g.*, the N-terminal cytoplasmic domain of human 44590 (*e.g*., residues 1 to 125 of SEQ ID NO:2).

In another embodiment, a 44590 cytoplasmic region includes at least one or two, preferably three or four, and most preferably, five cytoplasmic loops. As used herein, the term "loop" includes an amino acid sequence which is not included within a phospholipid membrane, having a length of at least about 600, preferably about 100 to 500, more preferably about 200 to 400, even more preferably about 300 to 375, and most preferably about 325 to 350 to amino acid residues, and has an amino acid sequence that connects two transmembrane domains within a protein or polypeptide. Accordingly, the N-terminal amino acid of a loop is adjacent to a C-terminal amino acid of a transmembrane domain in a 44590 molecule, and the C-terminal amino acid of a loop is adjacent to an N-terminal amino acid of a transmembrane domain in a 44590 molecule. As used herein, a "cytoplasmic loop" includes a loop located inside of a cell or within the cytoplasm of a cell. For example, a "cytoplasmic loop" can be found at about amino acid residues 469 to 479, about 532 to 584, about 741 to 1080, about 1345 to 1373, and about 1427 to 1435 of SEQ ID NO:2.

In a preferred embodiment, a 44590 polypeptide or protein has a cytoplasmic loop or a region which includes at least 600, preferably about 100 to 500, more preferably about 200 to 400, even more preferably about 300 to 375, and most preferably about 325 to 350 to amino acid residues amino acid residues and has at least about 60%, 70% 80% 90% 95%, 99%, or 100% homology with a cytoplasmic loop," e.g., a cytoplasmic loop of human 44590 (*e.g.*, residues about 469 to 479, about 532 to 584, about 741 to 1080, about 1345 to 1373, and about 1427 to 1435 of SEQ ID NO:2).

In another embodiment, a 44590 non-transmembrane region includes at least one, two, or three, preferably four or five, and most preferably six non-cytoplasmic loops. As used herein, an "non-cytoplasmic loops " includes a loop located outside of a cell or within an intracellular organelle. Non-cytoplasmic loops include extracellular domains (*i.e.*, outside of the cell) and intracellular domains (*i.e.*, within the cell). When referring to membrane-bound proteins found in intracellular organelles (*e.g*., mitochondria, endoplasmic reticulum, peroxisomes microsomes, vesicles, endosomes, and lysosomes), non-cytoplasmic loops include those domains of the protein that reside in the lumen of the organelle or the matrix or the intermembrane space. For example, a "non-cytoplasmic loop" can be found at about amino acid residues 421 to 445, about 505 to 510, about 607 to 722, about 1103 to 1321, about 1392 to 1401, and about 1459 to 1520 of SEQ ID NO:2.

In a preferred embodiment, a 44590 polypeptide or protein has at least one non-cytoplasmic loop or a region which includes at least about 300, preferably about 150 to 250, more preferably about 195 to 225 amino acid residues and has at least about 60%, 70% 80% 90% 95%, 99%, or 100% homology with a "non-cytoplasmic loop," *e.g.,* at least one non-cytoplasmic loop of human 44590 *(e.g.,* at about amino acid residues 421 to 445, about 505 to 510, about 607 to 722, about 1103 to 1321, about 1392 to 1401, and about 1459 to 1520 of SEQ ID NO:2).

In another embodiment, a cytoplasmic region of a 44590 protein can include the C-terminus and can be a "C-terminal cytoplasmic domain," also referred to herein as a "C-terminal cytoplasmic tail." As used herein, a "C-terminal cytoplasmic domain" includes an amino acid sequence having a length of at least about 250 to 500, preferably about 350 to 425, more preferably about 375 to 400 amino acid residues, is located inside of a cell or within the cytoplasm of a cell. The N-terminal amino acid residue of a "C-terminal cytoplasmic domain" is adjacent to a C-terminal amino acid residue of a transmembrane domain in a 44590 protein. For example, a C-terminal cytoplasmic domain is located at about amino acid residues 1543 to 1930 of SEQ ID NO:2.

In a preferred embodiment, a 44590 polypeptide or protein has a C-terminal cytoplasmic domain or a region which includes at least 250 to 500, preferably about 350 to 425, more preferably about 375 to 400 amino acid residues and has at least about 60%, 70% 80% 90% 95%, 99%, or 100% homology with a C-terminal cytoplasmic domain," *e.g.*, the C-terminal cytoplasmic domain of human 44590 (*e.g*., at about amino acid residues 421 to 445, about 505 to 510, about 607 to 722, about 1103 to 1321, about 1392 to 1401, and about 1459 to 1520 of SEQ ID NO:2)).

A human 44590 protein can further include at least two ATP/GTP-binding site motifs A (P loop), at about amino acid residues 713 to 720, and 1625 to 1632 of SEQ ID NO:2, which are conserved in many proteins that bind ATP or GTP. In addition, the human 44590 has at least one ABC transporters family signature at about amino acid residues 814 to 828 of SEQ ID NO:2, which is a characteristic of proteins involved in active transport of small hydrophilic molecules across cell membranes.

A 44590 family member can include at least one, preferably two transporter domains, at least one, two, three, or four, preferably five, six, seven, or eight, more preferably nine ten or eleven, and most preferably, twelve transmembrane domains, at least one, two or three, preferably four , and most preferably, five cytoplasmic loops, an at least one, two or three, preferably four, or five, and most preferably six extracellular loops. A 44590 family member can include at least one, preferably two ATP/GTP binding site motif A's (P-loop), and at least one ABC transporters family signature. Furthermore, a 44590 family member can include: at least, two, three, four, five, six, seven, eight, nine, ten, eleven, twelve, thirteen, fourteen, fifteen, sixteen, seventeen, eighteen, nineteen, twenty, twenty one, or most preferably twenty two N-glycosylation sites (Prosite PS00001); at least one glycosaminoglycan attachment site, (Prosite PS00002); at least one, two, preferably three cAMP/cGMP protein kinase phosphorylation sites (Prosite PS00004); at least one, two, three, four, five, six, seven, eight, nine, ten, eleven, twelve, thirteen, forteen, fifteen, sixteen, seventeen, eighteen, nineteen, twenty, twenty one, twenty two, twenty three, twenty four, twenty five, twenty six, twenty seven, twenty eight, twenty nine, thirty, thirty one, thirty two, thirty three, or most preferably thirty four, protein kinase C phosphorylation sites (Prosite PS00005); at least one, two, three, four, five, six, seven, eight, nine, ten, eleven, twelve, thirteen, forteen, fifteen, sixteen, seventeen, eighteen, nineteen, twenty, twenty one, twenty two, twenty three, or most preferably twenty four casein kinase II phosphorylation sites (Prosite PS00006); and at least, two, three, four, five, six, seven, eight, nine, ten, eleven, twelve, thirteen, fourteen, fifteen, sixteen, seventeen, eighteen, nineteen, twenty, twenty one, or most preferably twenty two N-myristoylation sites (Prosite PS00008).

As the 44590 polypeptides of the invention can modulate 44590-mediated activities, they can be useful for developing novel diagnostic and therapeutic agents for ABC transporter-associated or other 44590-associated disorders, as described below.

As used herein, an "ABC transporter-associated activity" includes an activity which involves the transport of one or more biochemical molecules across a cellular membrane, *e*.*g*., biochemical molecules in a neuronal cell, a muscle cell, or a liver cell associated with the regulation of one or more cellular processes. ABC transporter-mediated activities include the import or export of biochemical molecules necessary for energy production or storage, for intra- or intercellular signaling, or for metabolism or catabolism of metabolically important biomolecules, and the export of potentially harmful compounds from the cell.

ABC transporters play important roles in the ability of the cell to regulate homeostasis, to grow and divide, and to communicate with other cells, *e.g.,* to secrete and receive signaling molecules, such as hormones, reactive oxygen species, ions, neurotransmitters, and cytokines. A wide variety of human diseases and disorders are associated with defects in ABC transporter molecules, including cystic fibrosis (Zielenski *et al.* (1995) *Annu. Rev. Genet.* 29: 777-807)), persistent hyperinsulinemic hypoglycemia of infancy (Thomas *et al.* (1995) *Science* 268: 426-429), atherosclerosis (Brooks-Wilson, A. *et al.* (1999) *Nature Genet.* 22:336-345) and multidrug resistance of cancer cells (Chen *et al.* (1986) *Cell* 47:381-389; and A. van Helvoort *et al.* (1996) *Cell* 87: 507-517).

As used herein, a "44590 activity", "biological activity of 44590" or "functional activity of 44590", refers to an activity exerted by a 44590 protein, polypeptide or nucleic acid molecule on *e.g.,* a 44590-responsive cell or on a 44590 substrate, *e.g.,* a protein substrate, as determined *in vivo* or *in vitro.* In one embodiment, a 44590 activity is a direct activity, such as an association with a 44590 target molecule. A "target molecule" or "binding partner" is a molecule with which a 44590 protein binds or interacts in nature. In an exemplary embodiment, 44590 is an ABC transporter, and thus binds to or interacts in nature with a molecule, *e.g.,* a small hydrophilic molecule, an amino acid, a peptide, an ion, a sugar, a nucleic acid, a signaling molecule, vitamin, or cofactor; and a second molecule, *e.g.,* a nucleotide, *e.g.,* ATP or GTP.

A 44590 activity can also be an indirect activity, *e.g*., a cellular signaling activity mediated by interaction of the 44590 protein with a 44590 receptor. Based on the above-described sequence structures and similarities to molecules of known function, the 44590 molecules of the present invention can have similar biological activities as ABC transporter family members. For example, the 44590 proteins of the present invention can have the ability to modulate, either directly or indirectly, any one or more of the following activities: (1) growth, proliferation, and function of cells of the breast, (i.e., myoepithelial cells, duct-lining cells, cells of the secretory alveoli, (i.e., alveolar duct epithelium); (2)) growth, proliferation, and function of cells of the skin (i.e., epidermal cells (i.e., stratum basale (germinal cells), stratum spinosum (i.e., 'prickle cells' of tonofibrils), stratum granulosom (i.e, basophilic granules), Merkel cells, Langerhan's cells, and melanocytes)(3) the ability to reside within a membrane, *e.g.,* a cell or organelle membrane; (4) formation of a dimer with a second protein *e.g.,* another ABC transporter; (5) the ability to interact with, *e.g.,* bind to, a substrate or target molecule; (6) transport of a substrate or target molecule, *e.g.,* an amino acid, a peptide, an ion, a sugar, a nucleic acid, a signaling molecule, a vitamin, or a cofactor across a membrane, *e.g.,* the plasma membrane, or the membrane of the mitochondrion, the peroxisome, the lysosome, the endoplasmic reticulum, the nucleus, or a vacuole; (7) interaction with a second substrate or target molecule, *e.g.,* a nucleotide *(e.g.,* ATP or GTP); (8) hydrolysis of a nucleotide, *e.g.,* ATP or GTP to remove a phosphate group; (9) interaction with and/or modulate the activity of a non-transporter protein; (10) intra- or intercellular signaling and/or gene transcription *(e.g.,* either directly or indirectly); (11) the import and export of molecules from cells, *e.g.,* hormones, ions, cytokines, neurotransmitters, and metabolites; or (12) modulation of the removal of potentially harmful compounds from the cell, or facilitate the compartmentalization of these molecules into a sequestered intracellular space *(e.g.,* the peroxisome).
The 44590 molecules of the invention can modulate the activities of cells in tissues where they are expressed. For example, 44590 mRNA is expressed in tumourous breast tissue cells, and to a lesser extent in normal breast tissue cells. The 44590 molecules of the invention are also expressed in skin cells. Accordingly, the 44590 molecules of the invention can act as therapeutic or diagnostic agents for disorders of the breast. The 44590 molecules can be used to treat breast related tumors (i.e., cellular and/or proliferative disorders of the breast) in part because the 44590 mRNA is expressed higher in tumorous breast cells than in nomal breast cells. Examples of breast tumour related disorders that may be identified or treated with the 44590 molecules include, but are not limited to:
proliferative cell breast diseases including, but not limited to, epithelial hyperplasia, sclerosing adenosis, and small duct papillomas; tumors including, but not limited to, stromal tumors such as fibroadenoma, phyllodes tumor, and sarcomas, and epithelial tumors such as large duct papilloma; carcinoma of the breast including in situ (noninvasive) carcinoma that includes ductal carcinoma in situ (including Paget's disease) and lobular carcinoma in situ, and invasive (infiltrating) carcinoma including, but not limited to, invasive ductal carcinoma, no special type, invasive lobular carcinoma, medullary carcinoma, colloid (mucinous) carcinoma, tubular carcinoma, and invasive papillary carcinoma, and miscellaneous malignant neoplasms.

Thus, the 44590 molecules can act as novel diagnostic targets and therapeutic agents for controlling one or more transporter disorders or other ABC transporter disorders. As used herein, "ABC transporter disorders" are diseases or disorders whose pathogenesis is caused by, is related to, or is associated with aberrant or deficient ABC transporter protein function or expression. Examples of such disorders, e.g., ABC transporter-associated or other 44590-associated disorders, include but are not limited to, breast disorders.

The 44590 molecules can play a role in the breast disorders described above, but may also be involved in, but not limited to, disorders of development; inflammations, including but not limited to, acute mastitis, periductal mastitis, periductal mastitis (recurrent subareolar abscess, squamous metaplasia of lactiferous ducts), mammary duct ectasia, fat necrosis, granulomatous mastitis, and pathologies associated with silicone breast implants; fibrocystic changes; proliferative breast disease including, but not limited to, epithelial hyperplasia, sclerosing adenosis, and small duct papillomas. Disorders in the male breast include, but are not limited to, gynecomastia and carcinoma.

The 44590 protein, fragments thereof, and derivatives and other variants of the sequence in SEQ ID NO:2 thereof are collectively referred to as "polypeptides or proteins of the invention" or "44590 polypeptides or proteins". Nucleic acid molecules encoding such polypeptides or proteins are collectively referred to as "nucleic acids of the invention" or "44590 nucleic acids."

As used herein, the term "nucleic acid molecule" includes DNA molecules (*e.g.*, a cDNA or genomic DNA) and RNA molecules (*e.g*., an mRNA) and analogs of the DNA or RNA generated, *e.g.,* by the use of nucleotide analogs. The nucleic acid molecule can be single-stranded or double-stranded, but preferably is double-stranded DNA.

The term "isolated or purified nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in *Current Protocols in Molecular Biology* (1989) John Wiley & Sons, N.Y., 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

As used herein, a "naturally-occurring" nucleic acid molecule refers to an RNA or DNA molecule having a nucleotide sequence that occurs in nature (*e*.*g*., encodes a natural protein).

As used herein, the terms "gene" and "recombinant gene" refer to nucleic acid molecules which include an open reading frame encoding a 44590 protein, preferably a mammalian 44590 protein, and can further include non-coding regulatory sequences, and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 44590 protein having less than about 30%, 20%, 10% and more preferably 5% (by dry weight), of non-44590 protein (also referred to herein as a "contaminating protein"), or of chemical precursors or non-44590 chemicals. When the 44590 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 44590 (*e.g*., the sequence of SEQ ID NO:1 or 3) without abolishing or more preferably, without substantially altering a biological activity, whereas an "essential" amino acid residue results in such a change. For example, amino acid residues that are conserved among the polypeptides of the present invention, *e.g.,* those present in the ABC transporter domain, are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains *(e.g.,* alanine, valine, leucine, isoleucine, proline, phenylalanine, methionine, tryptophan), beta-branched side chains (*e.g*., threonine, valine, isoleucine) and aromatic side chains *(e.g.,* tyrosine, phenylalanine, tryptophan, histidine). Thus, a predicted nonessential amino acid residue in a 44590 protein is preferably replaced with another amino acid residue from the same side chain family. Alternatively, in another embodiment, mutations can be introduced randomly along all or part of a 44590 coding sequence, such as by saturation mutagenesis, and the resultant mutants can be screened for 44590 biological activity to identify mutants that retain activity. Following mutagenesis of SEQ ID NO: 1 or SEQ ID NO:3, the encoded protein can be expressed recombinantly and the activity of the protein can be determined.

As used herein, a "biologically active portion" of a 44590 protein includes a fragment of a 44590 protein which participates in an interaction between a 44590 molecule and a non-44590 molecule. Biologically active portions of a 44590 protein include peptides comprising amino acid sequences sufficiently homologous to or derived from the amino acid sequence of the 44590 protein, *e.g.,* the amino acid sequence shown in SEQ ID NO:2, which include fewer amino acids than the full length 44590 protein, and exhibit at least one activity of a 44590 protein. Typically, biologically active portions comprise a domain or motif with at least one activity of the 44590 protein, *e.g.,* the ability to: reside within a membrane; dimerize with a second transporter, *e.g.,* an ABC transporter; bind and hydrolyze a nucleotide, *e.g.,* ATP or GTP; or bind and transport a molecule *e.g.,* an amino acid, a peptide, an ion, a sugar, a nucleic acid, a signaling molecule, a vitamin, or a cofactor across a membrane, *e.g.,* a cell or organelle membrane. A biologically active portion of a 44590 protein can be a polypeptide which is, for example, 10, 25, 50, 100, 200 or more amino acids in length. Biologically active portions of a 44590 protein can be used as targets for developing agents which modulate a 44590 mediated activity, *e.g.,* the ability to: reside within a membrane; dimerize with a second transporter, *e.g.,* an ABC transporter; bind and hydrolyze a nucleotide, *e.g*., ATP or GTP; or bind and transport a molecule *e.g*., an amino acid, a peptide, an ion, a sugar, a nucleic acid, a signaling molecule, a vitamin, or a cofactor across a membrane, *e.g.,* a cell or organelle membrane. Calculations of homology or sequence identity (the terms "homology" and "identity" are used interchangeably herein) between sequences are performed as follows:

To determine the percent identity of two amino acid sequences, or of two nucleic acid sequences, the sequences are aligned for optimal comparison purposes (*e.g.,* gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence (*e.g.,* when aligning a second sequence to the 44590 amino acid sequence of SEQ ID NO:2 having 1930 amino acid residues, at least 579, preferably at least 772 , more preferably at least 965 even more preferably at least 1158, and even more preferably at least 1351, 1544, or 1737 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (1970) *J. Mol. Biol.* 48:444-453 algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a Blossum 62 scoring matrix with a gap penalty of 12, a gap extend penalty of 4, and a frameshift gap penalty of 5.

The percent identity between two amino acid or nucleotide sequences can be determined using the algorithm of Meyers and Miller ((1989) CABIOS, 4:11-17 which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul *et al.* (1990) *J. Mol. Biol.* 215:403-10. BLAST nucleotide searches can be performed with the NBLAST program, score = 100, wordlength = 12 to obtain nucleotide sequences homologous to 44590 nucleic acid molecules of the invention. BLAST protein searches can be performed with the XBLAST program, score = 50, wordlength = 3 to obtain amino acid sequences homologous to 44590 protein molecules of the invention. To obtain gapped alignments for comparison purposes, Gapped BLAST can be utilized as described in Altschul *et al*., (1997) *Nucleic Acids Res.* 25:3389-3402. When utilizing BLAST and Gapped BLAST programs, the default parameters of the respective programs (*e*.*g*., XBLAST and NBLAST) can be used.

Particular 44590 polypeptides of the present invention have an amino acid sequence substantially identical to the amino acid sequence of SEQ ID NO:2. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 90%. 91%, 92%, 93%, 94%, 95%, 96%, 97%, 98% or 99% identity to SEQ ID NO:2 are termed substantially identical.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 60%, or 65% identity, likely 75% identity, more likely 85%, 90%. 91%, 92%, 93%, 94%, 95%, 96%, 97%, 98% or 99% identity to SEQ ID NO:1 or 3 are termed substantially identical.

"Misexpression or aberrant expression", as used herein, refers to a non-wild type pattern of gene expression, at the RNA or protein level. It includes: expression at non-wild type levels, i.e., over or under expression; a pattern of expression that differs from wild type in terms of the time or stage at which the gene is expressed, *e.g.,* increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, *e.g.,* a pattern of increased or decreased expression (as compared with wild type) in the presence of an increase or decrease in the strength of the stimulus.

"Subject", as used herein, can refer to a mammal, *e*.*g*., a human, or to an experimental or animal or disease model. The subject can also be a non-human animal, *e.g.,* a horse, cow, goat, or other domestic animal.

A "purified preparation of cells", as used herein, refers to, in the case of plant or animal cells, an *in vitro* preparation of cells and not an entire intact plant or animal. In the case of cultured cells or microbial cells, it consists of a preparation of at least 10% and more preferably 50% of the subject cells.

Various aspects of the invention are described in further detail below.

### Isolated Nucleic Acid Molecules

In one aspect, the invention provides, an isolated or purified, nucleic acid molecule that encodes a 44590 polypeptide described herein, *e.g.,* a full length 44590 protein or a fragment thereof, *e.g.,* a biologically active portion of 44590 protein. Also included is a nucleic acid fragment suitable for use as a hybridization probe, which can be used, *e.g.,* to identify a nucleic acid molecule encoding a polypeptide of the invention, 44590 mRNA, and fragments suitable for use as primers, *e.g.,* PCR primers for the amplification or mutation of nucleic acid molecules.

In one embodiment, an isolated nucleic acid molecule of the invention includes the nucleotide sequence shown in SEQ ID NO:1, or a portion of any of this nucleotide sequence. In one embodiment, the nucleic acid molecule includes sequences encoding the human 44590 protein (i.e., "the coding region" of SEQ ID NO:1, as shown in SEQ ID NO:3), as well as 5' untranslated sequences (nucleotides 1 to 125 of SEQ ID NO:1) and 3' untranslated sequences (nucleotides 5919 to 5793 of SEQ ID NO:1). Alternatively, the nucleic acid molecule can include only the coding region of SEQ ID NO:1 *(e.g.,* SEQ ID NO:3) and, *e.g*., no flanking sequences which normally accompany the subject sequence. In another embodiment, the nucleic acid molecule encodes a sequence corresponding to a fragment of the protein from about amino acid 706 to 888, and amino acids 1618 to 1800, of SEQ ID NO:2.

In another embodiment, an isolated nucleic acid molecule of the invention includes a nucleic acid molecule which is a complement of the nucleotide sequence shown in SEQ ID NO:1 or SEQ ID NO:3, or a portion of any of these nucleotide sequences. In other embodiments, the nucleic acid molecule of the invention is sufficiently complementary to the nucleotide sequence shown in SEQ ID NO:1 or SEQ ID NO:3 such that it can hybridize to the nucleotide sequence shown in SEQ ID NO: 1 or 3, thereby forming a stable duplex.

In one embodiment, an isolated nucleic acid molecule of the present invention includes a nucleotide sequence which is at least about: 60%, 65%, 70%, 75%, 80%, 85%, 90%, 91%, 92%, 93%, 94%, 95%, 96%, 97%, 98%, 99%, or more homologous to the entire length of the nucleotide sequence shown in SEQ ID NO:1 or SEQ ID NO:3, or a portion, preferably of the same length, of any of these nucleotide sequences.

### 44590 Nucleic Acid Fragments

A nucleic acid molecule of the invention can include only a portion of the nucleic acid sequence of SEQ ID NO:1 or 3. For example, such a nucleic acid molecule can include a fragment which can be used as a probe or primer or a fragment encoding a portion of a 44590 protein, e.g., an immunogenic or biologically active portion of a 44590 protein. A fragment can comprise those nucleotides of SEQ ID NO:1, which encode an ABC transporter domain of human 44590. The nucleotide sequence determined from the cloning of the 44590 gene allows for the generation of probes and primers designed for use in identifying and/or cloning other 44590 family members, or fragments thereof, as well as 44590 homologs, or fragments thereof, from other species.

In another embodiment, a nucleic acid includes a nucleotide sequence that includes part, or all, of the coding region and extends into either (or both) the 5' or 3' noncoding region. Other embodiments include a fragment which includes a nucleotide sequence encoding an amino acid fragment described herein. Nucleic acid fragments can encode a specific domain or site described herein or fragments thereof, particularly fragments thereof which are at least 100 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 44590 nucleic acid fragment can include a sequence corresponding to an ABC transporter domain, as described herein.

44590 probes and primers are provided. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30, 35, 40, 45, 50, 55, 60, 65, or 75 consecutive nucleotides of a sense or antisense sequence of SEQ ID NO:1 or SEQ ID NO:3, or of a naturally occurring allelic variant or mutant of SEQ ID NO:1 or SEQ ID NO:3.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes: ABC transporter domains (amino acid residues 706 to 888 and 1618 to 1800 of SEQ ID NO:2); and a SR54-type protein, GTPase domains (amino acid residues 711 to 723 and 1623 to 1633 of SEQ ID NO:2).

In another embodiment a set of primers is provided, *e*.*g*., primers suitable for use in a PCR, which can be used to amplify a selected region of a 44590 sequence, *e.g.,* a domain, region, site or other sequence described herein. The primers should be at least 5, 10, or 50 base pairs in length and less than 100, or less than 200, base pairs in length. The primers should be identical, or differ by one base from a sequence disclosed herein or from a naturally occurring variant. For example, primers suitable for amplifying all or a portion of any of the following regions are provided: an ABC transporter domain from about amino acid 706 to 888, and amino acids 1618 to 1800, of SEQ ID NO:2, and a SR54-type protein, GTPase domain (amino acid residues 711 to 723 and 1623 to 1633 of SEQ ID NO:2)

A nucleic acid fragment can encode an epitope bearing region of a polypeptide described herein.

A nucleic acid fragment encoding a "biologically active portion of a 44590 polypeptide" can be prepared by isolating a portion of the nucleotide sequence of SEQ ID NO:1 or 3, which encodes a polypeptide having a 44590 biological activity (*e*.*g*., the biological activities of the 44590 proteins are described herein), expressing the encoded portion of the 44590 protein (*e.g.*, by recombinant expression *in vitro*) and assessing the activity of the encoded portion of the 44590 protein. For example, a nucleic acid fragment encoding a biologically active portion of 44590 includes an ABC transporter domain, *e.g.,* amino acid residues about 706 to 888, and amino acids 1618 to 1800, of SEQ ID NO:2. A nucleic acid fragment encoding a biologically active portion of a 44590 polypeptide, can comprise a nucleotide sequence which is greater than 300 or more nucleotides in length.

In preferred embodiments, a nucleic acid includes a nucleotide sequence which is about 250, 500, 750, 1000, 1250, 1300, 1310, 1320, 1330, 1340, 1350, 1360, 1370, 1380, 1390, 1400, 1410, 1420, 1430, 1440, 1450, 1500, 1750, 2000, 2250, 2500, 2750, 3000, 3250, 3500, 4000, 4250, 4500, 4750, 5000, 5250, 5500, 5790 or more nucleotides in length and hybridizes under stringent hybridization conditions to a nucleic acid molecule of SEQ ID NO:1 or SEQ ID NO:3.

### 44590 Nucleic Acid Variants

The invention further encompasses nucleic acid molecules that differ from the nucleotide sequence shown in SEQ ID NO:1 or SEQ ID NO:3. Such differences can be due to degeneracy of the genetic code (and result in a nucleic acid which encodes the same 44590 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2. If alignment is needed for this comparison the sequences should be aligned for maximum homology. "Looped" out sequences from deletions or insertions, or mismatches, are considered differences.

Nucleic acids of the inventor can be chosen for having codons, which are preferred, or non-preferred, for a particular expression system. *E.g.,* the nucleic acid can be one in which at least one codon, at preferably at least 10%, or 20% of the codons has been altered such that the sequence is optimized for expression in *E. coli,* yeast, human, insect, or CHO cells.

Nucleic acid variants can be naturally occurring, such as allelic variants (same locus), homologs (different locus), and orthologs (different organism) or can be non-naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO: 1 or 3, *e.g*., as follows: by at least one but less than 10, 20, 30, or 40 nucleotides; at least one but less than 1%, 5%, 10% or 20% of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the nucleotide sequence shown in SEQ ID NO:2 or a fragment of this sequence. Such nucleic acid molecules can readily be identified as being able to hybridize under stringent conditions, to the nucleotide sequence shown in SEQ ID NO 2 or a fragment of the sequence. Nucleic acid molecules corresponding to orthologs, homologs, and allelic variants of the 44590 cDNAs of the invention can further be isolated by mapping to the same chromosome or locus as the 44590 gene.

Preferred variants include those that are correlated with the ability to: reside within a membrane; dimerize with a second transporter, *e.g*., an ABC transporter; bind and hydrolyze a nucleotide, *e.g.,* ATP or GTP; or bind and transport a molecule *e.g.,* an amino acid, a peptide, an ion, a sugar, a nucleic acid, a signaling molecule, a vitamin, or a cofactor across a membrane, *e.g.,* a cell or organelle membrane. Allelic variants of 44590, *e.g.,* human 44590, include both functional and non-functional proteins. Functional allelic variants are naturally occurring amino acid sequence variants of the 44590 protein within a population that maintain the ability the ability to: reside within a membrane; dimerize with a second transporter, *e.g.,* an ABC transporter; bind and hydrolyze a nucleotide, *e.g.,* ATP or GTP; or bind and transport a molecule *e.g*., an amino acid, a peptide, an ion, a sugar, a nucleic acid, a signaling molecule, a vitamin, or a cofactor across a membrane, *e.g.,* a cell or organelle membrane. Functional allelic variants will typically contain only conservative substitution of one or more amino acids of SEQ ID NO:2, or substitution, deletion or insertion of non-critical residues in non-critical regions of the protein. Non-functional allelic variants are naturally-occurring amino acid sequence variants of the 44590, *e.g.*, human 44590, protein within a population that do not have the ability to the ability to: reside within a membrane; dimerize with a second transporter, *e.g.,* an ABC transporter; bind and hydrolyze a nucleotide, *e.g.,* ATP or GTP; or bind and transport a molecule *e.g.,* an amino acid, a peptide, an ion, a sugar, a nucleic acid, a signaling molecule, a vitamin, or a cofactor across a membrane, *e.g.,* a cell or organelle membrane. Non-functional allelic variants will typically contain a non-conservative substitution, a deletion, or insertion, or premature truncation of the amino acid sequence of SEQ ID NO:2, or a substitution, insertion, or deletion in critical residues or critical regions of the protein.

Moreover, nucleic acid molecules encoding other 44590 family members and, thus, which have a nucleotide sequence which differs from the 44590 sequences of SEQ ID NO:1 or SEQ ID NO:3 are intended to be within the scope of the invention.

### Antisense Nucleic Acid Molecules, Ribozymes and Modified 44590 Nucleic Acid Molecules

In another aspect, the invention features, an isolated nucleic acid molecule which is antisense to 44590. An "antisense" nucleic acid can include a nucleotide sequence which is complementary to a "sense" nucleic acid encoding a protein, *e.g.,* complementary to the coding strand of a double-stranded cDNA molecule or complementary to an mRNA sequence. The antisense nucleic acid can be complementary to an entire 44590 coding strand, or to only a portion thereof (*e.g*., the coding region of human 44590 corresponding to SEQ ID NO:3). In another embodiment, the antisense nucleic acid molecule is antisense to a "noncoding region" of the coding strand of a nucleotide sequence encoding 44590 *(e.g.,* the 5' and 3' untranslated regions).

An antisense nucleic acid can be designed such that it is complementary to the entire coding region of 44590 mRNA, but more preferably is an oligonucleotide which is antisense to only a portion of the coding or noncoding region of 44590 mRNA. For example, the antisense oligonucleotide can be complementary to the region surrounding the translation start site of 44590 mRNA, *e.g.,* between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid *(e.g.,* an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.,* phosphorothioate derivatives and acridine substituted nucleotides can be used. The antisense nucleic acid also can be produced biologically using an expression vector into which a nucleic acid has been subcloned in an antisense orientation (*i.e*., RNA transcribed from the inserted nucleic acid will be of an antisensc orientation to a target nucleic acid of interest, described further in the following subsection).

The antisense nucleic acid molecules of the invention are typically administered to a subject *(e.g.,* by direct injection at a tissue site), or generated *in situ* such that they hybridize with or bind to cellular mRNA and/or genomic DNA encoding a 44590 protein to thereby inhibit expression of the protein, *e.g.,* by inhibiting transcription and/or translation. Alternatively, antisense nucleic acid molecules can be modified to target selected cells and then administered systemically. For systemic administration, antisense molecules can be modified such that they specifically or selectively bind to receptors or antigens expressed on a selected cell surface, *e.g.,* by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β -units, the strands run parallel to each other (Gaultier *et al.* (1987) *Nucleic Acids. Res.* 15:6625-6641). The antisense nucleic acid molecule can also comprise a 2'-o-methylribonucleotide (Inoue *et al.* (1987) *Nucleic Acids Res.* 15:6131-6148) or a chimeric RNA-DNA analogue (Inoue *et al.* (1987) *FEBS Lett.* 215:327-330).

In still another embodiment, an antisense nucleic acid of the invention is a ribozyme. A ribozyme having specificity for a 44590-encoding nucleic acid can include one or more sequences complementary to the nucleotide sequence of a 44590 cDNA disclosed herein (i.e., SEQ ID NO:1 or SEQ ID NO:3), and a sequence having known catalytic sequence responsible for mRNA cleavage (see U.S. Pat. No. 5,093,246 or Haselhoff and Gerlach (1988) *Nature* 334:585-591). For example, a derivative of a *Tetrahymena* L-19 IVS RNA can be constructed in which the nucleotide sequence of the active site is complementary to the nucleotide sequence to be cleaved in a 44590-encoding mRNA. See, *e.g.*, Cech *et al.* U.S. Patent No. 4,987,071; and Cech *et al.* U.S. Patent No. 5,116,742. Alternatively, 44590 mRNA can be used to select a catalytic RNA having a specific ribonuclease activity from a pool of RNA molecules. See, *e.g.,* Bartel and Szostak (1993) *Science* 261:1411-1418.

44590 gene expression can be inhibited by targeting nucleotide sequences complementary to the regulatory region of the 44590 *(e.g.,* the 44590 promoter and/or enhancers) to form triple helical structures that prevent transcription of the 44590 gene in target cells. See generally, Helene (1991) *Anticancer Drug Des*. 6:569-84; Helene (1992) *Ann. N.Y. Acad. Sci.* 660:27-36; and Maher (1992) *Bioussays* 14:807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

A 44590 nucleic acid molecule can be modified at the base moiety, sugar moiety or phosphate backbone to improve, *e.g.*, the stability, hybridization, or solubility of the molecule. For example, the deoxyribose phosphate backbone of the nucleic acid molecules can be modified to generate peptide nucleic acids (see Hyrup *et al.* (1996) *Bioorganic & Medicinal Chemistry* 4: 5-23). As used herein, the terms "peptide nucleic acid" or "PNA" refers to a nucleic acid mimic, *e.g.,* a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup *et al*. (1996) *supra*; Perry-O'Keefe *et al.* (1996) *Proc. Natl. Acad. Sci*. 93: 14670-675.

PNAs of 44590 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 44590 nucleic acid molecules can also be used in the analysis of single base pair mutations in a gene, *(e.g.,* by PNA-directed PCR clamping); as 'artificial restriction enzymes' when used in combination with other enzymes, (*e.g*., S1 nucleases (Hyrup *et al.* (1996) *supra*)); or as probes or primers for DNA sequencing or hybridization (Hyrup *et al*. (1996) *supra*; Perry-O'Keefe *supra*).

In other embodiments, the oligonucleotide can include other appended groups such as peptides (*e.g.*, for targeting host cell receptors *in vivo*), or agents facilitating transport across the cell membrane (see, *e.g.,* Letsinger *et al*. (1989) *Proc. Natl. Acad. Sci. USA* 86:6553-6556; Lemaitre *et al.* (1987) *Proc. Natl. Acad. Sci. USA* 84:648-652; PCT Publication No. WO88/09810) or the blood-brain barrier (see, *e.g.,* PCT Publication No. W089/10134). In addition, oligonucleotides can be modified with hybridization-triggered cleavage agents (see, *e.g.,* Krol *et al*. (1988) *Bio-Techniques* 6:958-976) or intercalating agents. (see, *e.g.,* Zon (1988) *Pharm. Res.* 5:539-549). To this end, the oligonucleotide can be conjugated to another molecule, *(e.g.,* a peptide, hybridization triggered cross-linking agent, transport agent, or hybridization-triggered cleavage agent).

The invention also includes molecular beacon oligonucleotide primer and probe molecules having at least one region which is complementary to a 44590 nucleic acid of the invention, two complementary regions one having a fluorophore and one a quencher such that the molecular beacon is useful for quantitating the presence of the 44590 nucleic acid of the invention in a sample. Molecular beacon nucleic acids are described, for example, in Lizardi *et al*., U.S. Patent No. 5,854,033; Nazarenko *et al*., U.S. Patent No. 5,866,336, and Livak *et al*., U.S. Patent 5,876,930.

### Isolated 44590 Polypeptides

In another aspect, the invention features, an isolated 44590 protein, or fragment, *e.g.,* a biologically active portion, for use as immunogens or antigens to raise or test (or more generally to bind) anti-44590 antibodies. 44590 protein can be isolated from cells or tissue sources using standard protein purification techniques. 44590 protein or fragments thereof can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, *e.g.,* cultured cells, which result in substantially the same post-translational modifications present when the polypeptide is expressed in a native cell, or in systems which result in the alteration or omission of post-translational modifications, *e.g.,* glycosylation or cleavage, present in a native cell.

In a preferred embodiment, a 44590 polypeptide has one or more of the following characteristics:

it has the ability to reside within a membrane, *e.g.,* a cell or organelle membrane;

it has the ability to dimerize with a second transporter, *e.g.,* an ABC transporter;

it has the ability to bind and hydrolyze a nucleotide, *e.g.,* ATP or GTP;

it has the ability to bind and transport a molecule *e.g.,* an amino acid, a peptide, an ion, a sugar, a nucleic acid, a signaling molecule, a vitamin, or a cofactor across a membrane, *e.g.,* a cell or organelle membrane;

it has a molecular weight, *e.g.,* a deduced molecular weight, preferably ignoring any contribution of post translational modifications, amino acid composition or other physical characteristic of a 44590 polypeptide, *e.g.,* a polypeptide of SEQ ID NO:2;

it has an overall sequence similarity of at least 60%, preferably at least 70%, more preferably at least 80, 90, or 95%, with a polypeptide of SEQ ID NO:2;

it can be found in brain or testes;

it has at least two ABC transporter domains which are preferably about 70%, 80%, 90% or 95% identical to amino acid residues about 706 to 888, and amino acids 1618 to 1800, of SEQ ID NO:2;

In a preferred embodiment the 44590 protein, or fragment thereof, differs from the corresponding sequence in SEQ ID NO:2. In one embodiment it differs by at least one but by less than 15, 10 or 5 amino acid residues. In another it differs from the corresponding sequence in SEQ ID NO:2 by at least one residue but less than 20%, 15%, 10% or 5% of the residues in it differ from the corresponding sequence in SEQ ID NO:2. (If this comparison requires alignment the sequences should be aligned for maximum homology. "Looped" out sequences from deletions or insertions, or mismatches, are considered differences.) The differences are, preferably, differences or changes at a non-essential residue or a conservative substitution. In a preferred embodiment the differences are not in the ABC transporter domains located at about residues 706 to 888, and amino acids 1618 to 1800, of SEQ ID NO:2. In another embodiment one or more differences are in ABC transporter domains located at about residues 706 to 888, and amino acids 1618 to 1800, of SEQ ID NO:2.

Other embodiments include a protein that contains one or more changes in amino acid sequence, *e.g*., a change in an amino acid residue which is not essential for activity. Such 44590 proteins differ in amino acid sequence from SEQ ID NO:2, yet retain biological activity.

In one embodiment, the protein includes an amino acid sequence at least about 60%, 65%, 70%, 75%, 80%, 85%, 90%, 95%, 98% or more homologous to SEQ ID NO:2.

A 44590 protein or fragment is provided which varies from the sequence of SEQ ID NO:2 in regions defined by amino acids about 1 to 395 by at least one but by less than 15, 10 or 5 amino acid residues in the protein or fragment but which does not differ from SEQ ID NO:2 in regions defined by amino acids about 706 to 888, and amino acids 1618 to 1800,. (If this comparison requires alignment the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one embodiment, a biologically active portion of a 44590 protein includes an ABC transporter domain. Moreover, other biologically active portions, in which other regions of the protein are deleted, can be prepared by recombinant techniques and evaluated for one or more of the functional activities of a native 44590 protein.

In a preferred embodiment, the 44590 protein has an amino acid sequence shown in SEQ ID NO:2. In other embodiments, the 44590 protein is sufficiently or substantially identical to SEQ ID NO:2. In yet another embodiment, the 44590 protein is sufficiently or substantially identical to SEQ ID NO:2 and retains the functional activity of the protein of SEQ ID NO:2, as described in detail in the subsections above.

### 44590 Chimeric or Fusion Proteins

In another aspect, the invention provides 44590 chimeric or fusion proteins. As used herein, a 44590 "chimeric protein" or "fusion protein" includes a 44590 polypeptide linked to a non-44590 polypeptide. A "non-44590 polypeptide" refers to a polypeptide having an amino acid sequence corresponding to a protein which is not substantially homologous to the 44590 protein, *e.g.*, a protein which is different from the 44590 protein and which is derived from the same or a different organism. The 44590 polypeptide of the fusion protein can correspond to all or a portion *e.g.,* a fragment described herein of a 44590 amino acid sequence. In a preferred embodiment, a 44590 fusion protein includes at least one (or two) biologically active portion of a 44590 protein. The non-44590 polypeptide can be fused to the N-terminus or C-terminus of the 44590 polypeptide.

The fusion protein can include a moiety which has a high affinity for a ligand. For example, the fusion protein can be a GST-44590 fusion protein in which the 44590 sequences are fused to the C-terminus of the GST sequences. Such fusion proteins can facilitate the purification of recombinant 44590. Alternatively, the fusion protein can be a 44590 protein containing a heterologous signal sequence at its N-terminus. In certain host cells (*e*.*g*., mammalian host cells), expression and/or secretion of 44590 can be increased through use of a heterologous signal sequence.

Fusion proteins can include all or a part of a serum protein, *e*.*g*., a portion of an immunoglobulin (*e*.*g*., IgG, IgA, or IgE), *e*.*g*., an Fc region and/or the hinge C1 and C2 sequences of an immunoglobulin or human serum albumin.

The 44590 fusion proteins of the invention can be incorporated into pharmaceutical compositions and administered to a subject *in vivo*. The 44590 fusion proteins can be used to affect the bioavailability of a 44590 substrate. 44590 fusion proteins can be useful therapeutically for the treatment of disorders caused by, for example, (i) aberrant modification or mutation of a gene encoding a 44590 protein; (ii) mis-regulation of the 44590 gene; and (iii) aberrant post-translational modification of a 44590 protein.

Moreover, the 44590-fusion proteins of the invention can be used as immunogens to produce anti-44590 antibodies in a subject, to purify 44590 ligands and in screening assays to identify molecules which inhibit the interaction of 44590 with a 44590 substrate.

Expression vectors are commercially available that already encode a fusion moiety (*e.g.*, a GST polypeptide). A 44590-encoding nucleic acid can be cloned into such an expression vector such that the fusion moiety is linked in-frame to the 44590 protein.

### Variants of 44590 Proteins

In another aspect, the invention also features a variant of a 44590 polypeptide, *e.g.,* which functions as an agonist (mimetics) or as an antagonist. Variants of the 44590 proteins can be generated by mutagenesis, *e.g.,* discrete point mutation, the insertion or deletion of sequences or the truncation of a 44590 protein. An agonist of the 44590 proteins can retain substantially the same, or a subset, of the biological activities of the naturally occurring form of a 44590 protein. An antagonist of a 44590 protein can inhibit one or more of the activities of the naturally occurring form of the 44590 protein by, for example, competitively modulating a 44590-mediated activity of a 44590 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 44590 protein.

Variants of a 44590 protein can be identified by screening combinatorial libraries of mutants, *e*.*g*., truncation mutants, of a 44590 protein for agonist or antagonist activity.

Libraries of fragments *e*.*g*., N terminal, C terminal, or internal fragments, of a 44590 protein coding sequence can be used to generate a variegated population of fragments for screening and subsequent selection of variants of a 44590 protein.

Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property are known in the art. Recursive ensemble mutagenesis (REM), a new technique which enhances the frequency of functional mutants in the libraries, can be used in combination with the screening assays to identify 44590 variants (Arkin and Yourvan (1992) *Proc. Natl. Acad. Sci. USA* 89:7811-7815; Delgrave *et al.* (1993) *Protein Engineering* 6:327-331).

Cell based assays can be exploited to analyze a variegated 44590 library. For example, a library of expression vectors can be transfected into a cell line, *e.g.,* a cell line, which ordinarily responds to 44590 in a substrate-dependent manner. The transfected cells are then contacted with 44590 and the effect of the expression of the mutant on signaling by the 44590 substrate can be detected, *e*.*g*., by measuring the ability to: reside within a membrane; dimerize with a second transporter, *e.g.*, an ABC transporter; bind and hydrolyze a nucleotide, *e.g.,* ATP or GTP; or bind and transport a molecule *e.g.,* an amino acid, a peptide, an ion, a sugar, a nucleic acid, a signaling molecule, a vitamin, or a cofactor across a membrane, *e.g.,* a cell or organelle membrane. Plasmid DNA can then be recovered from the cells which score for inhibition, or alternatively, potentiation of signaling by the 44590 substrate, and the individual clones further characterized.

In another aspect, the invention features a method of making a 44590 polypeptide, *e*.*g*., a peptide having a non-wild type activity, *e.g.,* an antagonist, agonist, or super agonist of a naturally occurring 44590 polypeptide, *e.g.,* a naturally occurring 44590 polypeptide. The method includes altering the sequence of a 44590 polypeptide, *e.g.,* altering the sequence, *e.g*., by substitution or deletion of one or more residues of a non-conserved region, a domain or residue disclosed herein, and testing the altered polypeptide for the desired activity.

In another aspect, the invention features a method of making a fragment or analog of a 44590 polypeptide a biological activity of a naturally occurring 44590 polypeptide. The method includes altering the sequence, *e.g.,* by substitution or deletion of one or more residues, of a 44590 polypeptide, *e.g.,* altering the sequence of a non-conserved region, or a domain or residue described herein, and testing the altered polypeptide for the desired activity.

### Anti-44590 Antibodies

In another aspect, the invention provides an anti-44590 antibody. The term "antibody" as used herein refers to an immunoglobulin molecule or immunologically active portion thereof, i.e., an antigen-binding portion. Examples of immunologically active portions of immunoglobulin molecules include scFV and dcFV fragments, Fab and F(ab')₂ fragments which can be generated by treating the antibody with an enzyme such as papain or pepsin, respectively.

The antibody can be a polyclonal, monoclonal, recombinant, *e.g.,* a chimeric or humanized, fully human, non-human, *e.g.,* murine, or single chain antibody. In a preferred embodiment it has effector function and can fix complement. The antibody can be coupled to a toxin or imaging agent.

A full-length 44590 protein or, antigenic peptide fragment of 44590 can be used as an immunogen or can be used to identify anti-44590 antibodies made with other immunogens, *e.g.,* cells, membrane preparations, and the like. The antigenic peptide of 44590 should include at least 8 amino acid residues of the amino acid sequence shown in SEQ ID NO:2 and encompasses an epitope of 44590. Preferably, the antigenic peptide includes at least 10 amino acid residues, more preferably at least 15 amino acid residues, even more preferably at least 20 amino acid residues, and most preferably at least 30 amino acid residues.

Fragments of 44590 which include residues about 440 to 460, about 1080 to 1090 or about 1370 to 1385 of SEQ ID NO:2 can be used to make, *e.g.,* used as immunogens or used to characterize the specificity of an antibody, antibodies against hydrophilic regions of the 44590 protein (see Figure 1). Similarly, fragments of 44590 which include residues about 270 to 280, about 780 to 795, or about 1190 to 1200 of SEQ ID NO:2 can be used to make an antibody against a hydrophobic region of the 44590 protein; fragments of 44590 which include residues about 421 to 445, about 505 to 510, about 607 to 722, about 1103 to 1321, about 1392 to 1401, or about 1459 to 1520 or a subset thereof, e.g. about residues 607 to 722, or about 1103 to 1321 of SEQ ID NO:2 can be used to make an antibody against an extracellular region of the 44590 protein; fragments of 44590 which include residues about 469 to 479, about 532 to 584, about 741 to 1080, about 1345 to 1373, or about 1427 to 1435, of SEQ ID NO:2 can be used to make an antibody against an intracellular region of the 44590 protein; a fragment of 44590 which includes residues about 706 to 750, about 751 to 800, about 801 to 850, about 851 to 888, about 1618 to 1650, about 1651 to 1700, about 1701 to 1750, or about 1751 to 1800 to of SEQ ID NO:2 can be used to make an antibody against the ABC transporter region of the 44590 protein.

Antibodies reactive with, or specific or selective for, any of these regions, or other regions or domains described herein are provided.

Preferred epitopes encompassed by the antigenic peptide are regions of 44590 located on the surface of the protein, *e.g.,* hydrophilic regions, as well as regions with high antigenicity. For example, an Emini surface probability analysis of the human 44590 protein sequence can be used to indicate the regions that have a particularly high probability of being localized to the surface of the 44590 protein and are thus likely to constitute surface residues useful for targeting antibody production.

In a preferred embodiment the antibody can bind to the extracellular portion of the 44590 protein, *e.g.*, it can bind to a whole cell which expresses the 44590 protein. In another embodiment, the antibody binds an intracellular portion of the 44590 protein.

In a preferred embodiment the antibody binds an epitope on any domain or region on 44590 proteins described herein.

Additionally, chimeric, humanized, and completely human antibodies are also within the scope of the invention. Chimeric, humanized, but most preferably, completely human antibodies are desirable for applications which include repeated administration, *e.g.,* therapeutic treatment of human patients, and some diagnostic applications.

Chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson *et al.* International Application No. PCT/US86/02269; Akira, *et al.* European Patent Application 184,187; Taniguchi, European Patent Application 171,496; Morrison *et al.* European Patent Application 173,494; Neuberger *et al.* PCT International Publication No. WO 86/01533; Cabilly *et al.* U.S. Patent No. 4,816,567; Cabilly *et al.* European Patent Application 125,023; Better *et al.* (1988) *Science* 240:1041-1043; Liu *et al.* (1987) *Proc. Natl. Acad. Sci. USA* 84:3439-3443; Liu *et al.* (1987) *J. Immunol.* 139:3521-3526; Sun *et al.* (1987) *Proc. Natl. Acad. Sci. USA* 84:214-218; Nishimura *et al.* (1987) *Canc. Res.* 47:999-1005; Wood *et al.* (1985) *Nature* 314:446-449; and Shaw *et al.* (1988) *J*. *Natl. Cancer Inst.* 80:1553-1559).

A humanized or complementarity determining region (CDR)-grafted antibody will have at least one or two, but generally all three recipient CDR's (of heavy and or light immuoglobulin chains) replaced with a donor CDR. The antibody may be replaced with at least a portion of a non-human CDR or only some of the CDR's may be replaced with non-human CDR's. It is only necessary to replace the number of CDR's required for binding of the humanized antibody to a 44590 or a fragment thereof. Preferably, the donor will be a rodent antibody, *e.g.,* a rat or mouse antibody, and the recipient will be a human framework or a human consensus framework. Typically, the immunoglobulin providing the CDR's is called the "donor" and the immunoglobulin providing the framework is called the "acceptor." In one embodiment, the donor immunoglobulin is a non-human (*e.g.*, rodent). The acceptor framework is a naturally-occurring (*e.g.*, a human) framework or a consensus framework, or a sequence about 85% or higher, preferably 90%, 95%, 99% or higher identical thereto.

As used herein, the term "consensus sequence" refers to the sequence formed from the most frequently occurring amino acids (or nucleotides) in a family of related sequences (See *e.g.,* Winnaker, (1987) *From Genes to Clones* (Verlagsgesellschaft, Weinheim, Germany). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody can be humanized by methods known in the art. Humanized antibodies can be generated by replacing sequences of the Fv variable region which are not directly involved in antigen binding with equivalent sequences from human Fv variable regions. General methods for generating humanized antibodies are provided by Morrison (1985) *Science* 229:1202-1207, by Oi *et al.* (1986) *BioTechniques* 4:214, and by Queen *et* *al.* US patent Nos. 5,585,089, 5,693,761 and 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a 44590 polypeptide or fragment thereof. The recombinant DNA encoding the humanized antibody, or fragment thereof, can then be cloned into an appropriate expression vector.

Humanized or CDR-grafted antibodies can be produced by CDR-grafting or CDR substitution, wherein one, two, or all CDR's of an immunoglobulin chain can be replaced. See *e*.*g*., U.S. Patent No. 5,225,539; Jones *et al.* (1986) *Nature* 321:552-525; Verhoeyan *et al.* (1988) *Science* 239:1534; Beidler *et al.* (1988) *J. Immunol.* 141:4053-4060; Winter US patent No. 5,225,539, the contents of all of which are hereby expressly incorporated by reference. Winter describes a CDR-grafting method which may be used to prepare the humanized antibodies of the present invention (UK Patent Application GB 2188638A, filed on March 26, 1987; Winter US patent No. 5,225,539), the contents of which is expressly incorporated by reference.

Also within the scope of the invention are humanized antibodies in which specific amino acids have been substituted, deleted or added. Preferred humanized antibodies have amino acid substitutions in the framework region, such as to improve binding to the antigen. For example, a humanized antibody will have framework residues identical to the donor framework residue or to another amino acid other than the recipient framework residue. To generate such antibodies, a selected, small number of acceptor framework residues of the humanized immunoglobulin chain can be replaced by the corresponding donor amino acids. Preferred locations of the substitutions include amino acid residues adjacent to the CDR, or which are capable of interacting with a CDR (see *e.g.,* US patent No. 5,585,089). Criteria for selecting amino acids from the donor are described in US 5,585,089, *e.g.,* columns 12-16 of US 5,585,089, the *e.g.,* columns 12-16 of US 5,585,089, the contents of which are hereby incorporated by reference. Other techniques for humanizing antibodies are described in Padlan *et al.* EP 519596 A1, published on December 23, 1992.

Completely human antibodies are particularly desirable for therapeutic treatment of human patients. Such antibodies can be produced using transgenic mice that are incapable of expressing endogenous immunoglobulin heavy and light chains genes, but which can express human heavy and light chain genes. *See,* for example, Lonberg and Huszar (1995) *Int. Rev. Immunol. 13*:65-93); and U.S. Patent Nos. 5,625,126; 5,633,425; 5,569,825; 5,661,016; and 5,545,806. In addition, companies such as Abgenix, Inc. (Fremont, CA) and Medarex, Inc. (Princeton, NJ), can be engaged to provide human antibodies directed against a selected antigen using technology similar to that described above.

Completely human antibodies that recognize a selected epitope can be generated using a technique referred to as "guided selection." In this approach a selected non-human monoclonal antibody, *e.g.,* a murine antibody, is used to guide the selection of a completely human antibody recognizing the same epitope. This technology is described by Jespers *et al.* (1994) *Bio*/*Technology 12*:899-903).

The anti-44590 antibody can be a single chain antibody. A single-chain antibody (scFV) can be engineered as described in, for example, Colcher *et al.* (1999) *Ann. N Y Acad. Sci.* 880:263-80; and Reiter (1996) *Clin. Cancer Res.* 2:245-52. The single chain antibody can be dimerized or multimerized to generate multivalent antibodies having specificities for different epitopes of the same target 44590 protein.

In a preferred embodiment, the antibody has reduced or no ability to bind an Fc receptor. For example, it is an isotype or subtype, fragment or other mutant, which does not support binding to an Fc receptor, *e.g*., it has a mutagenized or deleted Fe receptor binding region.

An antibody (or fragment thereof) may be conjugated to a therapeutic moiety such as a cytotoxin, a therapeutic agent or a radioactive ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1 -dehydrotestosterone, glucocorticoids, procaine, tetracaine, lidocaine, propranolol, puromycin, maytansinoids, *e*.*g*., maytansinol (see US Patent No. 5,208,020), CC-1065 (see US Patent Nos. 5,475,092, 5,585,499, 5,846,545) and analogs or homologs thereof. Therapeutic agents include, but are not limited to, antimetabolites (*e.g.*, methotrexate, 6-mercaptopurine, 6-thioguanine, cytarabine, 5-fluorouracil decarbazine), alkylating agents (*e.g.,* mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines *(e.g.,* daunorubicin (formerly daunomycin) and doxorubicin), antibiotics *(e.g.,* dactinomycin (formerly actinomycin), bleomycin, mithramycin, and anthramycin (AMC)), and anti-mitotic agents (*e.g*., vincristine, vinblastine, taxol and maytansinoids). Radioactive ions include, but are not limited to iodine, yttrium and praseodymium.

The conjugates of the invention can be used for modifying a given biological response, the therapeutic moiety is not to be construed as limited to classical chemical therapeutic agents. For example, the therapeutic moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

An anti-44590 antibody *(e.g.,* monoclonal antibody) can be used to isolate 44590 by standard techniques, such as affinity chromatography or immunoprecipitation. Moreover, an anti-44590 antibody can be used to detect 44590 protein *(e.g.,* in a cellular lysate or cell supernatant) in order to evaluate the abundance and pattern of expression of the protein. Anti-44590 antibodies can be used diagnostically to monitor protein levels in tissue as part of a clinical testing procedure, *e.g.,* to determine the efficacy of a given treatment regimen. Detection can be facilitated by coupling (i.e., physically linking) the antibody to a detectable substance (i.e., antibody labelling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

In preferred embodiments, an antibody can be made by immunizing with a purified 44590 antigen, or a fragment thereof, *e.g.,* a fragment described herein, a membrane associated antigen, tissues, *e.g.,* crude tissue preparations, whole cells, preferably living cells, lysed cells, or cell fractions, *e*.*g*., membrane fractions.

Antibodies which bind only a native 44590 protein, only denatured or otherwise non-native 44590 protein, or which bind both, arc within the invention. Antibodies with linear or conformational epitopes are within the invention. Conformational epitopes sometimes can be identified by identifying antibodies which bind to native but not denatured 44590 protein.

### Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, *e.g.,* replication defective retroviruses, adenoviruses and adeno-associated viruses.

A vector can include a 44590 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements *(e.g.,* polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein *(e.g.,* 44590 proteins, mutant forms of 44590 proteins, fusion proteins, and the like).

The recombinant expression vectors of the invention can be designed for expression of 44590 proteins in prokaryotic or eukaryotic cells. For example, polypeptides of the invention can be expressed in *E. coli*, insect cells (*e.g.,* using baculovirus expression vectors), yeast cells or mammalian cells. Suitable host cells are discussed further in Goeddel, (1990) *Gene Expression Technology: Methods in Enzymology* 185, Academic Press, San Diego, CA . Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 44590 activity assays, *(e.g.,* direct assays or competitive assays described in detail below), or to generate antibodies specific or selective for 44590 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (*e.g*., six weeks).

To maximize recombinant protein expression in E. *coli* is to express the protein in a host bacteria with an impaired capacity to proteolytically cleave the recombinant protein (Gottesman (1990) *Gene Expression Technology: Methods in Enzymology* 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada *el al*., (1992) *Nucleic* *Acids Res.* 20:2111-2118). Such alteration of nucleic acid sequences of the invention can be carried out by standard DNA synthesis techniques.

The 44590 expression vector can be a yeast expression vector, a vector for expression in insect cells, e.g., a baculovirus expression vector or a vector suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

In another embodiment, the recombinant mammalian expression vector is capable of directing expression of the nucleic acid preferentially in a particular cell type (*e*.*g*., tissue-specific regulatory elements are used to express the nucleic acid). Non-limiting examples of suitable tissue-specific promoters include the albumin promoter (liver-specific; Pinkert *et al*. (1987) *Genes Dev*. 1:268-277), lymphoid-specific promoters (Calame and Eaton (1988) *Adv. Immunol*. 43:235-275), in particular promoters of T cell receptors (Winoto and Baltimore (1989) *EMBO J.* 8:729-733) and immunoglobulins (Banerji et *al.* (1983) *Cell* 33:729-740; Queen and Baltimore (1983) *Cell* 33:741-748), neuron-specific promoters *(e.g.,* the neurofilament promoter; Byrne and Ruddle (1989) *Proc. Natl. Acad. Sci. USA* 86:5473-5477), pancreas-specific promoters (Edlund *et al.* (1985) *Science* 230:912-916), and mammary gland-specific promoters (*e.g*., milk whey promoter; U.S. Patent No. 4,873,316 and European Application Publication No. 264,166). Developmentally-regulated promoters are also encompassed, for example, the murine hox promoters (Kessel and Gruss (1990) *Science* 249:374-379) and the α-fetoprotein promoter (Campes and Tilghman (1989) *Genes Dev*. 3:537-546).

The invention further provides a recombinant expression vector comprising a DNA molecule of the invention cloned into the expression vector in an antisense orientation. Regulatory sequences (*e.g.*, viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus. For a discussion of the regulation of gene expression using antisense genes see Weintraub *et al*., (1986) *Reviews - Trends in Genetics* 1:1.

Another aspect the invention provides a host cell which includes a nucleic acid molecule described herein, *e.g.*, a 44590 nucleic acid molecule within a recombinant expression vector or a 44590 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications can occur in succeeding generations due to either mutation or environmental influences, such progeny may not, in fact, be identical to the parent cell, but are still included within the scope of the term as used herein.

A host cell can be any prokaryotic or eukaryotic cell. For example, a 44590 protein can be expressed in bacterial cells such as E. *coli*, insect cells, yeast or mammalian cells (such as Chinese hamster ovary (CHO) cells or CV-1 origin, SV-40 (COS) cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride coprecipitation, DEAE-dextran-mediated transfection, lipofection, or electroporation.

A host cell of the invention can be used to produce (i.e., express) a 44590 protein. Accordingly, the invention further provides methods for producing a 44590 protein using the host cells of the invention. In one embodiment, the method includes culturing the host cell of the invention (into which a recombinant expression vector encoding a 44590 protein has been introduced) in a suitable medium such that a 44590 protein is produced. In another embodiment, the method further includes isolating a 44590 protein from the medium or the host cell.

In another aspect, the invention features, a cell or purified preparation of cells which include a 44590 transgene, or which otherwise misexpress 44590. The cell preparation can consist of human or non-human cells, *e.g.,* rodent cells, *e.g.,* mouse or rat cells, rabbit cells, or pig cells. In preferred embodiments, the cell or cells include a 44590 transgene, *e.g.,* a heterologous form of a 44590, *e.g.,* a gene derived from humans (in the case of a non-human cell). The 44590 transgene can be misexpressed, *e.g.,* overexpressed or underexpressed. In other preferred embodiments, the cell or cells include a gene which misexpresses an endogenous 44590, *e.g.,* a gene the expression of which is disrupted, *e.g.,* a knockout. Such cells can serve as a model for studying disorders which are related to mutated or misexpressed 44590 alleles or for use in drug screening.

In another aspect, the invention features, a human cell, *e.g.,* a hematopoietic stem cell, transformed with nucleic acid which encodes a subject 44590 polypeptide.

Also provided are cells, preferably human cells, *e.g.,* human hematopoietic or fibroblast cells, in which an endogenous 44590 is under the control of a regulatory sequence that does not normally control the expression of the endogenous 44590 gene. The expression characteristics of an endogenous gene within a cell, *e*.*g*., a cell line or microorganism, can be modified by inserting a heterologous DNA regulatory element into the genome of the cell such that the inserted regulatory element is operably linked to the endogenous 44590 gene. For example, an endogenous 44590 gene which is "transcriptionally silent," *e.g.,* not normally expressed, or expressed only at very low levels, can be activated by inserting a regulatory element which is capable of promoting the expression of a normally expressed gene product in that cell. Techniques such as targeted homologous recombinations, can be used to insert the heterologous DNA as described in, *e.g.,* Chappel, US 5,272,071; WO 91/06667, published in May 16, 1991.

### Transgenic Animals

The invention provides non-human transgenic animals. Such animals are useful for studying the function and/or activity of a 44590 protein and for identifying and/or evaluating modulators of 44590 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA or a rearrangement, *e.g.,* a deletion of endogenous chromosomal DNA, which preferably is integrated into or occurs in the genome of the cells of a transgenic animal. A transgene can direct the expression of an encoded gene product in one or more cell types or tissues of the transgenic animal, other transgenes, *e.g.,* a knockout, reduce expression. Thus, a transgenic animal can be one in which an endogenous 44590 gene has been altered by, *e.g.,* by homologous recombination between the endogenous gene and an exogenous DNA molecule introduced into a cell of the animal, *e.g.,* an embryonic cell of the animal, prior to development of the animal.

Intronic sequences and polyadenylation signals can also be included in the transgene to increase the efficiency of expression of the transgene. A tissue-specific regulatory sequence(s) can be operably linked to a transgene of the invention to direct expression of a 44590 protein to particular cells. A transgenic founder animal can be identified based upon the presence of a 44590 transgene in its genome and/or expression of 44590 mRNA in tissues or cells of the animals. A transgenic founder animal can then be used to breed additional animals carrying the transgene. Moreover, transgenic animals carrying a transgene encoding a 44590 protein can further be bred to other transgenic animals carrying other transgenes.

44590 proteins or polypeptides can be expressed in transgenic animals or plants, *e.g*., a nucleic acid encoding the protein or polypeptide can be introduced into the genome of an animal. In preferred embodiments the nucleic acid is placed under the control of a tissue specific promoter, *e.g.,* a milk or egg specific promoter, and recovered from the milk or eggs produced by the animal. Suitable animals are mice, pigs, cows, goats, and sheep.

The invention also includes a population of cells from a transgenic animal, as discussed, *e.g*., below.

### Uses

The nucleic acid molecules, proteins, protein homologs, and antibodies described herein can be used in one or more of the following methods: a) screening assays; b) predictive medicine (*e.g*., diagnostic assays, prognostic assays, monitoring clinical trials, and pharmacogenetics); and c) methods of treatment *(e.g.,* therapeutic and prophylactic).

The isolated nucleic acid molecules of the invention can be used, for example, to express a 44590 protein *(e.g.,* via a recombinant expression vector in a host cell in gene therapy applications), to detect a 44590 mRNA *(e.g.,* in a biological sample) or a genetic alteration in a 44590 gene, and to modulate 44590 activity, as described further below. The 44590 proteins can be used to treat disorders characterized by insufficient or excessive production of a 44590 substrate or production of 44590 inhibitors. In addition, the 44590 proteins can be used to screen for naturally occurring 44590 substrates, to screen for drugs or compounds which modulate 44590 activity, as well as to treat disorders characterized by insufficient or excessive production of 44590 protein or production of 44590 protein forms which have decreased, aberrant or unwanted activity compared to 44590 wild type protein (*e.g*., aberrant or deficient transporter function or expression). Moreover, the anti-44590 antibodies of the invention can be used to detect and isolate 44590 proteins, regulate the bioavailability of 44590 proteins, and modulate 44590 activity.

A method of evaluating a compound for the ability to interact with, *e.g.,* bind, a subject 44590 polypeptide is provided. The method includes: contacting the compound with the subject 44590 polypeptide; and evaluating ability of the compound to interact with, *e.g.*, to bind or form a complex with the subject 44590 polypeptide. This method can be performed *in vitro*, *e.g.,* in a cell free system, or *in vivo*, *e*.*g.*, in a two-hybrid interaction trap assay. This method can be used to identify naturally occurring molecules which interact with subject 44590 polypeptide. It can also be used to find natural or synthetic inhibitors of subject 44590 polypeptide. Screening methods are discussed in more detail below.

### Screening Assays:

The invention provides methods (also referred to herein as "screening assays") for identifying modulators, i.e., candidate or test compounds or agents (*e.g.*, proteins, peptides, peptidomimetics, peptoids, small molecules or other drugs) which bind to 44590 proteins, have a stimulatory or inhibitory effect on, for example, 44590 expression or 44590 activity, or have a stimulatory or inhibitory effect on, for example, the expression or activity of a 44590 substrate. Compounds thus identified can be used to modulate the activity of target gene products (*e.g.*, 44590 genes) in a therapeutic protocol, to elaborate the biological function of the target gene product, or to identify compounds that disrupt normal target gene interactions.

In one embodiment, the invention provides assays for screening candidate or test compounds which are substrates of a 44590 protein or polypeptide or a biologically active portion thereof. In another embodiment, the invention provides assays for screening candidate or test compounds which bind to or modulate the activity of a 44590 protein or polypeptide or a biologically active portion thereof.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, *e.g.,* Zuckermann *et al.* (1994) *J*. *Med. Chem.* 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) *Anticancer Drug Des.* 12:145).

Examples of methods for the synthesis of molecular libraries can be found in the art, for example in: DeWitt *et al.* (1993) *Proc. Natl. Acad*. *Sci. U.S.A.* 90:6909-13; Erb *et al.* (1994) *Proc. Natl. Acad. Sci. USA* 91:11422-426; Zuckermann *et al.* (1994). *J. Med. Chem.* 37:2678-85; Cho *et al.* (1993) *Science* 261:1303; Carrell *et al.* (1994) *Angew. Chem. Int. Ed. Engl.* 33:2059; Carell *et al.* (1994) *Angew. Chem. Int. Ed. Engl.* 33:2061; and in Gallop *et al.* (1994) *J*. *Med. Chem.* 37:1233-51.

Libraries of compounds can be presented in solution (*e.g.,* Houghten (1992) *Biotechniques* 13:412-421), or on beads (Lam (1991) *Nature* 354:82-84), chips (Fodor (1993) *Nature* 364:555-556), bacteria (Ladner, USP 5,223,409), spores (Ladner USP '409), plasmids (Cull *et al.* (1992) *Proc Natl Acad Sci USA* 89:1865-1869) or on phage (Scott and Smith (1990) *Science* 249:386-390; Devlin (1990) *Science* 249:404-406; Cwirla *et al.* (1990) *Proc. Natl. Acad. Sci.* 87:6378-6382; Felici (1991) *J*. *Mol. Biol.* 222:301-310; Ladner *supra.*).

In one embodiment, an assay is a cell-based assay in which a cell which expresses a 44590 protein or biologically active portion thereof is contacted with a test compound, and the ability of the test compound to modulate 44590 activity is determined. Determining the ability of the test compound to modulate 44590 activity can be accomplished by monitoring, for example the ability to: reside within a membrane; dimerize with a second transporter, *e.g.,* an ABC transporter; bind and hydrolyze a nucleotide, *e.g.,* ATP or GTP; or bind and transport a molecule *e.g*., an amino acid, a peptide, an ion, a sugar, a nucleic acid, a signaling molecule, a vitamin, or a cofactor across a membrane, *e.g.,* a cell or organelle membrane. The cell, for example, can be of mammalian origin, *e.g.,* human.

The ability of the test compound to modulate 44590 binding to a compound, *e.g.,* a 44590 substrate, or to bind to 44590 can also be evaluated. This can be accomplished, for example, by coupling the compound, *e.g*., the substrate, with a radioisotope or enzymatic label such that binding of the compound, *e.g.,* the substrate, to 44590 can be determined by detecting the labeled compound, *e.g*., substrate, in a complex. Alternatively, 44590 could be coupled with a radioisotope or enzymatic label to monitor the ability of a test compound to modulate 44590 binding to a 44590 substrate in a complex. For example, compounds (*e.g*., 44590 substrates) can be labeled with ¹²⁵I, ¹⁴C, ³⁵S or ³H., either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (*e.g*., *a* 44590 substrate) to interact with 44590 with or without the labeling of any of the interactants can be evaluated. For example, a microphysiometer can be used to detect the interaction of a compound with 44590 without the labeling of either the compound or the 44590. McConnell *et al.* (1992) *Science* 257:1906-1912. As used herein, a "microphysiometer" (*e.g*., Cytosensor) is an analytical instrument that measures the rate at which a cell acidifies its environment using a light-addressable potentiometric sensor (LAPS). Changes in this acidification rate can be used as an indicator of the interaction between a compound and 44590.

In yet another embodiment, a cell-free assay is provided in which a 44590 protein or biologically active portion thereof is contacted with a test compound and the ability of the test compound to bind to the 44590 protein or biologically active portion thereof is evaluated. Preferred biologically active portions of the 44590 proteins to be used in assays of the present invention include fragments which participate in interactions with non-44590 molecules, *e.g.*, fragments with high surface probability scores.

Soluble and/or membrane-bound forms of isolated proteins (*e*.*g*., 44590 proteins or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, *e.g.,* using fluorescence energy transfer (FET) (see, for example, Lakowicz *et al*., U.S. Patent No. 5,631,169; Stavrianopoulos, *et al*., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule can simply utilize the natural fluorescent energy of tryptophan residues. Labels are chosen that emit different wavelengths of light, such that the 'acceptor' molecule label can be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art *(e.g.,* using a fluorimeter).

In another embodiment, determining the ability of the 44590 protein to bind to a target molecule can be accomplished using real-time Biomolecular Interaction Analysis (BIA) (see, *e.g.,* Sjolander and Urbaniczky (1991) *Anal. Chem.* 63:2338-2345 and Szabo *et al.* (1995) *Curr. Opin. Struct. Biol*. 5:699-705). "Surface plasmon resonance" or "BIA" detects biospecific interactions in real time, without labeling any of the interactants *(e.g.,* BlAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 44590, an anti-44590 antibody or its target molecule to facilitate separation of complexed from uncomplexed forms of one or both of the proteins, as well as to accommodate automation of the assay. Binding of a test compound to a 44590 protein, or interaction of a 44590 protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/44590 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 44590 protein, and the mixture incubated under conditions conducive to complex formation (*e.g.*, at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 44590 binding or activity determined using standard techniques.

Other techniques for immobilizing either a 44590 protein or a target molecule on matrices include using conjugation of biotin and streptavidin. Biotinylated 44590 protein or target molecules can be prepared from biotin-NHS (N-hydroxy-succinimide) using techniques known in the art (*e.g*., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g.,* using a labeled antibody specific or selective for the immobilized component (the antibody, in turn, can be directly labeled or indirectly labeled with, *e.g.,* a labeled anti-Ig antibody).

In one embodiment, this assay is performed utilizing antibodies reactive with 44590 protein or target molecules but which do not interfere with binding of the 44590 protein to its target molecule. Such antibodies can be derivatized to the wells of the plate, and unbound target or 44590 protein trapped in the wells by antibody conjugation. Methods for detecting such complexes, in addition to those described above for the GST-immobilized complexes, include immunodetection of complexes using antibodies reactive with the 44590 protein or target molecule, as well as enzyme-linked assays which rely on detecting an enzymatic activity associated with the 44590 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas and Minton (1993) *Trends Biochem Sci* 18:284-7); chromatography (gel filtration chromatography, ion-exchange chromatography); electrophoresis (see, *e.g.,* Ausubel *et al*., eds. (1999) *Current Protocols in Molecular Biology, J.* Wiley, New York.); and immunoprecipitation (see, for example, Ausubel *et al*., eds. (1999) *Current Protocols in Molecular Biology, J.* Wiley, New York). Such resins and chromatographic techniques are known to one skilled in the art (see, *e.g.,* Heegaard (1998) *J Mol Recognit* 11:141-8; Hage and Tweed (1997) *J Chromatogr B Biomed Sci Appl.* 699:499-525). Further, fluorescence energy transfer can also be conveniently utilized, as described herein, to detect binding without further purification of the complex from solution.

In a preferred embodiment, the assay includes contacting the 44590 protein or biologically active portion thereof with a known compound which binds 44590 to form an assay mixture, contacting the assay mixture with a test compound, and determining the ability of the test compound to interact with a 44590 protein, wherein determining the ability of the test compound to interact with a 44590 protein includes determining the ability of the test compound to preferentially bind to 44590 or biologically active portion thereof, or to modulate the activity of a target molecule, as compared to the known compound.

The target gene products of the invention can, *in vivo*, interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 44590 genes herein identified. In an alternative embodiment, the invention provides methods for determining the ability of the test compound to modulate the activity of a 44590 protein through modulation of the activity of a downstream effector of a 44590 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, *e.g.,* by competition, can be identified by conducting the reaction in the presence of the test substance. Alternatively, test compounds that disrupt preformed complexes, *e.g.,* compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (*e.g.*, a microtiter plate), while the non-anchored species is labeled, either directly or indirectly. The anchored species can be immobilized by non-covalent or covalent attachments. Alternatively, an immobilized antibody specific or selective for the species to be anchored can be used to anchor the species to the solid surface.

In order to conduct the assay, the partner of the immobilized species is exposed to the coated surface with or without the test compound. After the reaction is complete, unreacted components are removed (*e.g*., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; *e.g.,* using a labeled antibody specific or selective for the initially non-immobilized species (the antibody, in turn, can be directly labeled or indirectly labeled with, *e.g.,* a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; *e.g.,* using an immobilized antibody specific or selective for one of the binding components to anchor any complexes formed in solution, and a labeled antibody specific or selective for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, *e.g*., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 44590 proteins can be used as "bait proteins" in a two-hybrid assay or three-hybrid assay (see, *e.g.,* U.S. Patent No. 5,283,317; Zervos *et al.* (1993) *Cell* 72:223-232; Madura *et al.* (1993) *J. Biol. Chem.* 268:12046-12054; Bartel *et al.* (1993) *Biotechniques* 14:920-924; Iwabuchi *et al.* (1993) *Oncogene* 8:1693-1696; and Brent WO94/10300), to identify other proteins, which bind to or interact with 44590 ("44590-binding proteins" or "44590-bp") and are involved in 44590 activity. Such 44590-bps can be activators or inhibitors of signals by the 44590 proteins or 44590 targets as, for example, downstream elements of a 44590-mediated signaling pathway.

The two-hybrid system is based on the modular nature of most transcription factors, which consist of separable DNA-binding and activation domains. Briefly, the assay utilizes two different DNA constructs. In one construct, the gene that codes for a 44590 protein is fused to a gene encoding the DNA binding domain of a known transcription factor (*e.g.,* GAL-4). In the other construct, a DNA sequence, from a library of DNA sequences, that encodes an unidentified protein ("prey" or "sample") is fused to a gene that codes for the activation domain of the known transcription factor. (Alternatively the: 44590 protein can be the fused to the activator domain.) If the "bait" and the "prey" proteins are able to interact, *in vivo*, forming a 44590-dependent complex, the DNA-binding and activation domains of the transcription factor are brought into close proximity. This proximity allows transcription of a reporter gene *(e.g.,* lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 44590 protein.

In another embodiment, modulators of 44590 expression are identified. For example, a cell or cell free mixture is contacted with a candidate compound and the expression of 44590 mRNA or protein evaluated relative to the level of expression of 44590 mRNA or protein in the absence of the candidate compound. When expression of 44590 mRNA or protein is greater in the presence of the candidate compound than in its absence, the candidate compound is identified as a stimulator of 44590 mRNA or protein expression. Alternatively, when expression of 44590 mRNA or protein is less (statistically significantly less) in the presence of the candidate compound than in its absence, the candidate compound is identified as an inhibitor of 44590 mRNA or protein expression. The level of 44590 mRNA or protein expression can be determined by methods described herein for detecting 44590 mRNA or protein.

In another aspect, the invention pertains to a combination of two or more of the assays described herein. For example, a modulating agent can be identified using a cell-based or a cell free assay, and the ability of the agent to modulate the activity of a 44590 protein can be confirmed *in vivo, e.g.,* in an animal such as an animal model for aberrant or deficient transporter function or expression.

This invention further pertains to novel agents identified by the above-described screening assays. Accordingly, it is within the scope of this invention to further use an agent identified as described herein (*e.g.*, a 44590 modulating agent, an antisense 44590 nucleic acid molecule, a 44590-specific antibody, or a 44590-binding partner) in an appropriate animal model to determine the efficacy, toxicity, side effects, or mechanism of action, of treatment with such an agent. Furthermore, novel agents identified by the above-described screening assays can be used for treatments as described herein.

### Detection Assays

Portions or fragments of the nucleic acid sequences identified herein can be used as polynucleotide reagents. For example, these sequences can be used to: (i) map their respective genes on a chromosome *e.g*., to locate gene regions associated with genetic disease or to associate 44590 with a disease; (ii) identify an individual from a minute biological sample (tissue typing); and (iii) aid in forensic identification of a biological sample. These applications are described in the subsections below.

### Chromosome Mapping

The 44590 nucleotide sequences or portions thereof can be used to map the location of the 44590 genes on a chromosome. This process is called chromosome mapping. Chromosome mapping is useful in correlating the 44590 sequences with genes associated with disease.

Briefly, 44590 genes can be mapped to chromosomes by preparing PCR primers (preferably 15-25 bp in length) from the 44590 nucleotide sequences. These primers can then be used for PCR screening of somatic cell hybrids containing individual human chromosomes. Only those hybrids containing the human gene corresponding to the 44590 sequences will yield an amplified fragment.

A panel of somatic cell hybrids in which each cell line contains either a single human chromosome or a small number of human chromosomes, and a full set of mouse chromosomes, can allow easy mapping of individual genes to specific human chromosomes. (D'Eustachio *et al*. (1983) *Science* 220:919-924).

Other mapping strategies *e.g.,* in situ hybridization (described in Fan *et al*. (1990) *Proc. Natl. Acad. Sci. USA*, 87:6223-27), pre-screening with labeled flow-sorted chromosomes, and pre-selection by hybridization to chromosome specific cDNA libraries can be used to map 44590 to a chromosomal location.

Fluorescence *in situ* hybridization (FISH) of a DNA sequence to a metaphase chromosomal spread can further be used to provide a precise chromosomal location in one step. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma *et al.* (1988) Human Chromosomes: A Manual of Basic Techniques, Pergamon Press, New York).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in McKusick, *Mendelian Inheritance in Man,* available on-line through Johns Hopkins University Welch Medical Library). The relationship between a gene and a disease, mapped to the same chromosomal region, can then be identified through linkage analysis (co-inheritance of physically adjacent genes), described in, for example, Egeland *et al.* (1987) *Nature*, 325:783-787.

Moreover, differences in the DNA sequences between individuals affected and unaffected with a disease associated with the 44590 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

44590 sequences can be used to identify individuals from biological samples using, *e.g.,* restriction fragment length polymorphism (RFLP). In this technique, an individual's genomic DNA is digested with one or more restriction enzymes, the fragments separated, *e.g*., in a Southern blot, and probed to yield bands for identification. The sequences of the present invention are useful as additional DNA markers for RFLP (described in U.S. Patent 5,272,057).

Furthermore, the sequences of the present invention can also be used to determine the actual base-by-base DNA sequence of selected portions of an individual's genome. Thus, the 44590 nucleotide sequences described herein can be used to prepare two PCR primers from the 5' and 3' ends of the sequences. These primers can then be used to amplify an individual's DNA and subsequently sequence it. Panels of corresponding DNA sequences from individuals, prepared in this manner, can provide unique individual identifications, as each individual will have a unique set of such DNA sequences due to allelic differences.

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1 can provide positive individual identification with a panel of perhaps 10 to 1,000 primers which each yield a noncoding amplified sequence of 100 bases. If predicted coding sequences, such as those in SEQ ID NO:3 are used, a more appropriate number of primers for positive individual identification would be 500-2,000.

If a panel of reagents from 44590 nucleotide sequences described herein is used to generate a unique identification database for an individual, those same reagents can later be used to identify tissue from that individual. Using the unique identification database, positive identification of the individual, living or dead, can be made from extremely small tissue samples.

### Use of Partial 44590 Sequences in Forensic Biology

DNA-based identification techniques can also be used in forensic biology. To make such an identification, PCR technology can be used to amplify DNA sequences taken from very small biological samples such as tissues, *e.g.,* hair or skin, or body fluids, *e.g.,* blood, saliva, or semen found at a crime scene. The amplified sequence can then be compared to a standard, thereby allowing identification of the origin of the biological sample.

The sequences of the present invention can be used to provide polynucleotide reagents, *e.g.,* PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1 *(e.g.,* fragments derived from the noncoding regions of SEQ ID NO: 1 having a length of at least 20 bases, preferably at least 30 bases) are particularly appropriate for this use.

The 44590 nucleotide sequences described herein can further be used to provide polynucleotide reagents, *e.g.,* labeled or labelable probes which can be used in, for example, an *in situ* hybridization technique, to identify a specific tissue. This can be very useful in cases where a forensic pathologist is presented with a tissue of unknown origin. Panels of such 44590 probes can be used to identify tissue by species and/or by organ type.

In a similar fashion, these reagents, *e.g.,* 44590 primers or probes can be used to screen tissue culture for contamination (i.e. screen for the presence of a mixture of different types of cells in a culture).

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 44590.

Such disorders include, *e.g.,* a disorder associated with the misexpression of 44590 gene; a disorder of the ABC transport system.

The method includes one or more of the following:

detecting, in a tissue of the subject, the presence or absence of a mutation which affects the expression of the 44590 gene, or detecting the presence or absence of a mutation in a region which controls the expression of the gene, *e.g.,* a mutation in the 5' control region;

detecting, in a tissue of the subject, the presence or absence of a mutation which alters the structure of the 44590 gene;

detecting, in a tissue of the subject, the misexpression of the 44590 gene, at the mRNA level, *e.g.,* detecting a non-wild type level of an mRNA;

detecting, in a tissue of the subject, the misexpression of the gene, at the protein level, *e.g.,* detecting a non-wild type level of a 44590 polypeptide.

In preferred embodiments the method includes: ascertaining the existence of at least one of: a deletion of one or more nucleotides from the 44590 gene; an insertion of one or more nucleotides into the gene, a point mutation, *e.g.,* a substitution of one or more nucleotides of the gene, a gross chromosomal rearrangement of the gene, *e.g*., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, or naturally occurring mutants thereof or 5' or 3' flanking sequences naturally associated with the 44590 gene; (ii) exposing the probe/primer to nucleic acid of the tissue; and detecting, by hybridization, *e.g., in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 44590 gene; the presence of a non-wild type splicing pattern of a messenger RNA transcript of the gene; or a non-wild type level of 44590.

Methods of the invention can be used prenatally or to determine if a subject's offspring will be at risk for a disorder.

In preferred embodiments the method includes determining the structure of a 44590 gene, an abnormal structure being indicative of risk for the disorder.

In preferred embodiments the method includes contacting a sample from the subject with an antibody to the 44590 protein or a nucleic acid, which hybridizes specifically with the gene. These and other embodiments are discussed below.

### Diagnostic and Prognostic Assays

The presence, level, or absence of 44590 protein or nucleic acid in a biological sample can be evaluated by obtaining a biological sample from a test subject and contacting the biological sample with a compound or an agent capable of detecting 44590 protein or nucleic acid (*e.g.*, mRNA, genomic DNA) that encodes 44590 protein such that the presence of 44590 protein or nucleic acid is detected in the biological sample. The term "biological sample" includes tissues, cells and biological fluids isolated from a subject, as well as tissues, cells and fluids present within a subject. A preferred biological sample is serum. The level of expression of the 44590 gene can be measured in a number of ways, including, but not limited to: measuring the mRNA encoded by the 44590 genes; measuring the amount of protein encoded by the 44590 genes; or measuring the activity of the protein encoded by the 44590 genes.

The level of mRNA corresponding to the 44590 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 44590 nucleic acid, such as the nucleic acid of SEQ ID NO:1, or a portion thereof, such as an oligonucleotide of at least 7, 15, 30, 50, 100, 250 or 500 nucleotides in length and sufficient to specifically hybridize under stringent conditions to 44590 mRNA or genomic DNA. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array. A skilled artisan can adapt known mRNA detection methods for use in detecting the level of mRNA encoded by the 44590 genes.

The level of mRNA in a sample that is encoded by one of 44590 can be evaluated with nucleic acid amplification, *e.g.,* by rtPCR (Mullis (1987) U.S. Patent No. 4,683,202), ligase chain reaction (Barany (1991) *Proc. Natl. Acad. Sci*. *USA* 88:189-193), self sustained sequence replication (Guatelli *et al*., (1990) *Proc. Natl. Acad. Sci. USA* 87:1874-1878), transcriptional amplification system (Kwoh *et al*., (1989), *Proc. Natl. Acad. Sci. USA* 86:1173-1177), Q-Beta Replicase (Lizardi *et al*., (1988) *Bio*/*Technology* 6:1197), rolling circle replication (Lizardi *et al*., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers arc from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 44590 gene being analyzed.

In another embodiment, the methods further contacting a control sample with a compound or agent capable of detecting 44590 mRNA, or genomic DNA, and comparing the presence of 44590 mRNA or genomic DNA in the control sample with the presence of 44590 mRNA or genomic DNA in the test sample.

A variety of methods can be used to determine the level of protein encoded by 44590. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (*e*.*g*., Fab or F(ab')₂) can be used. The term "labeled", with regard to the probe or antibody, is intended to encompass direct labeling of the probe or antibody by coupling (*i*.*e*., physically linking) a detectable substance to the probe or antibody, as well as indirect labeling of the probe or antibody by reactivity with a detectable substance. Examples of detectable substances are provided herein.

The detection methods can be used to detect 44590 protein in a biological sample *in vitro* as well as *in vivo*. *In vitro* techniques for detection of 44590 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 44590 protein include introducing into a subject a labeled anti-44590 antibody. For example, the antibody can be labeled with a radioactive marker whose presence and location in a subject can be detected by standard imaging techniques.

In another embodiment, the methods further include contacting the control sample with a compound or agent capable of detecting 44590 protein, and comparing the presence of 44590 protein in the control sample with the presence of 44590 protein in the test sample.

The invention also includes kits for detecting the presence of 44590 in a biological sample. For example, the kit can include a compound or agent capable of detecting 44590 protein or mRNA in a biological sample; and a standard. The compound or agent can be packaged in a suitable container. The kit can further comprise instructions for using the kit to detect 44590 protein or nucleic acid.

For antibody-based kits, the kit can include: (1) a first antibody (*e.g.*, attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, *e.g.,* a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (*e.g.,* an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 44590 expression or activity. As used herein, the term "unwanted" includes an unwanted phenomenon involved in a biological response such as pain or deregulated cell proliferation.

In one embodiment, a disease or disorder associated with aberrant or unwanted 44590 expression or activity is identified. A test sample is obtained from a subject and 44590 protein or nucleic acid *(e.g.,* mRNA or genomic DNA) is evaluated, wherein the level, *e.g.*, the presence or absence, of 44590 protein or nucleic acid is diagnostic for a subject having or at risk of developing a disease or disorder associated with aberrant or unwanted 44590 expression or activity. As used herein, a "test sample" refers to a biological sample obtained from a subject of interest, including a biological fluid (*e.g.*, serum), cell sample, or tissue.

The prognostic assays described herein can be used to determine whether a subject can be administered an agent (*e.g.*, an agonist, antagonist, peptidomimetic, protein, peptide, nucleic acid, small molecule, or other drug candidate) to treat a disease or disorder associated with aberrant or unwanted 44590 expression or activity. For example, such methods can be used to determine whether a subject can be effectively treated with an agent for an ABC transporter disorder.

The methods of the invention can also be used to detect genetic alterations in a 44590 gene, thereby determining if a subject with the altered gene is at risk for a disorder characterized by misregulation in 44590 protein activity or nucleic acid expression, such as an ABC transporter disorder. In preferred embodiments, the methods include detecting, in a sample from the subject, the presence or absence of a genetic alteration characterized by at least one of an alteration affecting the integrity of a gene encoding a 44590-protein, or the mis-expression of the 44590 gene. For example, such genetic alterations can be detected by ascertaining the existence of at least one of 1) a deletion of one or more nucleotides from a 44590 gene; 2) an addition of one or more nucleotides to a 44590 gene; 3) a substitution of one or more nucleotides of a 44590 gene, 4) a chromosomal rearrangement of a 44590 gene; 5) an alteration in the level of a messenger RNA transcript of a 44590 gene, 6) aberrant modification of a 44590 gene, such as of the methylation pattern of the genomic DNA, 7) the presence of a non-wild type splicing pattern of a messenger RNA transcript of a 44590 gene, 8) a non-wild type level of a 44590-protein, 9) allelic loss of a 44590 gene, and 10) inappropriate post-translational modification of a 44590-protein.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 44590-gene. This method can include the steps of collecting a sample of cells from a subject, isolating nucleic acid (*e.g.*, genomic, mRNA or both) from the sample, contacting the nucleic acid sample with one or more primers which specifically hybridize to a 44590 gene under conditions such that hybridization and amplification of the 44590 gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein. Alternatively, other amplification methods described herein or known in the art can be used.

In another embodiment, mutations in a 44590 gene from a sample cell can be identified by detecting alterations in restriction enzyme cleavage patterns. For example, sample and control DNA is isolated, amplified (optionally), digested with one or more restriction endonucleases, and fragment length sizes are determined, *e.g.,* by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 44590 can be identified by hybridizing a sample and control nucleic acids, *e.g.,* DNA or RNA, two dimensional arrays, *e.g.,* chip based arrays. Such arrays include a plurality of addresses, each of which is positionally distinguishable from the other. A different probe is located at each address of the plurality. The arrays can have a high density of addresses, *e.g.,* can contain hundreds or thousands of oligonucleotides probes (Cronin *et al.* (1996) *Human Mutation 7:* 244-255; Kozal *et al*. (1996) *Nature Medicine* 2: 753-759). For example, genetic mutations in 44590 can be identified in two dimensional arrays containing light-generated DNA probes as described in Cronin, M.T. *et al. supra*. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 44590 gene and detect mutations by comparing the sequence of the sample 44590 with the corresponding wild-type (control) sequence. Automated sequencing procedures can be utilized when performing the diagnostic assays (Naeve *et al*. (1995) *Biotechniques* 19:448-53), including sequencing by mass spectrometry.

Other methods for detecting mutations in the 44590 gene include methods in which protection from cleavage agents is used to detect mismatched bases in RNA/RNA or RNA/DNA heteroduplexes (Myers *et al*. (1985) *Science* 230:1242; Cotton *et al*. (1988) *Proc. Natl Acad Sci USA* 85:4397; Saleeba *et al*. (1992) *Methods Enzymol*. 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 44590 cDNAs obtained from samples of cells. For example, the mutY enzyme of *E*. *coli* cleaves A at G/A mismatches and the thymidine DNA glycosylase from HeLa cells cleaves T at G/T mismatches (Hsu *et al.* (1994) *Carcinogenesis* 15:1657-1662; U.S. Patent No. 5,459,039).

In other embodiments, alterations in electrophoretic mobility will be used to identify mutations in 44590 genes. For example, single strand conformation polymorphism (SSCP) can be used to detect differences in electrophoretic mobility between mutant and wild type nucleic acids (Orita *et al.* (1989) *Proc Natl. Acad. Sci USA:* 86:2766, see also Cotton (1993) *Mutat. Res.* 285:125-144; and Hayashi (1992) *Genet. Anal. Tech. Appl.* 9:73-79). Single-stranded DNA fragments of sample and control 44590 nucleic acids will be denatured and allowed to renature. The secondary structure of single-stranded nucleic acids varies according to sequence, the resulting alteration in electrophoretic mobility enables the detection of even a single base change. The DNA fragments can be labeled or detected with labeled probes. The sensitivity of the assay can be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen *et al.* (1991) *Trends Genel* 7:5).

In yet another embodiment, the movement of mutant or wild-type fragments in polyacrylamide gels containing a gradient of denaturant is assayed using denaturing gradient gel electrophoresis (DGGE) (Myers *et al.* (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys Chem* 265:12753).

Examples of other techniques for detecting point mutations include, but are not limited to, selective oligonucleotide hybridization, selective amplification, or selective primer extension (Saiki *et al.* (1986) *Nature* 324:163); Saiki *et al*. (1989) *Proc. Natl Acad*. *Sci USA* 86:6230).

Alternatively, allele specific amplification technology which depends on selective PCR amplification can be used in conjunction with the instant invention. Oligonucleotides used as primers for specific amplification can carry the mutation of interest in the center of the molecule (so that amplification depends on differential hybridization) (Gibbs *et al.* (1989) *Nucleic Acids Res*. 17:2437-2448) or at the extreme 3' end of one primer where, under appropriate conditions, mismatch can prevent, or reduce polymerase extension (Prossner (1993) *Tibtech* 11:238). In addition it may be desirable to introduce a novel restriction site in the region of the mutation to create cleavage-based detection (Gasparini *et al*. (1992) *Mol. Cell Probes* 6:1). It is anticipated that in certain embodiments amplification can also be performed using Taq ligase for amplification (Barany (1991) *Proc. Natl*. *Acad. Sci USA* 88:189-93). In such cases, ligation will occur only if there is a perfect match at the 3' end of the 5' sequence making it possible to detect the presence of a known mutation at a specific site by looking for the presence or absence of amplification.

The methods described herein can be performed, for example, by utilizing prepackaged diagnostic kits comprising at least one probe nucleic acid or antibody reagent described herein, which can be conveniently used, *e.g*., in clinical settings to diagnose patients exhibiting symptoms or family history of a disease or illness involving a 44590 gene.

### Use of 44590 Molecules as Surrogate Markers

The 44590 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the 44590 molecules of the invention can be detected, and can be correlated with one or more biological states *in vivo*. For example, the 44590 molecules of the invention can serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (*e.g.*, with the presence or absence of a tumor). The presence or quantity of such markers is independent of the disease. Therefore, these markers can serve to indicate whether a particular course of treatment is effective in lessening a disease state or disorder. Surrogate markers are of particular use when the presence or extent of a disease state or disorder is difficult to assess through standard methodologies *(e.g.,* early stage tumors), or when an assessment of disease progression is desired before a potentially dangerous clinical endpoint is reached *(e.g.,* an assessment of cardiovascular disease can be made using cholesterol levels as a surrogate marker, and an analysis of HIV infection can be made using HIV RNA levels as a surrogate marker, well in advance of the undesirable clinical outcomes of myocardial infarction or fully-developed AIDS). Examples of the use of surrogate markers in the art include: Koomen *et al.* (2000) *J. Mass. Spectrom*. 35: 258-264; and James (1994) *AIDS Treatment News Archive* 209.

The 44590 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker can be indicative of the concentration of the drug in a biological tissue, in that the marker is either expressed or transcribed or not expressed or transcribed in that tissue in relationship to the level of the drug. In this fashion, the distribution or uptake of the drug can be monitored by the pharmacodynamic marker. Similarly, the presence or quantity of the pharmacodynamic marker can be related to the presence or quantity of the metabolic product of a drug, such that the presence or quantity of the marker is indicative of the relative breakdown rate of the drug *in vivo*. Pharmacodynamic markers are of particular use in increasing the sensitivity of detection of drug effects, particularly when the drug is administered in low doses. Since even a small amount of a drug can be sufficient to activate multiple rounds of marker (*e.g.,* a 44590 marker) transcription or expression, the amplified marker can be in a quantity which is more readily detectable than the drug itself. Also, the marker can be more easily detected due to the nature of the marker itself; for example, using the methods described herein, anti-44590 antibodies can be employed in an immune-based detection system for a 44590 protein marker, or 44590-specific radiolabeled probes can be used to detect a 44590 mRNA marker. Furthermore, the use of a pharmacodynamic marker can offer mechanism-based prediction of risk due to drug treatment beyond the range of possible direct observations. Examples of the use of pharmacodynamic markers in the art include: Matsuda *et al.* US 6,033,862; Hattis *et al.* (1991) *Env. Health Perspect*. 90: 229-238; Schentag (1999) *Am. J Health-Syst. Pharm.* 56 Suppl. 3: S21-S24; and Nicolau (1999) *Am. J. Health-Syst*. *Pharm*. 56 Suppl. 3: S16-S20.

The 44590 molecules of the invention are also useful as pharmacogenomic markers. As used herein, a "pharmacogenomic marker" is an objective biochemical marker which correlates with a specific clinical drug response or susceptibility in a subject (see, *e.g.,* McLeod *et al*. (1999) *Eur. J. Cancer* 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, can be selected. For example, based on the presence or quantity of RNA, or protein (*e.g*., 44590 protein or RNA) for specific tumor markers in a subject, a drug or course of treatment can be selected that is optimized for the treatment of the specific tumor likely to be present in the subject. Similarly, the presence or absence of a specific sequence mutation in 44590 DNA can correlate with a 44590 drug response. The use of pharmacogenomie markers therefore permits the application of the most appropriate treatment for each subject without having to administer the therapy.

### Pharmaceutical Compositions

The nucleic acid and polypeptides, fragments thereof, as well as anti-44590 antibodies (also referred to herein as "active compounds") of the invention can be incorporated into pharmaceutical compositions. Such compositions typically include the nucleic acid molecule, protein, or antibody and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, *e.g.,* intravenous, intradermal, subcutaneous, oral (*e.g.*, inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injcctable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g.,* gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.*, with conventional suppository bases such as cocoa butter and other glyccrides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ ED₅₀. Compounds which exhibit high therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma can be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody, unconjugated or conjugated as described herein, can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (*e.g.*, into the brain). A method for lipidation of antibodies is described by Cruikshank *et al.* ((1997) *J*. *Acquired Immune Deficiency Syndromes and Human Retrovirology* 14:193).

The present invention encompasses agents which modulate expression or activity. An agent can, for example, be a small molecule. For example, such small molecules include, but are not limited to, peptides, peptidomimetics (*e.g*., peptoids), amino acids, amino acid analogs, polynucleotides, polynucleotide analogs, nucleotides, nucleotide analogs, organic or inorganic compounds (i.e., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (*e.g.*, about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal *(e.g.,* a human) in order to modulate expression or activity of a polypeptide or nucleic acid of the invention, a physician, veterinarian, or researcher can, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see *e.g.,* Chen *et al.* (1994) *Proc. Natl. Read. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells which produce the gene delivery system.

The pharmaceutical compositions can be included in a container, pack, or dispenser together with instructions for administration.

### Methods of Treatment:

The present invention provides for both prophylactic and therapeutic methods of treating a subject at risk of (or susceptible to) a disorder or having a disorder associated with aberrant or unwanted 44590 expression or activity. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides.

With regards to both prophylactic and therapeutic methods of treatment, such treatments can be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 44590 molecules of the present invention or 44590 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

In one aspect, the invention provides a method for preventing in a subject, a disease or condition associated with an aberrant or unwanted 44590 expression or activity, by administering to the subject a 44590 or an agent which modulates 44590 expression or at least one 44590 activity. Subjects at risk for a disease which is caused or contributed to by aberrant or unwanted 44590 expression or activity can be identified by, for example, any or a combination of diagnostic or prognostic assays as described herein. Administration of a prophylactic agent can occur prior to the manifestation of symptoms characteristic of the 44590 aberrance, such that a disease or disorder is prevented or, alternatively, delayed in its progression. Depending on the type of 44590 aberrance, for example, a 44590, 44590 agonist or 44590 antagonist agent can be used for treating the subject. The appropriate agent can be determined based on screening assays described herein.

It is possible that some 44590 disorders can be caused, at least in part, by an abnormal level of gene product, or by the presence of a gene product exhibiting abnormal activity. As such, the reduction in the level and/or activity of such gene products would bring about the amelioration of disorder symptoms.

The 44590 molecules can act as novel diagnostic targets and therapeutic agents for controlling one or more of breast associated disorders described above. The molecules of the invention also can act as novel diagnostic targets and therapeutic agents for controlling one or more of, skin disorders, pain or metabolic disorders, hormonal disorders, neurological disorders, cellular proliferative and/or differentiative disorders, immune disorders *e.g.*, inflammatory disorders, cardiovascular disorders, including endothelial cell disorders, or liver disorders, disorders associated with bone metabolism, and viral diseases described below.

Diseases of the skin that the 44590 molecules may beinvolved in, include but are not limited to, disorders of pigmentation and melanocytes, including but not limited to, vitiligo, freckle, melasma, lentigo, nevocellular nevus, dysplastic nevi, and malignant melanoma; benign epithelial tumors, including but not limited to, seborrheic keratoses, acanthosis nigricans, fibroepithelial polyp, epithelial cyst, keratoacanthoma, and adnexal (appendage) tumors; premalignant and malignant epidermal tumors, including but not limited to, actinic keratosis, squamous cell carcinoma, basal cell carcinoma, and merkel cell carcinoma; tumors of the dermis, including but not limited to, benign fibrous histiocytoma, dermatofibrosarcoma protuberans, xanthomas, and dermal vascular tumors; tumors of cellular immigrants to the skin, including but not limited to, histiocytosis X, mycosis fungoides (cutaneous T-cell lymphoma), and mastocytosis; disorders of epidermal maturation, including but not limited to, ichthyosis; acute inflammatory dermatoses, including but not limited to, urticaria, acute eczematous dermatitis, and erythema multiforme; chronic inflammatory dermatoses, including but not limited to, psoriasis, lichen planus, and lupus erythematosus; blistering (bullous) diseases, including but not limited to, pemphigus, bullous pemphigoid, dermatitis herpetiformis, and noninflammatory blistering diseases: epidermolysis bullosa and porphyria; disorders of epidermal appendages, including but not limited to, acne vulgaris; panniculitis, including but not limited to, erythema nodosum and erythema induratum; and infection and infestation, such as verrucae, molluscum contagiosum, impetigo, superficial fungal infections, and arthropod bites, stings, and infestations.

The 44590 molecules can play an important role in the regulation of metabolism or pain disorders. Diseases of metabolic imbalance include, but are not limited to, obesity, anorexia nervosa, cachexia, lipid disorders, and diabetes. Examples of pain disorders include, but are not limited to, pain response elicited during various forms of tissue injury, *e.g.,* inflammation, infection, and ischemia, usually referred to as hyperalgesia (described in, for example, Fields (1987) *Pain,* New York:McGraw-Hill); pain associated with musculoskeletal disorders, *e.g.*, joint pain; tooth pain; headaches; pain associated with surgery; pain related to irritable bowel syndrome; or chest pain.

ABC transporter-associated or other 44590associated disorders also can include hormonal disorders, such as conditions or diseases in which the production and/or regulation of hormones in an organism is aberrant. Examples of such disorders and diseases include type I and type II diabetes mellitus, pituitary disorders (*e.g*., growth disorders), thyroid disorders *(e.g.,* hypothyroidism or hyperthyroidism), and reproductive or fertility disorders *(e.g.,* disorders which affect the organs of the reproductive system, *e.g.,* the prostate gland, the uterus, or the vagina; disorders which involve an imbalance in the levels of a reproductive hormone in a subject; disorders affecting the ability of a subject to reproduce; and disorders affecting secondary sex characteristic development, e.g., adrenal hyperplasia).

Neurological disorders include CNS, cognitive and neurodegenerative disorders, Examples of neurological disorders include, but are not limited to, autonomic function disorders such as hypertension and sleep disorders, and neuropsychiatric disorders, such as depression, schizophrenia, schizoaffective disorder, Korsakoff's psychosis, alcoholism, anxiety disorders, or phobic disorders; learning or memory disorders, *e.g.,* amnesia or age-related memory loss, attention deficit disorder, dysthymic disorder, major depressive disorder, mania, obsessive-compulsive disorder, psychoactive substance use disorders, anxiety, phobias, panic disorder, as well as bipolar affective disorder, *e.g.,* severe bipolar affective (mood) disorder (BP-1), and bipolar affective neurological disorders, *e.g.,* migraine and obesity. Such neurological disorders include, for example, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states--global cerebral ischemia and focal cerebral ischemia--infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicella-zoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyclinating diseases, including multiple sclerosis, multiple sclerosis variants, acute disseminated encephalomyelitis and acute necrotizing hemorrhagic encephalomyelitis, and other diseases with demyelination; degenerative discases, such as degenerative diseases affecting the cerebral cortex, including Alzheimer's disease and Pick's disease, degenerative diseases of basal ganglia and brain stem, including Parkinsonism, idiopathic Parkinson's disease (paralysis agitans) and other Lewy diffuse body diseases, progressive supranuclear palsy, corticobasal degenration, multiple system atrophy, including striatonigral degenration, Shy-Drager syndrome, and olivopontocerebellar atrophy, and Huntington's disease, senile dementia, Gilles de la Tourettc's syndrome, epilepsy, and Jakob-Creutzfieldt disease; spinocerebellar degenerations, including spinocerebellar ataxias, including Friedreich ataxia, and ataxia-telanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease. Further CNS-related disorders include, for example, those listed in the American Psychiatric Association's Diagnostic and Statistical manual of Mental Disorders (DSM), the most current version of which is incorporated herein by reference in its entirety.

Examples of cellular proliferative and/or differentiative disorders include cancer, *e.g.,* carcinoma, sarcoma, metastatic disorders or hematopoietic neoplastic disorders, *e.g.,* leukemias. A metastatic tumor can arise from a multitude of primary tumor types, including but not limited to those of prostate, colon, lung, breast and liver origin.

As used herein, the term "cancer" (also used interchangeably with the terms, "hyperprolifcrative" and "neoplastic") refers to cells having the capacity for autonomous growth, i.e., an abnormal state or condition characterized by rapidly proliferating cell growth. Cancerous disease states may be categorized as pathologic, i.e., characterizing or constituting a disease state, *e.g.,* malignant tumor growth, or may be categorized as non-pathologic, i.e., a deviation from normal but not associated with a disease state, *e.g.,* cell proliferation associated with wound repair. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, *e.g.,* which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

The 44590 molecules of the invention can be used to monitor, treat and/or diagnose a variety of proliferative disorders. Such disorders include hematopoietic neoplastic disorders. As used herein, the term "hematopoietic neoplastic disorders" includes diseases involving hyperplastic/neoplastic cells of hematopoietic origin, *e.g.,* arising from myeloid, lymphoid or erythroid lineages, or precursor cells thereof. Preferably, the diseases arise from poorly differentiated acute leukemias, *e.g.,* erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus (1991) *Cril Rev. in Oncol*./*Hemotol*. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

The 44590 nucleic acid and protein of the invention can be used to treat and/or diagnose a variety of immune, *e.g*., inflammatory, (*e*.*g.* respiratory inflammatory) disorders. Examples of immune disorders or diseases include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, inflammatory bowel disease, *e.g.* Crohn's disease and ulcerative colitis, aphthous ulcer, iritis, conjunctivitis, keratoconjunctivitis, asthma, allergic asthma, chronic obstructive pulmonary disease, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

As used herein, disorders involving the heart, or "cardiovascular disease" or a "cardiovascular disorder" includes a disease or disorder which affects the cardiovascular system, e.g., the heart, the blood vessels, and/or the blood. A cardiovascular disorder can be caused by an imbalance in arterial pressure, a malfunction of the heart, or an occlusion of a blood vessel, *e.g.,* by a thrombus. A cardiovascular disorder includes, but is not limited to disorders such as arteriosclerosis, atherosclerosis, cardiac hypertrophy, ischemia reperfusion injury, restenosis, arterial inflammation, vascular wall remodeling, ventricular remodeling, rapid ventricular pacing, coronary microembolism, tachycardia, bradycardia, pressure overload, aortic bending, coronary artery ligation, vascular heart disease, valvular disease, including but not limited to, valvular degeneration caused by calcification, rheumatic heart disease, endocarditis, or complications of artificial valves; atrial fibrillation, long-QT syndrome, congestive heart failure, sinus node dysfunction, angina, heart failure, hypertension, atrial fibrillation, atrial flutter, pericardial disease, including but not limited to, pericardial effusion and pericarditis; cardiomyopathies, *e.g*., dilated cardiomyopathy or idiopathic cardiomyopathy, myocardial infarction, coronary artery disease, coronary artery spasm, ischemic disease, arrhythmia, sudden cardiac death, and cardiovascular developmental disorders *(e.g.,* arteriovenous malformations, arteriovenous fistulae, raynaud's syndrome, neurogenic thoracic outlet syndrome, causalgia/reflex sympathetic dystrophy, hemangioma, aneurysm, cavernous angioma, aortic valve stenosis, atrial septal defects, atrioventricular canal, coarctation of the aorta, ebsteins anomaly, hypoplastic left heart syndrome, interruption of the aortic arch, mitral valve prolapse, ductus arteriosus, patent foramen ovale, partial anomalous pulmonary venous return, pulmonary atresia with ventricular septal defect, pulmonary atresia without ventricular septal defect, persistance of the fetal circulation, pulmonary valve stenosis, single ventricle, total anomalous pulmonary venous return, transposition of the great vessels, tricuspid atresia, truncus arteriosus, ventricular septal defects). A cardiovascular disease or disorder also can include an endothelial cell disorder.

As used herein, an "endothelial cell disorder" includes a disorder characterized by aberrant, unregulated, or unwanted endothelial cell activity, *e.g.,* proliferation, migration, angiogenesis, or vascularization; or aberrant expression of cell surface adhesion molecules or genes associated with angiogenesis, *e.g.,* TIE-2, FLT and FLK. Endothelial cell disorders include tumorigenesis, tumor metastasis, psoriasis, diabetic retinopathy, endometriosis, Grave's disease, ischemic disease (*e.g*., atherosclerosis), and chronic inflammatory diseases (e.g., rheumatoid arthritis).

Disorders which can be treated or diagnosed by methods described herein include, but are not limited to, disorders associated with an accumulation in the liver of fibrous tissue, such as that resulting from an imbalance between production and degradation of the extracellular matrix accompanied by the collapse and condensation of preexisting fibers. The methods described herein can be used to diagnose or treat hepatocellular necrosis or injury induced by a wide variety of agents including processes which disturb homeostasis, such as an inflammatory process, tissue damage resulting from toxic injury or altered hepatic blood flow, and infections (*e.g.*, bacterial, viral and parasitic). For example, the methods can be used for the early detection of hepatic injury, such as portal hypertension or hepatic fibrosis. In addition, the methods can be employed to detect liver fibrosis attributed to inborn errors of metabolism, for example, fibrosis resulting from a storage disorder such as Gaucher's disease (lipid abnormalities) or a glycogen storage disease, A1-antitrypsin deficiency; a disorder mediating the accumulation (*e.g.,* storage) of an exogenous substance, for example, hemochromatosis (iron-overload syndrome) and copper storage diseases (Wilson's disease), disorders resulting in the accumulation of a toxic metabolite (*e.g.,* tyrosinemia, fructosemia and galactosemia) and peroxisomal disorders (*e.g.,* Zellweger syndrome). Additionally, the methods described herein can be used for the early detection and treatment of liver injury associated with the administration of various chemicals or drugs, such as for example, methotrexate, isonizaid, oxyphenisatin, methyldopa, chlorpromazine, tolbutamide or alcohol, or which represents a hepatic manifestation of a vascular disorder such as obstruction of either the intrahepatic or extrahepatic bile flow or an alteration in hepatic circulation resulting, for example, from chronic heart failure, veno-occlusive disease, portal vein thrombosis or Budd-Chiari syndrome.

Aberrant expression and/or activity of 44590 molecules can mediate disorders associated with bone metabolism. "Bone metabolism" refers to direct or indirect effects in the formation or degeneration of bone structures, *e.g.*, bone formation, bone resorption, etc., which can ultimately affect the concentrations in serum of calcium and phosphate. This term also includes activities mediated by 44590 molecules in bone cells, *e.g.* osteoclasts and osteoblasts, that can in turn result in bone formation and degeneration. For example, 44590 molecules can support different activities of bone resorbing osteoclasts such as the stimulation of differentiation of monocytes and mononuclear phagocytes into osteoclasts. Accordingly, 44590 molecules that modulate the production of bone cells can influence bone formation and degeneration, and thus can be used to treat bone disorders. Examples of such disorders include, but are not limited to, osteoporosis, osteodystrophy, osteomalacia, rickets, osteitis fibrosa cystica, renal osteodystrophy, osteosclerosis, anti-convulsant treatment, osteopenia, fibrogenesis-imperfecta ossium, secondary hyperparathyrodism, hypoparathyroidism, hyperparathyroidism, cirrhosis, obstructive jaundice, drug induced metabolism, medullary carcinoma, chronic renal disease, rickets, sarcoidosis, glucocorticoid antagonism, malabsorption syndrome, steatorrhea, tropical sprue, idiopathic hypercalcemia and milk fever.
Additionally, 44590 molecules can play an important role in the etiology of certain viral diseases, including but not limited to Hepatitis B, Hepatitis C and Herpes Simplex Virus (HSV). Modulators of 44590 activity could be used to control viral diseases. The modulators can be used in the treatment and/or diagnosis of viral infected tissue or virus-associated tissue fibrosis, especially liver and liver fibrosis. Also, 44590 modulators can be used in the treatment and/or diagnosis of virus-associated carcinoma, especially hepatocellular cancer.

As discussed, successful treatment of 44590 disorders can be brought about by techniques that serve to inhibit the expression or activity of target gene products. For example, compounds, *e.g*., an agent identified using an assays described above, that proves to exhibit negative modulatory activity, can be used in accordance with the invention to prevent and/or ameliorate symptoms of 44590 disorders. Such molecules can include, but are not limited to peptides, phosphopeptides, small organic or inorganic molecules, or antibodies (including, for example, polyclonal, monoclonal, humanized, human, anti-idiotypic, chimeric or single chain antibodies, and Fab, F(ab')₂ and Fab expression library fragments, scFV molecules, and epitope-binding fragments thereof).

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to co-administer normal target gene protein into the cell or tissue in order to maintain the requisite level of cellular or tissue target gene activity.

Another method by which nucleic acid molecules can be utilized in treating or preventing a disease characterized by 44590 expression is through the use of aptamer molecules specific for 44590 protein. Aptamers are nucleic acid molecules having a tertiary structure which permits them to specifically or selectively bind to protein ligands (see, *e.g.*, Osborne *et al.* (1997) *Curr. Opin. Chem Biol.* 1: 5-9; and Patel (1997) *Curr Opin Chem Biol* 1:32-46). Since nucleic acid molecules can in many cases be more conveniently introduced into target cells than therapeutic protein molecules can be, aptamers offer a method by which 44590 protein activity can be specifically decreased without the introduction of drugs or other molecules which can have pluripotent effects.

Antibodies can be generated that are both specific for target gene product and that reduce target gene product activity. Such antibodies can, therefore, by administered in instances whereby negative modulatory techniques are appropriate for the treatment of 44590 disorders. For a description of antibodies, see the Antibody section above.

In circumstances wherein injection of an animal or a human subject with a 44590 protein or epitope for stimulating antibody production is harmful to the subject, it is possible to generate an immune response against 44590 through the use of anti-idiotypic antibodies (see, for example, Herlyn (1999) *Ann Med* 31:66-78; and Bhattacharya-Chatterjee and Foon (1998) *Cancer Treat Res.* 94:51-68). If an anti-idiotypic antibody is introduced into a mammal or human subject, it should stimulate the production of anti-anti-idiotypic antibodies, which should be specific to the 44590 protein. Vaccines directed to a disease characterized by 44590 expression can also be generated in this fashion.

In instances where the target antigen is intracellular and whole antibodies are used, internalizing antibodies can be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see *e.g.,* Marasco *et al.* (1993) *Proc. Natl. Acad. Sci. USA* 90:7889-7893).

The identified compounds that inhibit target gene expression, synthesis and/or activity can be administered to a patient at therapeutically effective doses to prevent, treat or ameliorate 44590 disorders. A therapeutically effective dose refers to that amount of the compound sufficient to result in amelioration of symptoms of the disorders. Toxicity and therapeutic efficacy of such compounds can be determined by standard pharmaceutical procedures as described above.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of determination of effective dose for an individual is the ability to directly assay levels of "free" and "bound" compound in the serum of the test subject. Such assays can utilize antibody mimics and/or "biosensors" that have been created through molecular imprinting techniques. The compound which is able to modulate 44590 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell *et al* (1996) *Current Opinion in Biotechnology* 7:89-94 and in Shea (1994) *Trends in Polymer Science* 2:166-173. Such "imprinted" affinity matrixes are amenable to ligand-binding assays, whereby the immobilized monoclonal antibody component is replaced by an appropriately imprinted matrix. An example of the use of such matrixes in this way can be seen in Vlatakis *et al* (1993) *Nature* 361:645-647. Through the use of isotope-labeling, the "free" concentration of compound which modulates the expression or activity of 44590 can be readily monitored and used in calculations of IC₅₀.

Such "imprinted" affinity matrixes can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. A rudimentary example of such a "biosensor" is discussed in Kriz *et al* (1995) *Analytical Chemistry* 67:2142-2144.

Another aspect of the invention pertains to methods of modulating 44590 expression or activity for therapeutic purposes. Accordingly, in an exemplary embodiment, the modulatory method of the invention involves contacting a cell with a 44590 or agent that modulates one or more of the activities of 44590 protein activity associated with the cell. An agent that modulates 44590 protein activity can be an agent as described herein, such as a nucleic acid or a protein, a naturally-occurring target molecule of a 44590 protein (*e*.*g*., a 44590 substrate or receptor), a 44590 antibody, a 44590 agonist or antagonist, a peptidomimetic of a 44590 agonist or antagonist, or other small molecule.

In one embodiment, the agent stimulates one or 44590 activities. Examples of such stimulatory agents include active 44590 protein and a nucleic acid molecule encoding 44590. In another embodiment, the agent inhibits one or more 44590 activities. Examples of such inhibitory agents include antisense 44590 nucleic acid molecules, anti-44590 antibodies, and 44590 inhibitors. These modulatory methods can be performed *in vitro* (*e.g*., by culturing the cell with the agent) or, alternatively, *in vivo (e.g.,* by administering the agent to a subject). As such, the present invention provides methods of treating an individual afflicted with a disease or disorder characterized by aberrant or unwanted expression or activity of a 44590 protein or nucleic acid molecule. In one embodiment, the method involves administering an agent (*e.g*., an agent identified by a screening assay described herein), or combination of agents that modulates (*e.g*., up regulates or down regulates) 44590 expression or activity. In another embodiment, the method involves administering a 44590 protein or nucleic acid molecule as therapy to compensate for reduced, aberrant, or unwanted 44590 expression or activity.

Stimulation of 44590 activity is desirable in situations in which 44590 is abnormally downregulated and/or in which increased 44590 activity is likely to have a beneficial effect. For example, stimulation of 44590 activity is desirable in situations in which a 44590 is downregulated and/or in which increased 44590 activity is likely to have a beneficial effect. Likewise, inhibition of 44590 activity is desirable in situations in which 44590 is abnormally upregulated and/or in which decreased 44590 activity is likely to have a beneficial effect.

### Pharmacogenomics

The 44590 molecules of the present invention, as well as agents, or modulators which have a stimulatory or inhibitory effect on 44590 activity *(e.g.,* 44590 gene expression) as identified by a screening assay described herein can be administered to individuals to treat (prophylactically or therapeutically) 44590-associated disorders *(e.g.,* aberrant or deficient function or expression) associated with aberrant or unwanted 44590 activity. In conjunction with such treatment, pharmacogenomics (i.e., the study of the relationship between an individual's genotype and that individual's response to a foreign compound or drug) can be considered. Differences in metabolism of therapeutics can lead to severe toxieity or therapeutic failure by altering the relation between dose and blood concentration of the pharmacologically active drug. Thus, a physician or clinician can consider applying knowledge obtained in relevant pharmacogenomics studies in determining whether to administer a 44590 molecule or 44590 modulator as well as tailoring the dosage and/or therapeutic regimen of treatment with a 44590 molecule or 44590 modulator.

Pharmacogenomics deals with clinically significant hereditary variations in the response to drugs due to altered drug disposition and abnormal action in affected persons. See, for example, Eichelbaum *et al.* (1996) *Clin. Exp. Pharmacol. Physiol*. 23:983-985 and Linder *et al.* (1997) *Clin. Chem.* 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers *(e.g.,* a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP can occur once per every 1000 bases of DNA. A SNP can be involved in a disease process, however, the vast majority can not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that can be common among such genetically similar individuals.

Alternatively, a method termed the "candidate gene approach", can be utilized to identify genes that predict drug response. According to this method, if a gene that encodes a drug's target is known *(e.g.,* a 44590 protein of the present invention), all common variants of that gene can be fairly easily identified in the population and it can be determined if having one version of the gene versus another is associated with a particular drug response.

Alternatively, a method termed the "gene expression profiling", can be utilized to identify genes that predict drug response. For example, the gene expression of an animal dosed with a drug (*e.g*., a 44590 molecule or 44590 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 44590 molecule or 44590 modulator, such as a modulator identified by one of the exemplary screening assays described herein.

The present invention further provides methods for identifying new agents, or combinations, that are based on identifying agents that modulate the activity of one or more of the gene products encoded by one or more of the 44590 genes of the present invention, wherein these products can be associated with resistance of the cells to a therapeutic agent. Specifically, the activity of the proteins encoded by the 44590 genes of the present invention can be used as a basis for identifying agents for overcoming agent resistance. By blocking the activity of one or more of the resistance proteins, target cells, *e.g*., human cells, will become sensitive to treatment with an agent to which the unmodified target cells were resistant.

Monitoring the influence of agents (*e.g*., drugs) on the expression or activity of a 44590 protein can be applied in clinical trials. For example, the effectiveness of an agent determined by a screening assay as described herein to increase 44590 gene expression, protein levels, or upregulate 44590 activity, can be monitored in clinical trials of subjects exhibiting decreased 44590 gene expression, protein levels, or downregulated 44590 activity. Alternatively, the effectiveness of an agent determined by a screening assay to decrease 44590 gene expression, protein levels, or downregulate 44590 activity, can be monitored in clinical trials of subjects exhibiting increased 44590 gene expression, protein levels, or upregulated 44590 activity. In such clinical trials, the expression or activity of a 44590 gene, and preferably, other genes that have been implicated in, for example, a ABC transporter-associated or another 44590-associated disorder can be used as a "read out" or markers of the phenotype of a particular cell.

### Other Embodiments

In another aspect, the invention features a method of analyzing a plurality of capture probes. The method is useful, *e.g.,* to analyze gene expression. The method includes: providing a two dimensional array having a plurality of addresses, each address of the plurality being positionally distinguishable from each other address of the plurality, and each address of the plurality having a unique capture probe, *e*.*g*., a nucleic acid or peptide sequence, wherein the capture probes are from a cell or subject which expresses 44590 or from a cell or subject in which a 44590 mediated response has been elicited; contacting the array with a 44590 nucleic acid (preferably purified), a 44590 polypeptide (preferably purified), or an anti-44590 antibody, and thereby evaluating the plurality of capture probes. Binding, *e.g*., in the case of a nucleic acid, hybridization with a capture probe at an address of the plurality, is detected, *e.g.,* by a signal generated from a label attached to the 44590 nucleic acid, polypeptide, or antibody.

The capture probes can be a set of nucleic acids from a selected sample, *e.g.,* a sample of nucleic acids derived from a control or non-stimulated tissue or cell.

The method can include contacting the 44590 nucleic acid, polypeptide, or antibody with a first array having a plurality of capture probes and a second array having a different plurality of capture probes. The results of each hybridization can be compared, *e.g.,* to analyze differences in expression between a first and second sample. The first plurality of capture probes can be from a control sample, *e.g.,* a wild type, normal, or non-diseased, non-stimulated, sample, *e.g.,* a biological fluid, tissue, or cell sample. The second plurality of capture probes can be from an experimental sample, *e.g.,* a mutant type, at risk, disease-state or disorder-state, or stimulated, sample, *e.g.,* a biological fluid, tissue, or cell sample.

The plurality of capture probes can be a plurality of nucleic acid probes each of which specifically hybridizes, with an allele of 44590. Such methods can be used to diagnose a subject, *e.g.,* to evaluate risk for a disease or disorder, to evaluate suitability of a selected treatment for a subject, to evaluate whether a subject has a disease or disorder.

The method can be used to detect SNPs, as described above.

In another aspect, the invention features, a method of analyzing 44590, *e.g.,* analyzing structure, function, or relatedness to other nucleic acid or amino acid sequences. The method includes: providing a 44590 nucleic acid or amino acid sequence; comparing the 44590 sequence with one or more preferably a plurality of sequences from a collection of sequences, *e.g.,* a nucleic acid or protein sequence database; to thereby analyze 44590.

The method can include evaluating the sequence identity between a 44590 sequence and a database sequence. The method can be performed by accessing the database at a second site, *e.g.,* over the internet. Preferred databases include GenBank™ and SwissProt.

In another aspect, the invention features, a set of oligonucleotides, useful, *e.g*., for identifying SNP's, or identifying specific alleles of 44590. The set includes a plurality of oligonucleotides, each of which has a different nucleotide at an interrogation position, *e.g.*, an SNP or the site of a mutation. In a preferred embodiment, the oligonucleotides of the plurality identical in sequence with one another (except for differences in length). The oligonucleotides can be provided with differential labels, such that an oligonucleotide which hybridizes to one allele provides a signal that is distinguishable from an oligonucleotides which hybridizes to a second allele.

The sequences of 44590 molecules are provided in a variety of mediums to facilitate use thereof. A sequence can be provided as a manufacture, other than an isolated nucleic acid or amino acid molecule, which contains a 44590 molecule. Such a manufacture can provide a nucleotide or amino acid sequence, *e.g.*, an open reading frame, in a form which allows examination of the manufacture using means not directly applicable to examining the nucleotide or amino acid sequences, or a subset thereof, as they exist in nature or in purified form.

A 44590 nucleotide or amino acid sequence can be recorded on computer readable media. As used herein, "computer readable media" refers to any medium that can be read and accessed directly by a computer. Such media include, but are not limited to: magnetic storage media, such as floppy discs, hard disc storage medium, and magnetic tape; optical storage media such as compact disc and CD-ROM; electrical storage media such as RAM, ROM, EPROM, EEPROM, and the like; and general hard disks and hybrids of these categories such as magnetic/optical storage media. The medium is adapted or configured for having thereon 44590 sequence information of the present invention.

As used herein, the term "electronic apparatus" is intended to include any suitable computing or processing apparatus of other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phones, pagers, and the like; and local and distributed processing systems.

As used herein, "recorded" refers to a process for storing or encoding information on the electronic apparatus readable medium. Those skilled in the art can readily adopt any of the presently known methods for recording information on known media to generate manufactures comprising the 44590 sequence information.

A variety of data storage structures are available to a skilled artisan for creating a computer readable medium having recorded thereon a 44590 nucleotide or amino acid sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. The skilled artisan can readily adapt any number of data processor structuring formats (*e.g.*, text file or database) in order to obtain computer readable medium having recorded thereon the nucleotide sequence information of the present invention.

By providing the 44590 nucleotide or amino acid sequences of the invention in computer readable form, the skilled artisan can routinely access the sequence information for a variety of purposes. For example, one skilled in the art can use the nucleotide or amino acid sequences of the invention in computer readable form to compare a target sequence or target structural motif with the sequence information stored within the data storage means. A search is used to identify fragments or regions of the sequences of the invention which match a particular target sequence or target motif.

The present invention therefore provides a medium for holding instructions for performing a method for determining whether a subject has a ABC transporter-associated or another 44590-associated disease or disorder or a pre-disposition to a ABC transporter-associated or another 44590-associated disease or disorder, wherein the method comprises the steps of determining 44590 sequence information associated with the subject and based on the 44590 sequence information, determining whether the subject has a ABC transporter-associated or another 44590-associated disease or disorder and/or recommending a particular treatment for the disease, disorder, or pre-disease condition.

The present invention further provides in an electronic system and/or in a network, a method for determining whether a subject has a ABC transporter-associated or another 44590-associated disease or disorder or a pre-disposition to a disease associated with 44590, wherein the method comprises the steps of determining 44590 sequence information associated with the subject, and based on the 44590 sequence information, determining whether the subject has a ABC transporter-associated or another 44590-associated disease or disorder or a pre-disposition to a ABC transporter-associated or another 44590-associated disease or disorder, and/or recommending a particular treatment for the disease, disorder, or pre-disease condition. The method may further comprise the step of receiving phenotypic information associated with the subject and/or acquiring from a network phenotypic information associated with the subject.

The present invention also provides in a network, a method for determining whether a subject has a ABC transporter-associated or another 44590-associated disease or disorder or a pre-disposition to a ABC transporter-associated or another 44590-associated disease or disorder, said method comprising the steps of receiving 44590 sequence information from the subject and/or information related thereto, receiving phenotypic information associated with the subject, acquiring information from the network corresponding to 44590 and/or corresponding to a ABC transporter-associated or another 44590-associated disease or disorder, and based on one or more of the phenotypic information, the 44590 information *(e.g.,* sequence information and/or information related thereto), and the acquired information, determining whether the subject has a ABC transporter-associated or another 44590-associated disease or disorder or a pre-disposition to a ABC transporter-associated or another 44590-associated disease or disorder. The method may further comprise the step of recommending a particular treatment for the disease, disorder, or pre-disease condition.

The present invention also provides a business method for determining whether a subject has a ABC transporter-associated or another 44590-associated disease or disorder or a pre-disposition to a ABC transporter-associated or another 44590-associated disease or disorder, said method comprising the steps of receiving information related to 44590 *(e.g.,* sequence information and/or information related thereto), receiving phenotypic information associated with the subject, acquiring information from the network related to 44590 and/or related to a ABC transporter-associated or another 44590-associated disease or disorder, and based on one or more of the phenotypic information, the 44590 information, and the acquired information, determining whether the subject has a ABC transporter-associated or another 44590-associated disease or disorder or a pre-disposition to a ABC transporter-associated or another 44590-associated disease or disorder. The method may further comprise the step of recommending a particular treatment for the disease, disorder, or pre-disease condition.

The invention also includes an array comprising a 44590 sequence of the present invention. The array can be used to assay expression of one or more genes in the array. In one embodiment, the array can be used to assay gene expression in a tissue to ascertain tissue specificity of genes in the array. In this manner, up to about 7600 genes can be simultaneously assayed for expression, one of which can be 44590. This allows a profile to be developed showing a battery of genes specifically expressed in one or more tissues.

In addition to such qualitative information, the invention allows the quantitation of gene expression. Thus, not only tissue specificity, but also the level of expression of a battery of genes in the tissue if ascertainable. Thus, genes can be grouped on the basis of their tissue expression *per se* and level of expression in that tissue. This is useful, for example, in ascertaining the relationship of gene expression in that tissue. Thus, one tissue can be perturbed and the effect on gene expression in a second tissue can be determined. In this context, the effect of one cell type on another cell type in response to a biological stimulus can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more genes in the array. This can occur in various biological contexts, as disclosed herein, for example development of a ABC transporter-associated or another 44590-associated disease or disorder, progression of ABC transporter-associated or another 44590-associated disease or disorder, and processes, such a cellular transformation associated with the ABC transporter-associated or another 44590-associated disease or disorder.

The array is also useful for ascertaining the effect of the expression of a gene on the expression of other genes in the same cell or in different cells (*e.g.*, acertaining the effect of 44590 expression on the expression of other genes). This provides, for example, for a selection of alternate molecular targets for therapeutic intervention if the ultimate or downstream target cannot be regulated.

The array is also useful for ascertaining differential expression patterns of one or more genes in normal and abnormal cells. This provides a battery of genes (*e.g*., including 44590) that could serve as a molecular target for diagnosis or therapeutic intervention.

As used herein, a "target sequence" can be any DNA or amino acid sequence of six or more nucleotides or two or more amino acids. A skilled artisan can readily recognize that the longer a target sequence is, the less likely a target sequence will be present as a random occurrence in the database. Typical sequence lengths of a target sequence are from about 10 to 100 amino acids or from about 30 to 300 nucleotide residues. However, it is well recognized that commercially important fragments, such as sequence fragments involved in gene expression and protein processing, may be of shorter length.

Computer software is publicly available which allows a skilled artisan to access sequence information provided in a computer readable medium for analysis and comparison to other sequences. A variety of known algorithms are disclosed publicly and a variety of commercially available software for conducting search means are and can be used in the computer-based systems of the present invention. Examples of such software include, but are not limited to, MacPattern (EMBL), BLASTN and BLASTX (NCBI).

Thus, the invention features a method of making a computer readable record of a sequence of a 44590 sequence which includes recording the sequence on a computer readable matrix. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention features a method of analyzing a sequence. The method includes: providing a 44590 sequence, or record, in computer readable form; comparing a second sequence to the 44590 sequence; thereby analyzing a sequence. Comparison can include comparing to sequences for sequence identity or determining if one sequence is included within the other, *e.g*., determining if the 44590 sequence includes a sequence being compared. In a preferred embodiment the 44590 or second sequence is stored on a first computer, *e.g.,* at a first site and the comparison is performed, read, or recorded on a second computer, *e.g.*, at a second site. *E.g*., the 44590 or second sequence can be stored in a public or proprietary database in one computer, and the results of the comparison performed, read, or recorded on a second computer. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

The contents of all references, patents and published patent applications cited throughout this application are incorporated herein by reference.

### EXEMPLIFICATION

### Gene Expression Analysis

Total RNA was prepared from various human tissues by a single step extraction method using RNA STAT-60 according to the manufacturer's instructions (TelTest, Inc). Each RNA preparation was treated with DNase I (Ambion) at 37°C for 1 hour. DNAse I treatment was determined to be complete if the sample required at least 38 PCR amplification cycles to reach a threshold level of fluorescence using β-2 microglobulin as an internal amplicon reference. The integrity of the RNA samples following DNase I treatment was confirmed by agarose gel electrophoresis and ethidium bromide staining. After phenol extraction cDNA was prepared from the sample using the SUPERSCRIPT™ Choice System following the manufacturer's instructions (GibcoBRL). A negative control of RNA without reverse transcriptase was mock reverse transcribed for each RNA sample.

Human 44590 expression was measured by TaqMan® quantitative PCR (Perkin Elmer Applied Biosystems) in cDNA prepared from a variety of normal and diseased (e.g., cancerous) human tissues or cell lines.

Probes were designed by PrimerExpress software (PE Biosystems) based on the sequence of the human 44590 genes. Each human 44590 gene probe was labeled using FAM (6-carboxyfluorcscein), and the β2-microglobulin reference probe was labeled with a different fluorescent dye, VIC. The differential labeling of the target gene and internal reference gene thus enabled measurement in same well. Forward and reverse primers and the probes for both β2-microglobulin and target gene were added to the TaqMan® Universal PCR Master Mix (PE Applied Biosystems). Although the final concentration of primer and probe could vary, each was internally consistent within a given experiment. A typical experiment contained 200nM of forward and reverse primers plus 100nM probe for β-2 microglobulin and 600 nM forward and reverse primers plus 200 nM probe for the target gene. TaqMan matrix experiments were carried out on an ABI PRISM 7700 Sequence Detection System (PE Applied Biosystems). The thermal cycler conditions were as follows: hold for 2 min at 50°C and 10 min at 95°C, followed by two-step PCR for 40 cycles of 95°C for 15 sec followed by 60°C for 1 min.

The following method was used to quantitatively calculate human 44590 gene expression in the various tissues relative to β-2 microglobulin expression in the same tissue. The threshold cycle (Ct) value is defined as the cycle at which a statistically significant incrcase in fluorescence is detected. A lower Ct value is indicative of a higher mRNA concentration. The Ct value of the human 44590 genes is normalized by subtracting the Ct value of the β-2 microglobulin gene to obtain a _{Δ}Ct value using the following formula: _{Δ}Ct=Ct_{human 44590} - Ct _{β-2 microglobulin}. Expression is then calibrated against a cDNA sample showing a comparatively low level of expression of the human 44590 gene. The _{Δ}Ct value for the calibrator sample is then subtracted from _{Δ}Ct for each tissue sample according to the following formula: _{ΔΔ}Ct=_{Δ}Ct-ₛₐₘₚₗₑ - _{Δ}Ct-_{calibrator}. Relative expression is then calculated using the arithmetic formula given by 2^{-ΔΔCt}. Expression of the target human 44590 genes in each of the tissues tested is then graphically represented as discussed in more detail below.

The results indicate high levels of 44590 expression in tumor cells associated with breast tissues; furthermore, low levels of 44590 expression are associated with normal cells of breast tissue origin. Results also intidcate high levels of 44590 expression in human skin cells.

The contents of all references, patents and published patent applications cited throughout this application are incorporated herein by reference.

### Equivalents

Those skilled in the art will recognize, or be able to ascertain using no more than routine experimentation, many equivalents to the specific embodiments of the invention described herein.

## Claims

1. An isolated nucleic acid molecule selected from the group consisting of:
a. a nucleic acid molecule comprising a nucleotide sequence which is at least 95% identical to the nucleotide sequence of SEQ ID NO:1 or SEQ ID NO:3;
b. a nucleic acid molecule comprising a fragment of at least 1380 nucleotides of the nucleotide sequence of SEQ ID NO:1 or SEQ ID NO:3;
c. a nucleic acid molecule which encodes a polypeptide comprising the amino acid sequence of SEQ ID NO:2;
d. a nucleic acid molecule which encodes a fragment of a polypeptide comprising the amino acid sequence of SEQ ID NO:2 wherein the fragment comprises at least 460 contiguous amino acids of SEQ ID NO:2; and
e. a nucleic acid molecule which encodes a naturally occurring allelic variant of a polypeptide comprising the amino acid sequence of SEQ ID NO:2, wherein the nucleic acid molecule hybridizes to a nucleic acid molecule comprising SEQ ID NO:1 or 3, or a complement thereof, under stringent conditions.

2. The isolated nucleic acid molecule of claim 1, which is at least 99% identical to the nucleotide sequence of SEQ ID NO:1 or SEQ ID NO:3,

3. The isolated nucleic acid molecule of claim 1 or 2, which encodes a fragment of a polypeptide comprising the amino acid sequence of SEQ ID NO:2, wherein the fragment comprises at least 1380 contiguous amino acids of SEQ ID NO:2.

4. The isolated nucleic acid molecule of claim 1, 2 or 3, which is selected from the group consisting of:
a. a nucleic acid comprising the nucleotide sequence of SEQ ID NO: 1 or SEQ ID NO:3; and
b. a nucleic acid molecule which encodes a polypeptide comprising the amino acid sequence of SEQ ID NO:2.

5. The nucleic acid molecule of any of claims 1 to 4, further comprising vector nucleic acid sequences.

6. A host cell which contains the nucleic acid molecule of any of claims 1 to 5.

7. An isolated polypeptide selected from the group consisting of:
a. a polypeptide which is encoded by a nucleic acid molecule comprising a nucleotide sequence which is at least 95% identical to a nucleic acid comprising the nucleotide sequence of SEQ ID NO:1 or SEQ ID NO:3, or a complement thereof;
b. a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO: 1 or SEQ ID NO:3; and
c. a fragment of a polypeptide comprising the amino acid sequence of SEQ ID NO:2, wherein the fragment comprises at least 1380 contiguous amino acids of SEQ ID NO:2.

8. The isolated polypeptide of claim 7, comprising a polypeptide which is encoded by a nucleic acid molecule comprising a nucleotide sequence which is at least 99% identical to a nucleic acid comprising the nucleotide sequence of SEQ ID NO:1 or SEQ ID NO:3, or a complement thereof.

9. The isolated polypeptide of claim 7 or 8, comprising the amino acid sequence of SEQ ID NO:2.

10. The polypeptide of claim 7, 8 or 9, further comprising heterologous amino acid sequences.

11. An antibody which selectively binds to a polypeptide of any of claims 7 to 10.

12. The antibody of claim 11 which is a monoclonal antibody.

13. A method for producing a polypeptide selected from the group consisting of:
a. a polypeptide comprising the amino acid sequence of SEQ ID NO:2;
b. a polypeptide comprising a fragment of the amino acid sequence of SEQ ID NO:2, wherein the fragment comprises at least 182 contiguous amino acids of SEQ ID NO:2; and
c. a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO:1 or SEQ ID NO:3, or a complement thereof under stringent conditions;
wherein the host cell of claim 6 is cultured under conditions in which the nucleic acid molecule is expressed.

14. A method for detecting the presence of a polypeptide of any of claims 7 to 10 in a sample, comprising:
contacting the sample with a compound which selectively binds to a polypeptide of any of claims 7 to 10, as the case may be; and
determining whether the compound binds to the polypeptide in the sample.

15. A kit comprising a compound which selectively binds to a polypeptide of any of cliams 7 to 10 and instructions for use.

16. A method for detecting the presence of a nucleic acid molecule of claims 1 to 5 in a sample, comprising the steps of:
contacting the sample with a nucleic acid probe or primer which selectively hybridizes to the nucleic acid molecule; and
determining whether the nucleic acid probe or primer binds to a nucleic acid molecule in a sample.

17. A kit comprising a compound which selectively hybridizes to a nucleic acid molecule of any of claims 1 to 5 and instructions for use.

18. A method for identifying a compound which binds to a polypeptide of claims 7 to 10, comprising the steps of:
contacting a polypeptide, or a cell expressing a polypeptide of any of claims 7 to 10, as the case may be, with a test compound; and
determining whether the polypeptide binds to the test compound.

19. A method for modulating the activity of a polypeptide of any of claims 7 to 10, comprising contacting a polypeptide or a cell expressing a polypeptide of any of claims 7 to 10, as the case may be, with a compound which binds to the polypeptide in a sufficient concentration to modulate the activity of the polypeptide.

20. A method for identifying a compound which modulates the activity of a polypeptide of any of claims 7 to 10, comprising:
contacting a polypeptide of any of claims 7 to 10, as the case may be, with a test compound; and
determining the effect of the test compound on the activity of the polypeptide to thereby identify a compound which modulates the activity of the polypeptide.


SEQUENCE LISTING

<110> Curtis, Rory A.J.

<120> 44590, A Human ABC Transporter Family
  Member and Uses Therefor

<130> P101131EP

<150> 60/325,427

<151> 2001-09-27

<160> 5

<170> FastSEQ for Windows Version 4.0

<210> 1
<211> 6747
<212> DNA
<213> Homo sapiens

<220>
<221> CDS
<222> (126)...(5915)

<400> 1
accacgcgtc cgagagatcc cggcagtctt acctcatcgg actcaccctt ctgcactact 60
taaacattta caacttcaca tacaaggtgt ttttcccgag gaaagatcaa aagccagtag 120
aaaag atg atg gag ctc ttc ata aga cta aaa gag att ctc aat cag atg 170
      Met Met Glu Leu Phe Ile Arg Leu Lys Glu Ile Leu Asn Gln Met
       1               5                  10                  15

gct tct ggc aca cat ccg ctg cta gac aaa atg aga tcc ctg aag caa   218
Ala Ser Gly Thr His Pro Leu Leu Asp Lys Met Arg Ser Leu Lys Gln
                20                  25                  30

atg cat ctg ccc aga agt gtt cca tta aca cag gca atg tac aga agc   266
Met His Leu Pro Arg Ser Val Pro Leu Thr Gln Ala Met Tyr Arg Ser
            35                  40                  45

aac cga atg aac aca tca caa gga tca ttt agc acc atc tcc caa gca   314
Asn Arg Met Asn Thr Ser Gln Gly Ser Phe Ser Thr Ile Ser Gln Ala
        50                  55                  60

tta tgt tct caa gga att acc act gaa tat tta act gcc atg ctg ccc   362
Leu Cys Ser Gln Gly Ile Thr Thr Glu Tyr Leu Thr Ala Met Leu Pro
    65                  70                  75

tct tcc cag agg cca aaa ggc aac cac acc aag gat ttt ttg act tat   410
Ser Ser Gln Arg Pro Lys Gly Asn His Thr Lys Asp Phe Leu Thr Tyr
80                  85                  90                  95

aaa tta act aaa gag caa att gct tca aaa tat gga att ccc ata aat   458
Lys Leu Thr Lys Glu Gln Ile Ala Ser Lys Tyr Gly Ile Pro Ile Asn
                100                 105                 110

acc aca cca ttt tgc ttc tcc ctt tat aaa gac atc att aac atg ccc    506
Thr Thr Pro Phe Cys Phe Ser Leu Tyr Lys Asp Ile Ile Asn Met Pro
            115                 120                 125

gct gga cct gtg att tgg gct ttc ttg aaa cct atg ttg ttg gga aga    554
Ala Gly Pro Val Ile Trp Ala Phe Leu Lys Pro Met Leu Leu Gly Arg
        130                 135                 140

att ttg tat gca cca tat aac cca gtc aca aag gca ata atg gaa aag    602
Ile Leu Tyr Ala Pro Tyr Asn Pro Val Thr Lys Ala Ile Met Glu Lys
    145                 150                 155

tcc aat gta act ctg aga cag ctg gcg gaa tta aga gaa aaa tct caa    650
Ser Asn Val Thr Leu Arg Gln Leu Ala Glu Leu Arg Glu Lys Ser Gln
160                 165                 170                 175

gag tgg atg gat aag tcg cca ctt ttc atg aat tcc ttc cat ctg tta    698
Glu Trp Met Asp Lys Ser Pro Leu Phe Met Asn Ser Phe His Leu Leu
                180                 185                 190

aac cag gca att cca atg ctc cag aat act cta agg aac cct ttt gtg    746
Asn Gln Ala Ile Pro Met Leu Gln Asn Thr Leu Arg Asn Pro Phe Val
            195                 200                 205

caa gtt ttt gta aag ttc tcc gtg gga ctc gat gct gtt gaa cta ttg    794
Gln Val Phe Val Lys Phe Ser Val Gly Leu Asp Ala Val Glu Leu Leu
        210                 215                 220

aaa cag ata gat gaa ctc gat att cta aga ctg aaa tta gag aac aac    842
Lys Gln Ile Asp Glu Leu Asp Ile Leu Arg Leu Lys Leu Glu Asn Asn
    225                 230                 235

att gac atc atc gat cag ctt aac aca cta tct tcc ctg aca gta aat    890
Ile Asp Ile Ile Asp Gln Leu Asn Thr Leu Ser Ser Leu Thr Val Asn
240                 245                 250                 255

att tcc tct tgt gta tta tat gac cgt att cag gca gca aaa acc ata    938
Ile Ser Ser Cys Val Leu Tyr Asp Arg Ile Gln Ala Ala Lys Thr Ile
                260                 265                 270

gat gaa atg gag aga gag gct aaa agg ctc tac aaa agc aac gaa ctc    986
Asp Glu Met Glu Arg Glu Ala Lys Arg Leu Tyr Lys Ser Asn Glu Leu
            275                 280                 285

ttt gga agt gtt att ttt aag ctt cct tct aac aga agc tgg cac aga    1034
Phe Gly Ser Val Ile Phe Lys Leu Pro Ser Asn Arg Ser Trp His Arg
        290                 295                 300

ggc tat gac tct gga aat gtc ttt ctt cct cct gtc ata aaa tat acc    1082
Gly Tyr Asp Ser Gly Asn Val Phe Leu Pro Pro Val Ile Lys Tyr Thr
    305                 310                 315

atc cgg atg agt ctc aag acc gca cag acc aca aga agc cta aga acc    1130
Ile Arg Met Ser Leu Lys Thr Ala Gln Thr Thr Arg Ser Leu Arg Thr
320                 325                 330                 335

aag att tgg gct cca ggg cca cac aat tct ccg tca cac aac cag atc    1178
Lys Ile Trp Ala Pro Gly Pro His Asn Ser Pro Ser His Asn Gln Ile
                340                 345                 350

tat ggc agg gct ttt att tat tta cag gat agt att gaa aga gca atc    1226

Tyr Gly Arg Ala Phe Ile Tyr Leu Gln Asp Ser Ile Glu Arg Ala Ile
            355                 360                 365

att gaa ttg caa act gga agg aac tcc cag gaa ata gca gtc cag gtt 1274
Ile Glu Leu Gln Thr Gly Arg Asn Ser Gln Glu Ile Ala Val Gln Val
        370                 375                 380

caa gca att cct tat ccc tgc ttc atg aaa gac aac ttc cta acc agt 1322
Gln Ala Ile Pro Tyr Pro Cys Phe Met Lys Asp Asn Phe Leu Thr Ser
    385                 390                 395

gtc tct tat tct ctt cca att gtg ctt atg gtt gcc tgg gtt gta ttt 1370
Val Ser Tyr Ser Leu Pro Ile Val Leu Met Val Ala Trp Val Val Phe
400                 405                 410                 415

ata gct gcc ttt gta aaa aag ctt gtc tat gag aaa gac ctc cgg ctt 1418
Ile Ala Ala Phe Val Lys Lys Leu Val Tyr Glu Lys Asp Leu Arg Leu
                420                 425                 430

cat gag tac atg aag atg atg ggt gtg aac tcc tgc agc cat ttc ttt 1466
His Glu Tyr Met Lys Met Met Gly Val Asn Ser Cys Ser His Phe Phe
            435                 440                 445

gcc tgg ctt ata gag agt gtt gga ttt tta ctg gtt acc atc gtg atc 1514
Ala Trp Leu Ile Glu Ser Val Gly Phe Leu Leu Val Thr Ile Val Ile
        450                 455                 460

ctc atc att ata ctc aag ttt ggc aat att ctt cct aaa aca aat ggg 1562
Leu Ile Ile Ile Leu Lys Phe Gly Asn Ile Leu Pro Lys Thr Asn Gly
    465                 470                 475

ttc att ttg ttc ctg tat ttt tcg gac tac agc ttc tcg gtt att gcc 1610
Phe Ile Leu Phe Leu Tyr Phe Ser Asp Tyr Ser Phe Ser Val Ile Ala
480                 485                 490                 495

atg agc tat ctt atc agt gtc ttc ttc aac aac acc aac att gca gct 1658
Met Ser Tyr Leu Ile Ser Val Phe Phe Asn Asn Thr Asn Ile Ala Ala
                500                 505                 510

ctg atc gga agc ctc atc tac atc att gcc ttc ttt cca ttt att gtt 1706
Leu Ile Gly Ser Leu Ile Tyr Ile Ile Ala Phe Phe Pro Phe Ile Val
            515                 520                 525

ctg gtt aca gtg gag aat gag ttg agc tat gta ttg aaa gtg ttc atg 1754
Leu Val Thr Val Glu Asn Glu Leu Ser Tyr Val Leu Lys Val Phe Met
        530                 535                 540

agc ctg ctg tcc cca aca gca ttc agc tat gca aac caa tac att gca 1802
Ser Leu Leu Ser Pro Thr Ala Phe Ser Tyr Ala Asn Gln Tyr Ile Ala
    545                 550                 555

cga tac gaa gaa cag ggc att ggt ctt cag tgg gaa aat atg tac acc 1850
Arg Tyr Glu Glu Gln Gly Ile Gly Leu Gln Trp Glu Asn Met Tyr Thr
560                 565                 570                 575

tcc ccg gtt cag gat gac acc acc tca ttt ggc tgg ctg tgc tgt cta 1898
Ser Pro Val Gln Asp Asp Thr Thr Ser Phe Gly Trp Leu Cys Cys Leu
                580                 585                 590

atc cta gct gac tct ttc att tat ttc ctt att gct tgg tat gtc agg 1946
Ile Leu Ala Asp Ser Phe Ile Tyr Phe Leu Ile Ala Trp Tyr Val Arg

            595                 600                 605

aat gtc ttc cca ggg aca tac ggt atg gca gct ccc tgg tat ttt cca 1994
Asn Val Phe Pro Gly Thr Tyr Gly Met Ala Ala Pro Trp Tyr Phe Pro
        610                 615                 620

att ctt cct tcc tat tgg aag gag cga ttt ggg tgt gca gag gtg aag 2042
Ile Leu Pro Ser Tyr Trp Lys Glu Arg Phe Gly Cys Ala Glu Val Lys
    625                 630                 635

cct gag aag agc aat ggc ctc atg ttt act aac atc atg atg cag aat 2090
Pro Glu Lys Ser Asn Gly Leu Met Phe Thr Asn Ile Met Met Gln Asn
640                 645                 650                 655

acc aac cca tct gcc agt cct gaa tac atg ttt tcc tct aac atc gag 2138
Thr Asn Pro Ser Ala Ser Pro Glu Tyr Met Phe Ser Ser Asn Ile Glu
                660                 665                 670

cct gaa cct aaa gat ctc aca gtc ggg gtt gcc ctg cat ggg gtc aca 2186
Pro Glu Pro Lys Asp Leu Thr Val Gly Val Ala Leu His Gly Val Thr
            675                 680                 685

aag atc tat ggc tca aaa gtt gct gtt gat aac ctc aat ctg aac ttt 2234
Lys Ile Tyr Gly Ser Lys Val Ala Val Asp Asn Leu Asn Leu Asn Phe
        690                 695                 700

tat gaa ggg cat att act tca ttg ctg ggg ccc aat gga gct ggg aaa 2282
Tyr Glu Gly His Ile Thr Ser Leu Leu Gly Pro Asn Gly Ala Gly Lys
    705                 710                 715

act act acc att tcc atg tta act ggg ctg ttt ggg gcc tca gca ggc 2330
Thr Thr Thr Ile Ser Met Leu Thr Gly Leu Phe Gly Ala Ser Ala Gly
720                 725                 730                 735

acc att ttt gta tat gga aaa gat atc aaa aca gac cta cac acg gta 2378
Thr Ile Phe Val Tyr Gly Lys Asp Ile Lys Thr Asp Leu His Thr Val
                740                 745                 750

cgg aag aac atg gga gtc tgt atg cag cac gac gtc ttg ttc agt tac 2426
Arg Lys Asn Met Gly Val Cys Met Gln His Asp Val Leu Phe Ser Tyr
            755                 760                 765

ctc act act aag gag cac ctt ctc cta tat ggt tcc atc aaa gtt cct 2474
Leu Thr Thr Lys Glu His Leu Leu Leu Tyr Gly Ser Ile Lys Val Pro
        770                 775                 780

cac tgg act aaa aag cag ctc cac gag gaa gta aaa agg act tta aaa 2522
His Trp Thr Lys Lys Gln Leu His Glu Glu Val Lys Arg Thr Leu Lys
    785                 790                 795

gat act gga cta tat agc cat cgt cat aag aga gtt gga aca ctg tca 2570
Asp Thr Gly Leu Tyr Ser His Arg His Lys Arg Val Gly Thr Leu Ser
800                 805                 810                 815

gga ggc atg aag agg aag tta tct ata tcc ata gct ctc att ggt gga 2618
Gly Gly Met Lys Arg Lys Leu Ser Ile Ser Ile Ala Leu Ile Gly Gly
                820                 825                 830

tca agg gta gta att ttg gat gaa cca tct act gga gtt gac cca tgt 2666
Ser Arg Val Val Ile Leu Asp Glu Pro Ser Thr Gly Val Asp Pro Cys
            835                 840                 845

tct cgc cga agt ata tgg gat gtt ata tcc aag aac aaa act gcc aga    2714
Ser Arg Arg Ser Ile Trp Asp Val Ile Ser Lys Asn Lys Thr Ala Arg
        850                 855                 860

aca atc att ctg tca acg cac cac ttg gac gag gct gaa gtg ctg agt    2762
Thr Ile Ile Leu Ser Thr His His Leu Asp Glu Ala Glu Val Leu Ser
    865                 870                 875

gac cgc atc gcc ttc ctg gag cag ggt ggg ctt agg tgc tgt ggg tcc    2810
Asp Arg Ile Ala Phe Leu Glu Gln Gly Gly Leu Arg Cys Cys Gly Ser
880                 885                 890                 895

cca ttt tac ctc aag gaa gcc ttt ggc gat ggg tat cac ctc acg ctt    2858
Pro Phe Tyr Leu Lys Glu Ala Phe Gly Asp Gly Tyr His Leu Thr Leu
                900                 905                 910

acc aag aag aag agt cca aat tta aat gca aat gca gta tgt gac acc    2906
Thr Lys Lys Lys Ser Pro Asn Leu Asn Ala Asn Ala Val Cys Asp Thr
            915                 920                 925

atg gcc gtg aca gca atg atc caa tca cat ctc ccc gaa gcc tac ctc    2954
Met Ala Val Thr Ala Met Ile Gln Ser His Leu Pro Glu Ala Tyr Leu
        930                 935                 940

aag gag gat att ggg gga gag ctt gtt tat gta ctt cct cca ttc agc    3002
Lys Glu Asp Ile Gly Gly Glu Leu Val Tyr Val Leu Pro Pro Phe Ser
    945                 950                 955

acc aaa gtc tca ggg gcc tac ctg tca ctc cta cgg gca ctc gac aat    3050
Thr Lys Val Ser Gly Ala Tyr Leu Ser Leu Leu Arg Ala Leu Asp Asn
960                 965                 970                 975

ggc atg ggt gac ctc aac atc ggg tgc tac ggc att tca gat acc acc    3098
Gly Met Gly Asp Leu Asn Ile Gly Cys Tyr Gly Ile Ser Asp Thr Thr
                980                 985                 990

gtg gag gag gtc ttt ctg aac ttg acc aaa gag tca caa aaa aat agt    3146
Val Glu Glu Val Phe Leu Asn Leu Thr Lys Glu Ser Gln Lys Asn Ser
            995                 1000                1005

gct atg agt ctt gag cac tta aca caa aag aaa att ggg aat tcc aat    3194
Ala Met Ser Leu Glu His Leu Thr Gln Lys Lys Ile Gly Asn Ser Asn
        1010                1015                1020

gcc aat ggc atc tca act cct gac gat tta tct gtg agc agc agc aat    3242
Ala Asn Gly Ile Ser Thr Pro Asp Asp Leu Ser Val Ser Ser Ser Asn
    1025                1030                1035

ttc aca gac aga gat gac aaa atc ctg aca aga gga gag agg ctg gat    3290
Phe Thr Asp Arg Asp Asp Lys Ile Leu Thr Arg Gly Glu Arg Leu Asp
1040                1045                1050                1055

ggc ttt gga ctg ttg ctg aag aag atc atg gct ata ctc atc aag agg    3338
Gly Phe Gly Leu Leu Leu Lys Lys Ile Met Ala Ile Leu Ile Lys Arg
                1060                1065                1070

ttc cac cac acc cgc agg aac tgg aaa ggt ctc att gct cag gtt atc    3386
Phe His His Thr Arg Arg Asn Trp Lys Gly Leu Ile Ala Gln Val Ile
            1075                1080                1085

ctc ccc atc gtc ttt gtt acc act gcc atg ggc ctt ggc aca ctg aga    3434
Leu Pro Ile Val Phe Val Thr Thr Ala Met Gly Leu Gly Thr Leu Arg
        1090                1095                1100

aat tcc agc aac agt tat cca gag att cag atc tcc ccc tct ctt tat    3482
Asn Ser Ser Asn Ser Tyr Pro Glu Ile Gln Ile Ser Pro Ser Leu Tyr
    1105                1110                1115

ggt acc tcc gaa cag aca gcc ttc tat gct aat tat cac ccg agc acg    3530
Gly Thr Ser Glu Gln Thr Ala Phe Tyr Ala Asn Tyr His Pro Ser Thr
1120                1125                1130                1135

gaa gca ctt gtc tca gca atg tgg gac ttc cct gga att gac aac atg    3578
Glu Ala Leu Val Ser Ala Met Trp Asp Phe Pro Gly Ile Asp Asn Met
                1140                1145                1150

tgt ctg aac acc agt gat cta cag tgt tta aac aaa gac agt ctg gaa    3626
Cys Leu Asn Thr Ser Asp Leu Gln Cys Leu Asn Lys Asp Ser Leu Glu
            1155                1160                1165

aaa tgg aac acc agt gga gaa ccc atc act aat ttt ggt gtt tgc tcc    3674
Lys Trp Asn Thr Ser Gly Glu Pro Ile Thr Asn Phe Gly Val Cys Ser
        1170                1175                1180

tgc tca gaa aat gtc cag gaa tgt cct aaa ttt aac tat tcc cca ccg    3722
Cys Ser Glu Asn Val Gln Glu Cys Pro Lys Phe Asn Tyr Ser Pro Pro
    1185                1190                1195

cac aga aga act tac tca tcc cag gta att tat aac ctc act ggg caa    3770
His Arg Arg Thr Tyr Ser Ser Gln Val Ile Tyr Asn Leu Thr Gly Gln
1200                1205                1210                1215

cga gtg gaa aat tat ctt ata tca act gca aat gag ttt gtc caa aaa    3818
Arg Val Glu Asn Tyr Leu Ile Ser Thr Ala Asn Glu Phe Val Gln Lys
                1220                1225                1230

aga tat gga ggt tgg agt ttt ggg ctg cct ttg aca aaa gac ctt cgt    3866
Arg Tyr Gly Gly Trp Ser Phe Gly Leu Pro Leu Thr Lys Asp Leu Arg
            1235                1240                1245

ttt gat ata aca gga gtc cct gcc aat aga aca ctt gcc aag gta tgg    3914
Phe Asp Ile Thr Gly Val Pro Ala Asn Arg Thr Leu Ala Lys Val Trp
        1250                1255                1260

tat gat cca gaa ggc tat cac tcc ctt cca gct tac ctc aac agc ctg    3962
Tyr Asp Pro Glu Gly Tyr His Ser Leu Pro Ala Tyr Leu Asn Ser Leu
    1265                1270                1275

aat aat ttc ctt ctg cga gtt aac atg tca aaa tac gat gct gcc cga    4010
Asn Asn Phe Leu Leu Arg Val Asn Met Ser Lys Tyr Asp Ala Ala Arg
1280                1285                1290                1295

cat ggc atc atc atg tat agc cat cct tat cca gga gtg caa gac caa    4058
His Gly Ile Ile Met Tyr Ser His Pro Tyr Pro Gly Val Gln Asp Gln
                1300                1305                1310

gaa caa gcc aca atc agc agt tta atc gat att tta gtg gca ctg tct    4106
Glu Gln Ala Thr Ile Ser Ser Leu Ile Asp Ile Leu Val Ala Leu Ser
            1315                1320                1325

atc ttg atg ggc tac tct gtc acc acc gcc agc ttt gtc acc tat gtt    4154

Ile Leu Met Gly Tyr Ser Val Thr Thr Ala Ser Phe Val Thr Tyr Val
      1330                1335                1340

gta agg gaa cat caa acc aaa gcc aaa cag ttg cag cac att tca ggc    4202
Val Arg Glu His Gln Thr Lys Ala Lys Gln Leu Gln His Ile Ser Gly
    1345                1350                1355

att ggc gtg aca tgc tac tgg gta aca aac ttc att tat gac atg gtt    4250
Ile Gly Val Thr Cys Tyr Trp Val Thr Asn Phe Ile Tyr Asp Met Val
1360                1365                1370                1375

ttc tac ttg gtg cct gta gcg ttt tca att ggt atc att gcg att ttc    4298
Phe Tyr Leu Val Pro Val Ala Phe Ser Ile Gly Ile Ile Ala Ile Phe
                1380                1385                1390

aaa tta cct gca ttc tac agt gaa aac aac cta ggc gct gta tct ctc    4346
Lys Leu Pro Ala Phe Tyr Ser Glu Asn Asn Leu Gly Ala Val Ser Leu
            1395                1400                1405

cta ctt ctc ctg ttt ggg tat gca aca ttt tcc tgg atg tac ttg ctg    4394
Leu Leu Leu Leu Phe Gly Tyr Ala Thr Phe Ser Trp Met Tyr Leu Leu
        1410                1415                1420

gct ggg ctc ttc cat gaa aca gga atg gcc ttc atc act tac gtc tgt    4442
Ala Gly Leu Phe His Glu Thr Gly Met Ala Phe Ile Thr Tyr Val Cys
    1425                1430                1435

gtc aac ttg ttt ttt ggc att aat tcc att gtt tcc ctg tca gtg gta    4490
Val Asn Leu Phe Phe Gly Ile Asn Ser Ile Val Ser Leu Ser Val Val
1440                1445                1450                1455

tac ttt ctt tcc aag gaa aag cct aat gat ccg act tta gaa ctt att    4538
Tyr Phe Leu Ser Lys Glu Lys Pro Asn Asp Pro Thr Leu Glu Leu Ile
                1460                1465                1470

tct gaa acc ctc aag cgc att ttc ctg att ttc cca caa ttc tgt ttt    4586
Ser Glu Thr Leu Lys Arg Ile Phe Leu Ile Phe Pro Gln Phe Cys Phe
            1475                1480                1485

ggc tac ggt ttg att gaa ctt tct caa caa cag tcg gtc cta gac ttc    4634
Gly Tyr Gly Leu Ile Glu Leu Ser Gln Gln Gln Ser Val Leu Asp Phe
        1490                1495                1500

tta aaa gca tat gga gtg gaa tac cca aat gaa acc ttt gag atg aat    4682
Leu Lys Ala Tyr Gly Val Glu Tyr Pro Asn Glu Thr Phe Glu Met Asn
    1505                1510                1515

aaa cta ggt gca atg ttt gtg gct ttg gtt tct cag ggc acc atg ttt    4730
Lys Leu Gly Ala Met Phe Val Ala Leu Val Ser Gln Gly Thr Met Phe
1520                1525                1530                1535

ttt tcc ttg cga ctc tta atc aac gaa tcc ctg ata aag aaa ctc agg    4778
Phe Ser Leu Arg Leu Leu Ile Asn Glu Ser Leu Ile Lys Lys Leu Arg
                1540                1545                1550

ctt ttc ttc aga aaa ttt aat tct tca cat gta agg gag aca ata gat    4826
Leu Phe Phe Arg Lys Phe Asn Ser Ser His Val Arg Glu Thr Ile Asp
            1555                1560                1565

gag gat gaa gat gtg cgg gct gag aga tta aga gtt gag agt ggt gca    4874
Glu Asp Glu Asp Val Arg Ala Glu Arg Leu Arg Val Glu Ser Gly Ala

1570                1575                1580

gct gaa ttt gac ttg gtc caa ctt tat tgt ctc aca aag acc tac caa    4922
Ala Glu Phe Asp Leu Val Gln Leu Tyr Cys Leu Thr Lys Thr Tyr Gln
    1585                1590                1595

ctt atc cac aaa aag att ata gct gta aac aac atc agc att ggg ata    4970
Leu Ile His Lys Lys Ile Ile Ala Val Asn Asn Ile Ser Ile Gly Ile
1600                1605                1610                1615

cct gct gga gag tgt ttt ggg ctt ctt gga gtg aat gga gca gga aag    5018
Pro Ala Gly Glu Cys Phe Gly Leu Leu Gly Val Asn Gly Ala Gly Lys
                1620                1625                1630

acc act ata ttc aag atg ctg aca gga gac atc att cct tca agt gga    5066
Thr Thr Ile Phe Lys Met Leu Thr Gly Asp Ile Ile Pro Ser Ser Gly
            1635                1640                1645

aac att ctg atc aga aat aag acc gga tct ctg ggt cac gtt gat tct    5114
Asn Ile Leu Ile Arg Asn Lys Thr Gly Ser Leu Gly His Val Asp Ser
        1650                1655                1660

cac agc tca tta gtt ggc tac tgt cct cag gaa gat gcc tta gat gac    5162
His Ser Ser Leu Val Gly Tyr Cys Pro Gln Glu Asp Ala Leu Asp Asp
    1665                1670                1675

ctg gta act gtg gaa gaa cat ttg tat ttc tat gcc agg gta cat gga    5210
Leu Val Thr Val Glu Glu His Leu Tyr Phe Tyr Ala Arg Val His Gly
1680                1685                1690                1695

att cca gaa aag gat att aaa gaa act gtt cat aaa ctc ctt agg aga    5258
Ile Pro Glu Lys Asp Ile Lys Glu Thr Val His Lys Leu Leu Arg Arg
                1700                1705                1710

ctt cac ctg atg ccc ttc aag gac aga gct acc tct atg tgc agt tat    5306
Leu His Leu Met Pro Phe Lys Asp Arg Ala Thr Ser Met Cys Ser Tyr
            1715                1720                1725

ggc aca aaa aga aaa tta tcc act gca ctg gcc ttg ata ggg aaa cct    5354
Gly Thr Lys Arg Lys Leu Ser Thr Ala Leu Ala Leu Ile Gly Lys Pro
        1730                1735                1740

tcc att cta ctg ctg gat gag ccg agc tct ggc atg gat ccg aag tcg    5402
Ser Ile Leu Leu Leu Asp Glu Pro Ser Ser Gly Met Asp Pro Lys Ser
    1745                1750                1755

aaa cgg cac ctc tgg aag atc att tca gaa gaa gta cag aac aaa tgt    5450
Lys Arg His Leu Trp Lys Ile Ile Ser Glu Glu Val Gln Asn Lys Cys
1760                1765                1770                1775

tcc gtc atc ctc aca tct cac agc atg gaa gaa tgt gaa gct ctc tgt    5498
Ser Val Ile Leu Thr Ser His Ser Met Glu Glu Cys Glu Ala Leu Cys
                1780                1785                1790

acc agg ttg gcc att atg gtg aat gga aag ttt caa tgt att gga tct    5546
Thr Arg Leu Ala Ile Met Val Asn Gly Lys Phe Gln Cys Ile Gly Ser
            1795                1800                1805

ttg cag cac ata aag agc agg ttt gga cga gga ttt act gtc aaa gtt    5594
Leu Gln His Ile Lys Ser Arg Phe Gly Arg Gly Phe Thr Val Lys Val
        1810                1815                1820

cac ttg aag aat aac aaa gtg acc atg gag acc ctc aca aag ttc atg 5642
His Leu Lys Asn Asn Lys Val Thr Met Glu Thr Leu Thr Lys Phe Met
    1825                1830                1835

cag ctg cac ttt cca aaa aca tac tta aaa gat cag cac ctc agc atg 5690
Gln Leu His Phe Pro Lys Thr Tyr Leu Lys Asp Gln His Leu Ser Met
1840                1845                1850                1855

cta gag tat cat gta cca gtc aca gca gga gga gtc gca aac att ttt 5738
Leu Glu Tyr His Val Pro Val Thr Ala Gly Gly Val Ala Asn Ile Phe
                1860                1865                1870

gat ctg ctg gaa acc aac aag act gct tta aat att aca aat ttc tta 5786
Asp Leu Leu Glu Thr Asn Lys Thr Ala Leu Asn Ile Thr Asn Phe Leu
            1875                1880                1885

gtg agt cag acc act ctg gaa gag gtt ttc atc aac ttt gcc aaa gac 5834
Val Ser Gln Thr Thr Leu Glu Glu Val Phe Ile Asn Phe Ala Lys Asp
        1890                1895                1900

cag aag tcc tat gaa act gct gat acc agc agc caa ggt tcc act ata 5882
Gln Lys Ser Tyr Glu Thr Ala Asp Thr Ser Ser Gln Gly Ser Thr Ile
    1905                1910                1915

agt gtt gac tca caa gat gac cag atg gag tct taacacttcc agcaaactca 5935
Ser Val Asp Ser Gln Asp Asp Gln Met Glu Ser
1920                1925                1930

atctcagcgt gtgaccaatg gcttcatttt gaagaaaagc cacagaagat acacttccgc 5995
aagatatctt cattttaaag taaagtaata tactgtatgg aaagttacaa ctgtgttaga 6055
ctaacaagta attataaaag gaaatttttc cttctaaggt cagtgagtgt tgttgctact 6115
gaaatgaatt cctgtatact caacactgtg agcatgctaa tgtatatgct ggtgattctt 6175
atgcaaaggt gaagccacct caagatgaat atcttaattt attactttca ataaaaagac 6235
agtttaaaag gcatggattt tggtagttga aatataagag tggagaagaa aagtcagatg 6295
gtttgtggca ggtgccaccg ggcaagcaga caacataatt tatttccaga aaacaacaga 6355
atgaacatca tcatgaatac atgaatcggc tgtgatgtgt gaactgctaa gggccaaatg 6415
aacgtttgca gagcagtggg cacaatgttt acaatgtatg tgtatgtcac tttcggtacc 6475
tgtgaatgca tggggacgtg ctgaacccga aaaaaagtgc ctttccataa ggactgcaat 6535
agagagggca atttaccctg gtggtacacg gaacctagat tcactcctgc catgccttgc 6595
caatagtaag ctgcagggtg gaacaagaaa tcacttgctc tggggggaag ggagggggga 6655
atgggtgtgt cagctgggta gatacaaacc ctgaaaagag aatccatgtg ctactggcag 6715
gcaacatttt ttaaagctct ttcagaaacc ct 6747

<210> 2
<211> 1930
<212> PRT
<213> homo sapiens

<400> 2
Met Met Glu Leu Phe Ile Arg Leu Lys Glu Ile Leu Asn Gln Met Ala
1               5                   10                  15
Ser Gly Thr His Pro Leu Leu Asp Lys Met Arg Ser Leu Lys Gln Met
            20                  25                  30
His Leu Pro Arg Ser Val Pro Leu Thr Gln Ala Met Tyr Arg Ser Asn
        35                  40                  45
Arg Met Asn Thr Ser Gln Gly Ser Phe Ser Thr Ile Ser Gln Ala Leu
    50                  55                  60
Cys Ser Gln Gly Ile Thr Thr Glu Tyr Leu Thr Ala Met Leu Pro Ser
65                  70                  75                  80
Ser Gln Arg Pro Lys Gly Asn His Thr Lys Asp Phe Leu Thr Tyr Lys

                    85                  90                  95
Leu Thr Lys Glu Gln Ile Ala Ser Lys Tyr Gly Ile Pro Ile Asn Thr
            100                 105                 110
Thr Pro Phe Cys Phe Ser Leu Tyr Lys Asp Ile Ile Asn Met Pro Ala
        115                 120                 125
Gly Pro Val Ile Trp Ala Phe Leu Lys Pro Met Leu Leu Gly Arg Ile
    130                 135                 140
Leu Tyr Ala Pro Tyr Asn Pro Val Thr Lys Ala Ile Met Glu Lys Ser
145                 150                 155                 160
Asn Val Thr Leu Arg Gln Leu Ala Glu Leu Arg Glu Lys Ser Gln Glu
                165                 170                 175
Trp Met Asp Lys Ser Pro Leu Phe Met Asn Ser Phe His Leu Leu Asn
            180                 185                 190
Gln Ala Ile Pro Met Leu Gln Asn Thr Leu Arg Asn Pro Phe Val Gln
        195                 200                 205
Val Phe Val Lys Phe Ser Val Gly Leu Asp Ala Val Glu Leu Leu Lys
    210                 215                 220
Gln Ile Asp Glu Leu Asp Ile Leu Arg Leu Lys Leu Glu Asn Asn Ile
225                 230                 235                 240
Asp Ile Ile Asp Gln Leu Asn Thr Leu Ser Ser Leu Thr Val Asn Ile
                245                 250                 255
Ser Ser Cys Val Leu Tyr Asp Arg Ile Gln Ala Ala Lys Thr Ile Asp
            260                 265                 270
Glu Met Glu Arg Glu Ala Lys Arg Leu Tyr Lys Ser Asn Glu Leu Phe
        275                 280                 285
Gly Ser Val Ile Phe Lys Leu Pro Ser Asn Arg Ser Trp His Arg Gly
    290                 295                 300
Tyr Asp Ser Gly Asn Val Phe Leu Pro Pro Val Ile Lys Tyr Thr Ile
305                 310                 315                 320
Arg Met Ser Leu Lys Thr Ala Gln Thr Thr Arg Ser Leu Arg Thr Lys
                325                 330                 335
Ile Trp Ala Pro Gly Pro His Asn Ser Pro Ser His Asn Gln Ile Tyr
            340                 345                 350
Gly Arg Ala Phe Ile Tyr Leu Gln Asp Ser Ile Glu Arg Ala Ile Ile
        355                 360                 365
Glu Leu Gln Thr Gly Arg Asn Ser Gln Glu Ile Ala Val Gln Val Gln
    370                 375                 380
Ala Ile Pro Tyr Pro Cys Phe Met Lys Asp Asn Phe Leu Thr Ser Val
385                 390                 395                 400
Ser Tyr Ser Leu Pro Ile Val Leu Met Val Ala Trp Val Val Phe Ile
                405                 410                 415
Ala Ala Phe Val Lys Lys Leu Val Tyr Glu Lys Asp Leu Arg Leu His
            420                 425                 430
Glu Tyr Met Lys Met Met Gly Val Asn Ser Cys Ser His Phe Phe Ala
        435                 440                 445
Trp Leu Ile Glu Ser Val Gly Phe Leu Leu Val Thr Ile Val Ile Leu
    450                 455                 460
Ile Ile Ile Leu Lys Phe Gly Asn Ile Leu Pro Lys Thr Asn Gly Phe
465                 470                 475                 480
Ile Leu Phe Leu Tyr Phe Ser Asp Tyr Ser Phe Ser Val Ile Ala Met
                485                 490                 495
Ser Tyr Leu Ile Ser Val Phe Phe Asn Asn Thr Asn Ile Ala Ala Leu
            500                 505                 510
Ile Gly Ser Leu Ile Tyr Ile Ile Ala Phe Phe Pro Phe Ile Val Leu
        515                 520                 525
Val Thr Val Glu Asn Glu Leu Ser Tyr Val Leu Lys Val Phe Met Ser
    530                 535                 540
Leu Leu Ser Pro Thr Ala Phe Ser Tyr Ala Asn Gln Tyr Ile Ala Arg
545                 550                 555                 560
Tyr Glu Glu Gln Gly Ile Gly Leu Gln Trp Glu Asn Met Tyr Thr Ser
                565                 570                 575

Pro Val Gln Asp Asp Thr Thr Ser Phe Gly Trp Leu Cys Cys Leu Ile
            580                 585                 590
Leu Ala Asp Ser Phe Ile Tyr Phe Leu Ile Ala Trp Tyr Val Arg Asn
        595                 600                 605
Val Phe Pro Gly Thr Tyr Gly Met Ala Ala Pro Trp Tyr Phe Pro Ile
    610                 615                 620
Leu Pro Ser Tyr Trp Lys Glu Arg Phe Gly Cys Ala Glu Val Lys Pro
625                 630                 635                 640
Glu Lys Ser Asn Gly Leu Met Phe Thr Asn Ile Met Met Gln Asn Thr
                645                 650                 655
Asn Pro Ser Ala Ser Pro Glu Tyr Met Phe Ser Ser Asn Ile Glu Pro
            660                 665                 670
Glu Pro Lys Asp Leu Thr Val Gly Val Ala Leu His Gly Val Thr Lys
        675                 680                 685
Ile Tyr Gly Ser Lys Val Ala Val Asp Asn Leu Asn Leu Asn Phe Tyr
    690                 695                 700
Glu Gly His Ile Thr Ser Leu Leu Gly Pro Asn Gly Ala Gly Lys Thr
705                 710                 715                 720
Thr Thr Ile Ser Met Leu Thr Gly Leu Phe Gly Ala Ser Ala Gly Thr
                725                 730                 735
Ile Phe Val Tyr Gly Lys Asp Ile Lys Thr Asp Leu His Thr Val Arg
            740                 745                 750
Lys Asn Met Gly Val Cys Met Gln His Asp Val Leu Phe Ser Tyr Leu
        755                 760                 765
Thr Thr Lys Glu His Leu Leu Leu Tyr Gly Ser Ile Lys Val Pro His
    770                 775                 780
Trp Thr Lys Lys Gln Leu His Glu Glu Val Lys Arg Thr Leu Lys Asp
785                 790                 795                 800
Thr Gly Leu Tyr Ser His Arg His Lys Arg Val Gly Thr Leu Ser Gly
                805                 810                 815
Gly Met Lys Arg Lys Leu Ser Ile Ser Ile Ala Leu Ile Gly Gly Ser
            820                 825                 830
Arg Val Val Ile Leu Asp Glu Pro Ser Thr Gly Val Asp Pro Cys Ser
        835                 840                 845
Arg Arg Ser Ile Trp Asp Val Ile Ser Lys Asn Lys Thr Ala Arg Thr
    850                 855                 860
Ile Ile Leu Ser Thr His His Leu Asp Glu Ala Glu Val Leu Ser Asp
865                 870                 875                 880
Arg Ile Ala Phe Leu Glu Gln Gly Gly Leu Arg Cys Cys Gly Ser Pro
                885                 890                 895
Phe Tyr Leu Lys Glu Ala Phe Gly Asp Gly Tyr His Leu Thr Leu Thr
            900                 905                 910
Lys Lys Lys Ser Pro Asn Leu Asn Ala Asn Ala Val Cys Asp Thr Met
        915                 920                 925
Ala Val Thr Ala Met Ile Gln Ser His Leu Pro Glu Ala Tyr Leu Lys
    930                 935                 940
Glu Asp Ile Gly Gly Glu Leu Val Tyr Val Leu Pro Pro Phe Ser Thr
945                 950                 955                 960
Lys Val Ser Gly Ala Tyr Leu Ser Leu Leu Arg Ala Leu Asp Asn Gly
                965                 970                 975
Met Gly Asp Leu Asn Ile Gly Cys Tyr Gly Ile Ser Asp Thr Thr Val
            980                 985                 990
Glu Glu Val Phe Leu Asn Leu Thr Lys Glu Ser Gln Lys Asn Ser Ala
        995                 1000                1005
Met Ser Leu Glu His Leu Thr Gln Lys Lys Ile Gly Asn Ser Asn Ala
    1010                1015                1020
Asn Gly Ile Ser Thr Pro Asp Asp Leu Ser Val Ser Ser Ser Asn Phe
1025                1030                1035                1040
Thr Asp Arg Asp Asp Lys Ile Leu Thr Arg Gly Glu Arg Leu Asp Gly
                1045                1050                1055
Phe Gly Leu Leu Leu Lys Lys Ile Met Ala Ile Leu Ile Lys Arg Phe

                1060                1065                1070
His His Thr Arg Arg Asn Trp Lys Gly Leu Ile Ala Gln Val Ile Leu
        1075                1080                1085
Pro Ile Val Phe Val Thr Thr Ala Met Gly Leu Gly Thr Leu Arg Asn
    1090                1095                1100
Ser Ser Asn Ser Tyr Pro Glu Ile Gln Ile Ser Pro Ser Leu Tyr Gly
1105                1110                1115                1120
Thr Ser Glu Gln Thr Ala Phe Tyr Ala Asn Tyr His Pro Ser Thr Glu
                1125                1130                1135
Ala Leu Val Ser Ala Met Trp Asp Phe Pro Gly Ile Asp Asn Met Cys
            1140                1145                1150
Leu Asn Thr Ser Asp Leu Gln Cys Leu Asn Lys Asp Ser Leu Glu Lys
        1155                1160                1165
Trp Asn Thr Ser Gly Glu Pro Ile Thr Asn Phe Gly Val Cys Ser Cys
    1170                1175                1180
Ser Glu Asn Val Gln Glu Cys Pro Lys Phe Asn Tyr Ser Pro Pro His
1185                1190                1195                1200
Arg Arg Thr Tyr Ser Ser Gln Val Ile Tyr Asn Leu Thr Gly Gln Arg
                1205                1210                1215
Val Glu Asn Tyr Leu Ile Ser Thr Ala Asn Glu Phe Val Gln Lys Arg
            1220                1225                1230
Tyr Gly Gly Trp Ser Phe Gly Leu Pro Leu Thr Lys Asp Leu Arg Phe
        1235                1240                1245
Asp Ile Thr Gly Val Pro Ala Asn Arg Thr Leu Ala Lys Val Trp Tyr
    1250                1255                1260
Asp Pro Glu Gly Tyr His Ser Leu Pro Ala Tyr Leu Asn Ser Leu Asn
1265                1270                1275                1280
Asn Phe Leu Leu Arg Val Asn Met Ser Lys Tyr Asp Ala Ala Arg His
                1285                1290                1295
Gly Ile Ile Met Tyr Ser His Pro Tyr Pro Gly Val Gln Asp Gln Glu
            1300                1305                1310
Gln Ala Thr Ile Ser Ser Leu Ile Asp Ile Leu Val Ala Leu Ser Ile
        1315                1320                1325
Leu Met Gly Tyr Ser Val Thr Thr Ala Ser Phe Val Thr Tyr Val Val
    1330                1335                1340
Arg Glu His Gln Thr Lys Ala Lys Gln Leu Gln His Ile Ser Gly Ile
1345                1350                1355                1360
Gly Val Thr Cys Tyr Trp Val Thr Asn Phe Ile Tyr Asp Met Val Phe
                1365                1370                1375
Tyr Leu Val Pro Val Ala Phe Ser Ile Gly Ile Ile Ala Ile Phe Lys
            1380                1385                1390
Leu Pro Ala Phe Tyr Ser Glu Asn Asn Leu Gly Ala Val Ser Leu Leu
        1395                1400                1405
Leu Leu Leu Phe Gly Tyr Ala Thr Phe Ser Trp Met Tyr Leu Leu Ala
    1410                1415                1420
Gly Leu Phe His Glu Thr Gly Met Ala Phe Ile Thr Tyr Val Cys Val
1425                1430                1435                1440
Asn Leu Phe Phe Gly Ile Asn Ser Ile Val Ser Leu Ser Val Val Tyr
                1445                1450                1455
Phe Leu Ser Lys Glu Lys Pro Asn Asp Pro Thr Leu Glu Leu Ile Ser
            1460                1465                1470
Glu Thr Leu Lys Arg Ile Phe Leu Ile Phe Pro Gln Phe Cys Phe Gly
        1475                1480                1485
Tyr Gly Leu Ile Glu Leu Ser Gln Gln Gln Ser Val Leu Asp Phe Leu
    1490                1495                1500
Lys Ala Tyr Gly Val Glu Tyr Pro Asn Glu Thr Phe Glu Met Asn Lys
1505                1510                1515                1520
Leu Gly Ala Met Phe Val Ala Leu Val Ser Gln Gly Thr Met Phe Phe
                1525                1530                1535
Ser Leu Arg Leu Leu Ile Asn Glu Ser Leu Ile Lys Lys Leu Arg Leu
            1540                1545                1550

Phe Phe Arg Lys Phe Asn Ser Ser His Val Arg Glu Thr Ile Asp Glu
        1555                1560                1565
Asp Glu Asp Val Arg Ala Glu Arg Leu Arg Val Glu Ser Gly Ala Ala
    1570                1575                1580
Glu Phe Asp Leu Val Gln Leu Tyr Cys Leu Thr Lys Thr Tyr Gln Leu
1585                1590                1595                1600
Ile His Lys Lys Ile Ile Ala Val Asn Asn Ile Ser Ile Gly Ile Pro
                1605                1610                1615
Ala Gly Glu Cys Phe Gly Leu Leu Gly Val Asn Gly Ala Gly Lys Thr
            1620                1625                1630
Thr Ile Phe Lys Met Leu Thr Gly Asp Ile Ile Pro Ser Ser Gly Asn
        1635                1640                1645
Ile Leu Ile Arg Asn Lys Thr Gly Ser Leu Gly His Val Asp Ser His
    1650                1655                1660
Ser Ser Leu Val Gly Tyr Cys Pro Gln Glu Asp Ala Leu Asp Asp Leu
1665                1670                1675                1680
Val Thr Val Glu Glu His Leu Tyr Phe Tyr Ala Arg Val His Gly Ile
                1685                1690                1695
Pro Glu Lys Asp Ile Lys Glu Thr Val His Lys Leu Leu Arg Arg Leu
            1700                1705                1710
His Leu Met Pro Phe Lys Asp Arg Ala Thr Ser Met Cys Ser Tyr Gly
        1715                1720                1725
Thr Lys Arg Lys Leu Ser Thr Ala Leu Ala Leu Ile Gly Lys Pro Ser
    1730                1735                1740
Ile Leu Leu Leu Asp Glu Pro Ser Ser Gly Met Asp Pro Lys Ser Lys
1745                1750                1755                1760
Arg His Leu Trp Lys Ile Ile Ser Glu Glu Val Gln Asn Lys Cys Ser
                1765                1770                1775
Val Ile Leu Thr Ser His Ser Met Glu Glu Cys Glu Ala Leu Cys Thr
            1780                1785                1790
Arg Leu Ala Ile Met Val Asn Gly Lys Phe Gln Cys Ile Gly Ser Leu
        1795                1800                1805
Gln His Ile Lys Ser Arg Phe Gly Arg Gly Phe Thr Val Lys Val His
    1810                1815                1820
Leu Lys Asn Asn Lys Val Thr Met Glu Thr Leu Thr Lys Phe Met Gln
1825                1830                1835                1840
Leu His Phe Pro Lys Thr Tyr Leu Lys Asp Gln His Leu Ser Met Leu
                1845                1850                1855
Glu Tyr His Val Pro Val Thr Ala Gly Gly Val Ala Asn Ile Phe Asp
            1860                1865                1870
Leu Leu Glu Thr Asn Lys Thr Ala Leu Asn Ile Thr Asn Phe Leu Val
        1875                1880                1885
Ser Gln Thr Thr Leu Glu Glu Val Phe Ile Asn Phe Ala Lys Asp Gln
    1890                1895                1900
Lys Ser Tyr Glu Thr Ala Asp Thr Ser Ser Gln Gly Ser Thr Ile Ser
1905                1910                1915                1920
Val Asp Ser Gln Asp Asp Gln Met Glu Ser
                1925                1930

<210> 3
<211> 5790
<212> DNA
<213> Homo sapiens

<220>
<221> CDS
<222> (1)...(5790)

<400> 3
atg atg gag ctc ttc ata aga cta aaa gag att ctc aat cag atg gct    48

Met Met Glu Leu Phe Ile Arg Leu Lys Glu Ile Leu Asn Gln Met Ala
1               5                   10                  15

tct ggc aca cat ccg ctg cta gac aaa atg aga tcc ctg aag caa atg     96
Ser Gly Thr His Pro Leu Leu Asp Lys Met Arg Ser Leu Lys Gln Met
            20                  25                  30

cat ctg ccc aga agt gtt cca tta aca cag gca atg tac aga agc aac     144
His Leu Pro Arg Ser Val Pro Leu Thr Gln Ala Met Tyr Arg Ser Asn
        35                  40                  45

cga atg aac aca tca caa gga tca ttt agc acc atc tcc caa gca tta     192
Arg Met Asn Thr Ser Gln Gly Ser Phe Ser Thr Ile Ser Gln Ala Leu
    50                  55                  60

tgt tct caa gga att acc act gaa tat tta act gcc atg ctg ccc tct     240
Cys Ser Gln Gly Ile Thr Thr Glu Tyr Leu Thr Ala Met Leu Pro Ser
65                  70                  75                  80

tcc cag agg cca aaa ggc aac cac acc aag gat ttt ttg act tat aaa     288
Ser Gln Arg Pro Lys Gly Asn His Thr Lys Asp Phe Leu Thr Tyr Lys
                85                  90                  95

tta act aaa gag caa att gct tca aaa tat gga att ccc ata aat acc     336
Leu Thr Lys Glu Gln Ile Ala Ser Lys Tyr Gly Ile Pro Ile Asn Thr
            100                 105                 110

aca cca ttt tgc ttc tcc ctt tat aaa gac atc att aac atg ccc gct     384
Thr Pro Phe Cys Phe Ser Leu Tyr Lys Asp Ile Ile Asn Met Pro Ala
        115                 120                 125

gga cct gtg att tgg gct ttc ttg aaa cct atg ttg ttg gga aga att     432
Gly Pro Val Ile Trp Ala Phe Leu Lys Pro Met Leu Leu Gly Arg Ile
    130                 135                 140

ttg tat gca cca tat aac cca gtc aca aag gca ata atg gaa aag tcc     480
Leu Tyr Ala Pro Tyr Asn Pro Val Thr Lys Ala Ile Met Glu Lys Ser
145                 150                 155                 160

aat gta act ctg aga cag ctg gcg gaa tta aga gaa aaa tct caa gag     528
Asn Val Thr Leu Arg Gln Leu Ala Glu Leu Arg Glu Lys Ser Gln Glu
                165                 170                 175

tgg atg gat aag tcg cca ctt ttc atg aat tcc ttc cat ctg tta aac     576
Trp Met Asp Lys Ser Pro Leu Phe Met Asn Ser Phe His Leu Leu Asn
            180                 185                 190

cag gca att cca atg ctc cag aat act cta agg aac cct ttt gtg caa     624
Gln Ala Ile Pro Met Leu Gln Asn Thr Leu Arg Asn Pro Phe Val Gln
        195                 200                 205

gtt ttt gta aag ttc tcc gtg gga ctc gat gct gtt gaa cta ttg aaa     672
Val Phe Val Lys Phe Ser Val Gly Leu Asp Ala Val Glu Leu Leu Lys
    210                 215                 220

cag ata gat gaa ctc gat att cta aga ctg aaa tta gag aac aac att     720
Gln Ile Asp Glu Leu Asp Ile Leu Arg Leu Lys Leu Glu Asn Asn Ile
225                 230                 235                 240

gac atc atc gat cag ctt aac aca cta tct tcc ctg aca gta aat att     768
Asp Ile Ile Asp Gln Leu Asn Thr Leu Ser Ser Leu Thr Val Asn Ile

                245                 250                 255

tcc tct tgt gta tta tat gac cgt att cag gca gca aaa acc ata gat 816
Ser Ser Cys Val Leu Tyr Asp Arg Ile Gln Ala Ala Lys Thr Ile Asp
            260                 265                 270

gaa atg gag aga gag gct aaa agg ctc tac aaa agc aac gaa ctc ttt 864
Glu Met Glu Arg Glu Ala Lys Arg Leu Tyr Lys Ser Asn Glu Leu Phe
        275                 280                 285

gga agt gtt att ttt aag ctt cct tct aac aga agc tgg cac aga ggc 912
Gly Ser Val Ile Phe Lys Leu Pro Ser Asn Arg Ser Trp His Arg Gly
    290                 295                 300

tat gac tct gga aat gtc ttt ctt cct cct gtc ata aaa tat acc atc 960
Tyr Asp Ser Gly Asn Val Phe Leu Pro Pro Val Ile Lys Tyr Thr Ile
305                 310                 315                 320

cgg atg agt ctc aag acc gca cag acc aca aga agc cta aga acc aag 1008
Arg Met Ser Leu Lys Thr Ala Gln Thr Thr Arg Ser Leu Arg Thr Lys
                325                 330                 335

att tgg gct cca ggg cca cac aat tct ccg tca cac aac cag atc tat 1056
Ile Trp Ala Pro Gly Pro His Asn Ser Pro Ser His Asn Gln Ile Tyr
            340                 345                 350

ggc agg gct ttt att tat tta cag gat agt att gaa aga gca atc att 1104
Gly Arg Ala Phe Ile Tyr Leu Gln Asp Ser Ile Glu Arg Ala Ile Ile
        355                 360                 365

gaa ttg caa act gga agg aac tcc cag gaa ata gca gtc cag gtt caa 1152
Glu Leu Gln Thr Gly Arg Asn Ser Gln Glu Ile Ala Val Gln Val Gln
    370                 375                 380

gca att cct tat ccc tgc ttc atg aaa gac aac ttc cta acc agt gtc 1200
Ala Ile Pro Tyr Pro Cys Phe Met Lys Asp Asn Phe Leu Thr Ser Val
385                 390                 395                 400

tct tat tct ctt cca att gtg ctt atg gtt gcc tgg gtt gta ttt ata 1248
Ser Tyr Ser Leu Pro Ile Val Leu Met Val Ala Trp Val Val Phe Ile
                405                 410                 415

gct gcc ttt gta aaa aag ctt gtc tat gag aaa gac ctc cgg ctt cat 1296
Ala Ala Phe Val Lys Lys Leu Val Tyr Glu Lys Asp Leu Arg Leu His
            420                 425                 430

gag tac atg aag atg atg ggt gtg aac tcc tgc agc cat ttc ttt gcc 1344
Glu Tyr Met Lys Met Met Gly Val Asn Ser Cys Ser His Phe Phe Ala
        435                 440                 445

tgg ctt ata gag agt gtt gga ttt tta ctg gtt acc atc gtg atc ctc 1392
Trp Leu Ile Glu Ser Val Gly Phe Leu Leu Val Thr Ile Val Ile Leu
    450                 455                 460

atc att ata ctc aag ttt ggc aat att ctt cct aaa aca aat ggg ttc 1440
Ile Ile Ile Leu Lys Phe Gly Asn Ile Leu Pro Lys Thr Asn Gly Phe
465                 470                 475                 480

att ttg ttc ctg tat ttt tcg gac tac agc ttc tcg gtt att gcc atg 1488
Ile Leu Phe Leu Tyr Phe Ser Asp Tyr Ser Phe Ser Val Ile Ala Met
                485                 490                 495

agc tat ctt atc agt gtc ttc ttc aac aac acc aac att gca gct ctg    1536
Ser Tyr Leu Ile Ser Val Phe Phe Asn Asn Thr Asn Ile Ala Ala Leu
            500                 505                 510

atc gga agc ctc atc tac atc att gcc ttc ttt cca ttt att gtt ctg    1584
Ile Gly Ser Leu Ile Tyr Ile Ile Ala Phe Phe Pro Phe Ile Val Leu
        515                 520                 525

gtt aca gtg gag aat gag ttg agc tat gta ttg aaa gtg ttc atg agc    1632
Val Thr Val Glu Asn Glu Leu Ser Tyr Val Leu Lys Val Phe Met Ser
    530                 535                 540

ctg ctg tcc cca aca gca ttc agc tat gca aac caa tac att gca cga    1680
Leu Leu Ser Pro Thr Ala Phe Ser Tyr Ala Asn Gln Tyr Ile Ala Arg
545                 550                 555                 560

tac gaa gaa cag ggc att ggt ctt cag tgg gaa aat atg tac acc tcc    1728
Tyr Glu Glu Gln Gly Ile Gly Leu Gln Trp Glu Asn Met Tyr Thr Ser
                565                 570                 575

ccg gtt cag gat gac acc acc tca ttt ggc tgg ctg tgc tgt cta atc    1776
Pro Val Gln Asp Asp Thr Thr Ser Phe Gly Trp Leu Cys Cys Leu Ile
            580                 585                 590

cta gct gac tct ttc att tat ttc ctt att gct tgg tat gtc agg aat    1824
Leu Ala Asp Ser Phe Ile Tyr Phe Leu Ile Ala Trp Tyr Val Arg Asn
        595                 600                 605

gtc ttc cca ggg aca tac ggt atg gca gct ccc tgg tat ttt cca att    1872
Val Phe Pro Gly Thr Tyr Gly Met Ala Ala Pro Trp Tyr Phe Pro Ile
    610                 615                 620

ctt cct tcc tat tgg aag gag cga ttt ggg tgt gca gag gtg aag cct    1920
Leu Pro Ser Tyr Trp Lys Glu Arg Phe Gly Cys Ala Glu Val Lys Pro
625                 630                 635                 640

gag aag agc aat ggc ctc atg ttt act aac atc atg atg cag aat acc    1968
Glu Lys Ser Asn Gly Leu Met Phe Thr Asn Ile Met Met Gln Asn Thr
                645                 650                 655

aac cca tct gcc agt cct gaa tac atg ttt tcc tct aac atc gag cct    2016
Asn Pro Ser Ala Ser Pro Glu Tyr Met Phe Ser Ser Asn Ile Glu Pro
            660                 665                 670

gaa cct aaa gat ctc aca gtc ggg gtt gcc ctg cat ggg gtc aca aag    2064
Glu Pro Lys Asp Leu Thr Val Gly Val Ala Leu His Gly Val Thr Lys
        675                 680                 685

atc tat ggc tca aaa gtt gct gtt gat aac ctc aat ctg aac ttt tat    2112
Ile Tyr Gly Ser Lys Val Ala Val Asp Asn Leu Asn Leu Asn Phe Tyr
    690                 695                 700

gaa ggg cat att act tca ttg ctg ggg ccc aat gga gct ggg aaa act    2160
Glu Gly His Ile Thr Ser Leu Leu Gly Pro Asn Gly Ala Gly Lys Thr
705                 710                 715                 720

act acc att tcc atg tta act ggg ctg ttt ggg gcc tca gca ggc acc    2208
Thr Thr Ile Ser Met Leu Thr Gly Leu Phe Gly Ala Ser Ala Gly Thr
                725                 730                 735

att ttt gta tat gga aaa gat atc aaa aca gac cta cac acg gta cgg 2256
Ile Phe Val Tyr Gly Lys Asp Ile Lys Thr Asp Leu His Thr Val Arg
            740                 745                 750

aag aac atg gga gtc tgt atg cag cac gac gtc ttg ttc agt tac ctc 2304
Lys Asn Met Gly Val Cys Met Gln His Asp Val Leu Phe Ser Tyr Leu
        755                 760                 765

act act aag gag cac ctt ctc cta tat ggt tcc atc aaa gtt cct cac 2352
Thr Thr Lys Glu His Leu Leu Leu Tyr Gly Ser Ile Lys Val Pro His
    770                 775                 780

tgg act aaa aag cag ctc cac gag gaa gta aaa agg act tta aaa gat 2400
Trp Thr Lys Lys Gln Leu His Glu Glu Val Lys Arg Thr Leu Lys Asp
785                 790                 795                 800

act gga cta tat agc cat cgt cat aag aga gtt gga aca ctg tca gga 2448
Thr Gly Leu Tyr Ser His Arg His Lys Arg Val Gly Thr Leu Ser Gly
                805                 810                 815

ggc atg aag agg aag tta tct ata tcc ata gct ctc att ggt gga tca 2496
Gly Met Lys Arg Lys Leu Ser Ile Ser Ile Ala Leu Ile Gly Gly Ser
            820                 825                 830

agg gta gta att ttg gat gaa cca tct act gga gtt gac cca tgt tct 2544
Arg Val Val Ile Leu Asp Glu Pro Ser Thr Gly Val Asp Pro Cys Ser
        835                 840                 845

cgc cga agt ata tgg gat gtt ata tcc aag aac aaa act gcc aga aca 2592
Arg Arg Ser Ile Trp Asp Val Ile Ser Lys Asn Lys Thr Ala Arg Thr
    850                 855                 860

atc att ctg tca acg cac cac ttg gac gag gct gaa gtg ctg agt gac 2640
Ile Ile Leu Ser Thr His His Leu Asp Glu Ala Glu Val Leu Ser Asp
865                 870                 875                 880

cgc atc gcc ttc ctg gag cag ggt ggg ctt agg tgc tgt ggg tcc cca 2688
Arg Ile Ala Phe Leu Glu Gln Gly Gly Leu Arg Cys Cys Gly Ser Pro
                885                 890                 895

ttt tac ctc aag gaa gcc ttt ggc gat ggg tat cac ctc acg ctt acc 2736
Phe Tyr Leu Lys Glu Ala Phe Gly Asp Gly Tyr His Leu Thr Leu Thr
            900                 905                 910

aag aag aag agt cca aat tta aat gca aat gca gta tgt gac acc atg 2784
Lys Lys Lys Ser Pro Asn Leu Asn Ala Asn Ala Val Cys Asp Thr Met
        915                 920                 925

gcc gtg aca gca atg atc caa tca cat ctc ccc gaa gcc tac ctc aag 2832
Ala Val Thr Ala Met Ile Gln Ser His Leu Pro Glu Ala Tyr Leu Lys
    930                 935                 940

gag gat att ggg gga gag ctt gtt tat gta ctt cct cca ttc agc acc 2880
Glu Asp Ile Gly Gly Glu Leu Val Tyr Val Leu Pro Pro Phe Ser Thr
945                 950                 955                 960

aaa gtc tca ggg gcc tac ctg tca ctc cta cgg gca ctc gac aat ggc 2928
Lys Val Ser Gly Ala Tyr Leu Ser Leu Leu Arg Ala Leu Asp Asn Gly
                965                 970                 975

atg ggt gac ctc aac atc ggg tgc tac ggc att tca gat acc acc gtg 2976

Met Gly Asp Leu Asn Ile Gly Cys Tyr Gly Ile Ser Asp Thr Thr Val
        980                 985                 990

gag gag gtc ttt ctg aac ttg acc aaa gag tca caa aaa aat agt gct     3024
Glu Glu Val Phe Leu Asn Leu Thr Lys Glu Ser Gln Lys Asn Ser Ala
    995                 1000                1005

atg agt ctt gag cac tta aca caa aag aaa att ggg aat tcc aat gcc     3072
Met Ser Leu Glu His Leu Thr Gln Lys Lys Ile Gly Asn Ser Asn Ala
    1010                1015                1020

aat ggc atc tca act cct gac gat tta tct gtg agc agc agc aat ttc     3120
Asn Gly Ile Ser Thr Pro Asp Asp Leu Ser Val Ser Ser Ser Asn Phe
1025                1030                1035                1040

aca gac aga gat gac aaa atc ctg aca aga gga gag agg ctg gat ggc     3168
Thr Asp Arg Asp Asp Lys Ile Leu Thr Arg Gly Glu Arg Leu Asp Gly
                1045                1050                1055

ttt gga ctg ttg ctg aag aag atc atg gct ata ctc atc aag agg ttc     3216
Phe Gly Leu Leu Leu Lys Lys Ile Met Ala Ile Leu Ile Lys Arg Phe
            1060                1065                1070

cac cac acc cgc agg aac tgg aaa ggt ctc att gct cag gtt atc ctc     3264
His His Thr Arg Arg Asn Trp Lys Gly Leu Ile Ala Gln Val Ile Leu
        1075                1080                1085

ccc atc gtc ttt gtt acc act gcc atg ggc ctt ggc aca ctg aga aat     3312
Pro Ile Val Phe Val Thr Thr Ala Met Gly Leu Gly Thr Leu Arg Asn
    1090                1095                1100

tcc agc aac agt tat cca gag att cag atc tcc ccc tct ctt tat ggt     3360
Ser Ser Asn Ser Tyr Pro Glu Ile Gln Ile Ser Pro Ser Leu Tyr Gly
1105                1110                1115                1120

acc tcc gaa cag aca gcc ttc tat gct aat tat cac ccg agc acg gaa     3408
Thr Ser Glu Gln Thr Ala Phe Tyr Ala Asn Tyr His Pro Ser Thr Glu
                1125                1130                1135

gca ctt gtc tca gca atg tgg gac ttc cct gga att gac aac atg tgt     3456
Ala Leu Val Ser Ala Met Trp Asp Phe Pro Gly Ile Asp Asn Met Cys
            1140                1145                1150

ctg aac acc agt gat cta cag tgt tta aac aaa gac agt ctg gaa aaa     3504
Leu Asn Thr Ser Asp Leu Gln Cys Leu Asn Lys Asp Ser Leu Glu Lys
        1155                1160                1165

tgg aac acc agt gga gaa ccc atc act aat ttt ggt gtt tgc tcc tgc     3552
Trp Asn Thr Ser Gly Glu Pro Ile Thr Asn Phe Gly Val Cys Ser Cys
    1170                1175                1180

tca gaa aat gtc cag gaa tgt cct aaa ttt aac tat tcc cca ccg cac     3600
Ser Glu Asn Val Gln Glu Cys Pro Lys Phe Asn Tyr Ser Pro Pro His
1185                1190                1195                1200

aga aga act tac tca tcc cag gta att tat aac ctc act ggg caa cga     3648
Arg Arg Thr Tyr Ser Ser Gln Val Ile Tyr Asn Leu Thr Gly Gln Arg
                1205                1210                1215

gtg gaa aat tat ctt ata tca act gca aat gag ttt gtc caa aaa aga     3696
Val Glu Asn Tyr Leu Ile Ser Thr Ala Asn Glu Phe Val Gln Lys Arg

1220                1225                1230

tat gga ggt tgg agt ttt ggg ctg cct ttg aca aaa gac ctt cgt ttt     3744
Tyr Gly Gly Trp Ser Phe Gly Leu Pro Leu Thr Lys Asp Leu Arg Phe
        1235                1240                1245

gat ata aca gga gtc cct gcc aat aga aca ctt gcc aag gta tgg tat     3792
Asp Ile Thr Gly Val Pro Ala Asn Arg Thr Leu Ala Lys Val Trp Tyr
    1250                1255                1260

gat cca gaa ggc tat cac tcc ctt cca gct tac ctc aac agc ctg aat     3840
Asp Pro Glu Gly Tyr His Ser Leu Pro Ala Tyr Leu Asn Ser Leu Asn
1265                1270                1275                1280

aat ttc ctt ctg cga gtt aac atg tca aaa tac gat gct gcc cga cat     3888
Asn Phe Leu Leu Arg Val Asn Met Ser Lys Tyr Asp Ala Ala Arg His
                1285                1290                1295

ggc atc atc atg tat agc cat cct tat cca gga gtg caa gac caa gaa     3936
Gly Ile Ile Met Tyr Ser His Pro Tyr Pro Gly Val Gln Asp Gln Glu
            1300                1305                1310

caa gcc aca atc agc agt tta atc gat att tta gtg gca ctg tct atc     3984
Gln Ala Thr Ile Ser Ser Leu Ile Asp Ile Leu Val Ala Leu Ser Ile
        1315                1320                1325

ttg atg ggc tac tct gtc acc acc gcc agc ttt gtc acc tat gtt gta     4032
Leu Met Gly Tyr Ser Val Thr Thr Ala Ser Phe Val Thr Tyr Val Val
    1330                1335                1340

agg gaa cat caa acc aaa gcc aaa cag ttg cag cac att tca ggc att     4080
Arg Glu His Gln Thr Lys Ala Lys Gln Leu Gln His Ile Ser Gly Ile
1345                1350                1355                1360

ggc gtg aca tgc tac tgg gta aca aac ttc att tat gac atg gtt ttc     4128
Gly Val Thr Cys Tyr Trp Val Thr Asn Phe Ile Tyr Asp Met Val Phe
                1365                1370                1375

tac ttg gtg cct gta gcg ttt tca att ggt atc att gcg att ttc aaa     4176
Tyr Leu Val Pro Val Ala Phe Ser Ile Gly Ile Ile Ala Ile Phe Lys
            1380                1385                1390

tta cct gca ttc tac agt gaa aac aac cta ggc gct gta tct ctc cta     4224
Leu Pro Ala Phe Tyr Ser Glu Asn Asn Leu Gly Ala Val Ser Leu Leu
        1395                1400                1405

ctt ctc ctg ttt ggg tat gca aca ttt tcc tgg atg tac ttg ctg gct     4272
Leu Leu Leu Phe Gly Tyr Ala Thr Phe Ser Trp Met Tyr Leu Leu Ala
    1410                1415                1420

ggg ctc ttc cat gaa aca gga atg gcc ttc atc act tac gtc tgt gtc     4320
Gly Leu Phe His Glu Thr Gly Met Ala Phe Ile Thr Tyr Val Cys Val
1425                1430                1435                1440

aac ttg ttt ttt ggc att aat tcc att gtt tcc ctg tca gtg gta tac     4368
Asn Leu Phe Phe Gly Ile Asn Ser Ile Val Ser Leu Ser Val Val Tyr
                1445                1450                1455

ttt ctt tcc aag gaa aag cct aat gat ccg act tta gaa ctt att tct     4416
Phe Leu Ser Lys Glu Lys Pro Asn Asp Pro Thr Leu Glu Leu Ile Ser
            1460                1465                1470

gaa acc ctc aag cgc att ttc ctg att ttc cca caa ttc tgt ttt ggc    4464
Glu Thr Leu Lys Arg Ile Phe Leu Ile Phe Pro Gln Phe Cys Phe Gly
        1475                1480                1485

tac ggt ttg att gaa ctt tct caa caa cag tcg gtc cta gac ttc tta    4512
Tyr Gly Leu Ile Glu Leu Ser Gln Gln Gln Ser Val Leu Asp Phe Leu
    1490                1495                1500

aaa gca tat gga gtg gaa tac cca aat gaa acc ttt gag atg aat aaa    4560
Lys Ala Tyr Gly Val Glu Tyr Pro Asn Glu Thr Phe Glu Met Asn Lys
1505                1510                1515                1520

cta ggt gca atg ttt gtg gct ttg gtt tct cag ggc acc atg ttt ttt    4608
Leu Gly Ala Met Phe Val Ala Leu Val Ser Gln Gly Thr Met Phe Phe
                1525                1530                1535

tcc ttg cga ctc tta atc aac gaa tcc ctg ata aag aaa ctc agg ctt    4656
Ser Leu Arg Leu Leu Ile Asn Glu Ser Leu Ile Lys Lys Leu Arg Leu
            1540                1545                1550

ttc ttc aga aaa ttt aat tct tca cat gta agg gag aca ata gat gag    4704
Phe Phe Arg Lys Phe Asn Ser Ser His Val Arg Glu Thr Ile Asp Glu
        1555                1560                1565

gat gaa gat gtg cgg gct gag aga tta aga gtt gag agt ggt gca gct    4752
Asp Glu Asp Val Arg Ala Glu Arg Leu Arg Val Glu Ser Gly Ala Ala
    1570                1575                1580

gaa ttt gac ttg gtc caa ctt tat tgt ctc aca aag acc tac caa ctt    4800
Glu Phe Asp Leu Val Gln Leu Tyr Cys Leu Thr Lys Thr Tyr Gln Leu
1585                1590                1595                1600

atc cac aaa aag att ata gct gta aac aac atc agc att ggg ata cct    4848
Ile His Lys Lys Ile Ile Ala Val Asn Asn Ile Ser Ile Gly Ile Pro
                1605                1610                1615

gct gga gag tgt ttt ggg ctt ctt gga gtg aat gga gca gga aag acc    4896
Ala Gly Glu Cys Phe Gly Leu Leu Gly Val Asn Gly Ala Gly Lys Thr
            1620                1625                1630

act ata ttc aag atg ctg aca gga gac atc att cct tca agt gga aac    4944
Thr Ile Phe Lys Met Leu Thr Gly Asp Ile Ile Pro Ser Ser Gly Asn
        1635                1640                1645

att ctg atc aga aat aag acc gga tct ctg ggt cac gtt gat tct cac    4992
Ile Leu Ile Arg Asn Lys Thr Gly Ser Leu Gly His Val Asp Ser His
    1650                1655                1660

agc tca tta gtt ggc tac tgt cct cag gaa gat gcc tta gat gac ctg    5040
Ser Ser Leu Val Gly Tyr Cys Pro Gln Glu Asp Ala Leu Asp Asp Leu
1665                1670                1675                1680

gta act gtg gaa gaa cat ttg tat ttc tat gcc agg gta cat gga att    5088
Val Thr Val Glu Glu His Leu Tyr Phe Tyr Ala Arg Val His Gly Ile
                1685                1690                1695

cca gaa aag gat att aaa gaa act gtt cat aaa ctc ctt agg aga ctt    5136
Pro Glu Lys Asp Ile Lys Glu Thr Val His Lys Leu Leu Arg Arg Leu
            1700                1705                1710

cac ctg atg ccc ttc aag gac aga gct acc tct atg tgc agt tat ggc     5184
His Leu Met Pro Phe Lys Asp Arg Ala Thr Ser Met Cys Ser Tyr Gly
        1715                1720                1725

aca aaa aga aaa tta tcc act gca ctg gcc ttg ata ggg aaa cct tcc     5232
Thr Lys Arg Lys Leu Ser Thr Ala Leu Ala Leu Ile Gly Lys Pro Ser
    1730                1735                1740

att cta ctg ctg gat gag ccg agc tct ggc atg gat ccg aag tcg aaa     5280
Ile Leu Leu Leu Asp Glu Pro Ser Ser Gly Met Asp Pro Lys Ser Lys
1745                1750                1755                1760

cgg cac ctc tgg aag atc att tca gaa gaa gta cag aac aaa tgt tcc     5328
Arg His Leu Trp Lys Ile Ile Ser Glu Glu Val Gln Asn Lys Cys Ser
                1765                1770                1775

gtc atc ctc aca tct cac agc atg gaa gaa tgt gaa gct ctc tgt acc     5376
Val Ile Leu Thr Ser His Ser Met Glu Glu Cys Glu Ala Leu Cys Thr
            1780                1785                1790

agg ttg gcc att atg gtg aat gga aag ttt caa tgt att gga tct ttg     5424
Arg Leu Ala Ile Met Val Asn Gly Lys Phe Gln Cys Ile Gly Ser Leu
        1795                1800                1805

cag cac ata aag agc agg ttt gga cga gga ttt act gtc aaa gtt cac     5472
Gln His Ile Lys Ser Arg Phe Gly Arg Gly Phe Thr Val Lys Val His
    1810                1815                1820

ttg aag aat aac aaa gtg acc atg gag acc ctc aca aag ttc atg cag     5520
Leu Lys Asn Asn Lys Val Thr Met Glu Thr Leu Thr Lys Phe Met Gln
1825                1830                1835                1840

ctg cac ttt cca aaa aca tac tta aaa gat cag cac ctc agc atg cta     5568
Leu His Phe Pro Lys Thr Tyr Leu Lys Asp Gln His Leu Ser Met Leu
                1845                1850                1855

gag tat cat gta cca gtc aca gca gga gga gtc gca aac att ttt gat     5616
Glu Tyr His Val Pro Val Thr Ala Gly Gly Val Ala Asn Ile Phe Asp
            1860                1865                1870

ctg ctg gaa acc aac aag act gct tta aat att aca aat ttc tta gtg     5664
Leu Leu Glu Thr Asn Lys Thr Ala Leu Asn Ile Thr Asn Phe Leu Val
        1875                1880                1885

agt cag acc act ctg gaa gag gtt ttc atc aac ttt gcc aaa gac cag     5712
Ser Gln Thr Thr Leu Glu Glu Val Phe Ile Asn Phe Ala Lys Asp Gln
    1890                1895                1900

aag tcc tat gaa act gct gat acc agc agc caa ggt tcc act ata agt     5760
Lys Ser Tyr Glu Thr Ala Asp Thr Ser Ser Gln Gly Ser Thr Ile Ser
1905                1910                1915                1920

gtt gac tca caa gat gac cag atg gag tct                             5790
Val Asp Ser Gln Asp Asp Gln Met Glu Ser
                1925                1930

<210> 4
<211> 198
<212> PRT
<213> Artificial Sequence

<220>
<223> consensus

<400> 4
Gly Glu Val Leu Ala Leu Val Gly Pro Asn Gly Ala Gly Lys Ser Thr
1               5                   10                  15
Leu Leu Lys Leu Ile Ser Gly Leu Leu Pro Pro Thr Glu Gly Thr Ile
            20                  25                  30
Leu Leu Asp Gly Ala Arg Asp Leu Arg Leu Ser Lys Leu Lys Glu Arg
        35                  40                  45
Leu Glu Arg Leu Arg Lys Asn Ile Gly Val Val Phe Gln Asp Pro Thr
    50                  55                  60
Leu Phe Pro Asn Val Glu Leu Thr Val Arg Glu Asn Ile Ala Phe Gly
65                  70                  75                  80
Leu Arg Leu Ser Leu Gly Leu Ser Lys Asp Glu Gln Arg Ala Arg Leu
                85                  90                  95
Lys Lys Ala Gly Ala Glu Glu Leu Leu Glu Arg Leu Gly Leu Gly Tyr
            100                 105                 110
Asp His Leu Leu Asp Arg Arg Pro Gly Thr Leu Ser Gly Gly Gln Lys
        115                 120                 125
Gln Arg Val Ala Ile Ala Arg Ala Leu Leu Thr Lys Pro Lys Leu Leu
    130                 135                 140
Leu Leu Asp Glu Pro Thr Ala Gly Leu Asp Pro Ala Ser Arg Ala Gln
145                 150                 155                 160
Leu Leu Glu Leu Leu Arg Glu Leu Arg Gln Gln Gly Gly Thr Val Leu
                165                 170                 175
Leu Ile Thr His Asp Leu Asp Leu Leu Asp Arg Leu Ala Asp Arg Ile
            180                 185                 190
Leu Val Leu Glu Asp Gly
        195

<210> 5
<211> 15
<212> PRT
<213> Arificial sequence
<220>
<223> Consensus

<221> VARIANT
<222> (1)...(1)
<223> Xaa at position 1 can be L, I, V, M, F, Y,
      or C.

<221> VARIANT
<222> (2)...(2)
<223> Xaa  at position 2 can be S, or A.

<221> VARIANT
<222> (3)...(3)
<223> Xaa at position 3 can be S, A, P, G, L, V,
      F, Y, K, Q, or H

<221> VARIANT
<222> (5)...(5)

<223> Xaa at position 5 can be  D, E, N, Q, M,
      or W

<221> VARIANT
<222> (6)...(6)
<223> Xaa at position 6
      can be K, R, Q, A, S, P, C, L, I, M, F, or W

<221> VARIANT
<222> (7)...(7)
<223> Xaa at position can be K, R, N, Q, S, T,
      A, V, or M

<221> VARIANT
<222> (8)...(8)
<223> Xaa at position 8 can be K, R, A, C, L, V,
      or M.

<221> VARIANT
<222> (9)...(9)
<223> Xaa at position 9 can be L, I, V, M, F, Y,
      P, A, or N.

<221> VARIANT
<222> (10)...(10)
<223> Xaa at position 10 can be A, E, Q, D,
      N, L, G, K, S, V, R, T, I, M F, W, or C.

<221> VARIANT
<222> (11)...(11)
<223> Xaa at position 11 can be L, I, V, M, F,
      or W.

<221> VARIANT
<222> (12)...(12)
<223> Xaa at position can be  S, A, G, C, L, T,
      V, or P.

<221> VARIANT
<222> (13)...(13)
<223> Xaa at position 13 can be A, E, Q, D,
      N, L, G, K, S, V, R, T, I, M, or C.

<221> VARIANT
<222> (14)...(14)
<223> Xaa  at position 14 can be A, E, Q, D,
      N, L, G, S, V, T, I, M, F, Y, C, or W.

<221> VARIANT
<222> (15)...(15)
<223> The amino acid at position 15 can be L, I, V, M, F,
      Y, W, S, T, or A

<400> 5
Xaa Xaa Xaa Gly Xaa Xaa Xaa Xaa Xaa Xaa Xaa Xaa Xaa Xaa Xaa
 1               5                   10                  15